# EUROPEAN PATENT SPECIFICATION

(11) **EP 4 087 651 B1**
(45) Date of publication and mention of the grant of the patent: **02.12.2026**
(21) Application number: 20712065.0
(22) Date of filing: 10.01.2020
(51) Int. Cl.: A61P 7/00, A61K 31/396, A61P 9/00, A61P 35/00, C07D 203/24, C07D 401/12, C07D 405/12

(54) **AROMATIC SULPHONAMIDES DERIVATIVES THAT INHIBITS PDI A1, THEIR SYNTHESIS AND USE**
AROMATISCHE SULFONAMIDDERIVATE, DIE PDI A1 HEMMEN, IHRE SYNTHESE UND VERWENDUNG
DÉRIVÉS DE SULFONAMIDES AROMATIQUES INHIBANT PDI A1, LEUR SYNTHÈSE ET LEUR UTILISATION

(43) Date of publication of application: 16.11.2022
(73) Proprietor: UNIWERSYTET JAGIELLONSKI, 31-007 Kraków (PL)
(72) Inventor: KALVINS, Ivars, 1006 Riga (LV); CHLOPICKI, Stefan, 31-007 Kraków (PL); ANDRIANOV, Victor, 1006 Riga (LV); STOJAK, Marta, 31-007 Kraków (PL); DOMRACEVA, Ilona, 1006 Riga (LV); KANEPE-LAPSA, Iveta, 1006 Riga (LV); ZELENCOVA, Diana, 1006 Riga (LV); WIETRZYK, Joanna, 53-114 Wroclaw (PL); TURLEJ, Eliza, 53-114 Wroclaw (PL); STACHOWICZ, Martyna, 53-114 Wroclaw (PL); JAROSZ, Joanna, 53-114 Wroclaw (PL); MILCZAREK, Magdalena, 53-114 Wroclaw (PL); KRAMKOWSKI, Karol, 15-089 Bialystok (PL)
(74) Representative: Czarnik, Maciej
(86) International application number: PCT/PL2020/050004
(87) International publication number: WO 2021/141506

(56) References cited:
- WO-A1-2016/118639
- US-A- 4 267 174
- US-A1- 2008 242 677
- BALDWIN J E ET AL: "AMINO ACID SYNTHESIS VIA RING OPENING OF N-SULPHONYL AYIRIDINE-2- CARBOXYLATE ESTERS WITH ORGANOMETALLIC REAGENTS", TETRAHEDRON, ELSEVIER SCIENCE PUBLISHERS, AMSTERDAM, NL, vol. 49, no. 28, 1 January 1993 (1993-01-01), pages 6309 - 6330, XP002070483, ISSN: 0040-4020, DOI: 10.1016/S0040-4020(01)87968-0
- SZE KUI ET AL: "Electronic Supplementary Information Intermolecular (4+3) Cycloadditions of Aziridinyl Enolsilanes", 16 December 2013 (2013-12-16), pages S1 - S54, XP055719726, Retrieved from the Internet <URL:http://www.rsc.org/suppdata/cc/c3/c3cc48266a/c3cc48266a.pdf> [retrieved on 20200803]
- YOUHEI TAKEDA ET AL: "Supporting Information Asymmetric Synthesis of b 2 -Aryl Amino Acids through Pd- Catalyzed Enantiospecific and Regioselective Ring-Opening Suzuki-Miyaura Arylation of Aziridine-2-carboxylates", 3 June 2019 (2019-06-03), pages S1 - S126, XP055719731, Retrieved from the Internet <URL:https://chemistry-europe.onlinelibrary.wiley.com/action/downloadSupplement?doi=10.1002/chem.201902009&file=chem201902009-sup-0001-misc_information.pdf> [retrieved on 20200803]

## Description

### Field of the invention

The invention relates to a new group of aromatic sulphonamides derivatives and their synthesis and use for modulation of the activity of protein disulfide isomerase (PDI). More particularly, the invention provides small molecule inhibitors of PDI A1 that display antiplatelet, antithrombotic and anticancer activities.

### Background of the invention

Protein disulfide isomerase (PDI) is a thiol-oxidoreductase chaperone protein that is responsible for the isomerization, reduction, and oxidation of non-native disulfide bonds. There are known over 20 members of the PDI family of enzymes. Structurally, prototypic PDI consists of four domains with a thioredoxin fold: a, b, b' and a', an extended C-terminus with KDEL ER retention sequence, and an interdomain linker x between the b' and a' domains. The a and a' domains are catalytically active, contain redox active CGHC active site and independently can perform oxidation and reduction reactions (Darby and Creighton, 1995). The b and b' domains are noncatalytic, but provide a substrate-binding domain of PDI. All four domains are needed to achieve the isomerization and chaperone activity of PDI. Besides its catalytic role involving thiols and disulfides, PDI also serves an essential structural role as the beta subunit of prolyl-4-hydroxylase (Koivu et al., 1987) and as a microsomal triglyceride transfer protein (Wetterau et al., 1990).

Protein disulfide isomerase (PDI) catalyze posttranslational disulfide bond formation and exchange and serve as chaperones during protein folding (Hatahet et al., 2009). PDI has been also identified at many diverse subcellular locations outside the endoplasmic reticulum. It has biological functions on the cell surfaces of lymphocytes, hepatocytes, platelets, and endothelial cells (Manickam et al., 2008; Hotchkiss et al., 1998; Essex et al., 1999; Burgess et al., 2000; Bennett et al., 2000; Hotchkiss et al., 1998; Burgess et al., 2000). PDI is rapidly secreted from both endothelial cells and platelets during thrombus formation *in vivo* (Cho et al., 2008; Jasuja et al., 2010). Inhibition of PDI using neutralizing antibodies blocks thrombus formation in several thrombosis models (Bennett et al., 2000; Cho et al., 2008; Jasuja et al., 2010; Reinhardt et al. 2008). Inhibition of PDI in these models abrogates not only platelet accumulation at the injury site but also fibrin generation.

Inhibition of protein disulfide isomerase (PDI) by small molecules is also beneficial in cell and brain slice models and prevents cellular apoptosis (Hoffstrom et al., 2010) . Inhibition of lymphocyte surface-associated PDI blocks HIV/cell fusion and HIV-1 pathogenesis (Barbouche et al., 2003).

Importantly, PDI has been implicated in proliferation, survival and metastasis of several types of cancers (Lee et al, 2017, Xu et al., 2012; Hashida et al., 2011; Lovat et al., 2008),. These observations demonstrate a critical role for PDI in various pathologies (Cho et al., 2008) including thrombus formation and development of cancer. In particular, PDI A1 the major isoform of PDI is a novel interesting target to develop antiplatelet, antithrombotic effects and anticancer therapeutics..

Presently, almost all available inhibitors of PDI that are small molecules are sulfhydryl-reactive compounds that bind covalently and are non-selective, acting broadly on thiol isomerases (Karala et al., 2010) or are cytotoxic (Lovat et al., 2008; Khan et al., 2011).

Several patent documents provide compounds that inhibit enzyme activity of cell-associated protein disulfide isomerase e.g. US20160145209A1, WO2016118639, US20150133514A1, US20020115713A1, WO2017011890A1, but none of them relates to aromatic sulphonamides derivatives.

Several substituted sulfonylaziridine-2-carboxylates can be obtained via tosylation of the nitrogen of commercially available aminoacid methyl ester hydrochlorides, followed by Mitsunobu ring closure, (Takeda et al. 2019).

Thus, there is a clear need for new agents that interfere with PDI A1 activity but are otherwise selective and well tolerated in therapeutic contexts. Now it has been found that some of among N,N-disubstituted aromatic sulphonamides possess unique pharmacological properties associated with their ability to inhibit PDIA1 activity, which property affords their antiplatelet, antithrombotic, and anticancer activities.

### Detailed description of the invention

The invention relates to N,N-disubstituted aromatic sulphonamides derivatives of formula (I) in form of racemates or enantiomers that inhibits PDI A1:
1-(4-Nitrophenyl)sulfonylaziridine-2-carboxamide(C-3216)
1-(4-Aminophenyl)sulfonylaziridine-2-carboxamide (C-3218)
Methyl 1-[4-(trifluoromethyl)phenyl]sulfonylaziridine-2-carboxylate (C-3256)
Methyl 1-(4-butylphenyl)sulfonylaziridine-2-carboxylate (C-3257)
Methyl 1-(4-dodecylphenyl)sulfonylaziridine-2-carboxylate (C-3270)
Methyl 1-(4-butoxyphenyl)sulfonylaziridine-2-carboxylate (C-3271)
1-[1-(4-Butylphenyl)sulfonylaziridin-2-yl]ethanone (C-3272)
1-(4-Butylphenyl)sulfonylaziridine-2-carbaldehyde (C-3273)
Methyl 1-(4-pentylphenyl)sulfonylaziridine-2-carboxylate (C-3281)
Methyl 1-(4-hexylphenyl)sulfonylaziridine-2-carboxylate (C-3287)
Methyl 1-(4-heptylphenyl)sulfonylaziridine-2-carboxylate (C-3288)
Methyl 1-(4-tert-butylphenyl)sulfonylaziridine-2-carboxylate (C-3290)
Methyl 1-(4-phenylphenyl)sulfonylaziridine-2-carboxylate (C-3291)
Methyl 1-(2-naphthylsulfonyl)aziridine-2-carboxylate (C-3292)
Methyl 1-(4-isopropylphenyl)sulfonylaziridine-2-carboxylate (C-3295)
Methyl 1-(2-fluoro-4-methyl-phenyl)sulfonylaziridine-2-carboxylate (C-3297)
Methyl 1-(4-propylphenyl)sulfonylaziridine-2-carboxylate (C-3299)
Methyl 1-(m-tolylsulfonyl)aziridine-2-carboxylate (C-3303)
Methyl 1-[3-(trifluoromethy1)phenyl]sulfonylaziridine-2-carboxylate (C-3305)
Methyl 1-(4-acetamidophenyl)sulfonylaziridine-2-carboxylate (C-3308)
Methyl 1-(1-naphthylsulfonyl)aziridine-2-carboxylate (C-3311)
Methyl 1-(4-cyclohexylphenyl)sulfonylaziridine-2-carboxylate (C-3316)
Methyl 1-(4-octylphenyl)sulfonylaziridine-2-carboxylate (C-3319)
Methyl 1-(o-tolylsulfonyl)aziridine-2-carboxylate (C-3320)
1-[4-(Trifluoromethyl)phenyl]sulfonylaziridine-2-carboxamide (C-3324)
Methyl 1-[[5-(dimethylamino)-1-naphthyl]sulfonyl]aziridine-2-carboxylate (C-3326)
Methyl 1-[(6-methoxy-2-naphthyl)sulfonyl]aziridine-2-carboxylate (C-3327)
Methyl 1-[(6-methyl-2-naphthyl)sulfonyl]aziridine-2-carboxylate (C-3329)
Methyl 1-(4-ethylphenyl)sulfonylaziridine-2-carboxylate (C-3332)
Methyl 1-[4-(dimethylamino)phenyl]sulfonylaziridine-2-carboxylate (C-3343)
Methyl 1-(2,4-difluorophenyl)sulfonylaziridine-2-carboxylate (C-3353)
1-[4-(Dimethylamino)phenyl]sulfonyl-N,N-dimethyl-aziridine-2-carboxamide (C-3357)
1-(4-Butylphenyl)sulfonyl-N,N-dimethyl-aziridine-2-carboxamide (C-3362)
1-(4-Isopropylphenyl)sulfonyl-N,N-dimethyl-aziridine-2-carboxamide (C-3364)
N,N-Dimethyl-1-(1-naphthylsulfonyl)aziridine-2-carboxamide (C-3365)
N,N-Dimethyl-1-[(4-methyl-1-naphthyl)sulfonyl]aziridine-2-carboxamide (C-3366)
N,N-Dimethyl-1-(4-pentylphenyl)sulfonyl-aziridine-2-carboxamide (C-3368)
1-(4-Hexylphenyl)sulfonyl-N,N-dimethyl-aziridine-2-caiboxamide (C-3369)
N,N-Dimethyl-1-[4-(trifluoromethyl)phenyl]sulfonyl-aziridine-2-carboxamide (C-3374)
1-(4-Hexylphenyl)sulfonyl-N-methyl-aziridine-2-carboxamide (C-3380)
Methyl 1-[[5-(dimethylamino)-2-naphthyl]sulfonyl]aziridine-2-carboxylate (C-3399)
1-(4-Hexylphenyl)sulfonylaziridine-2-carboxamide (C-3389)
1-(4-Cyclohexylphenyl)sulfonyl-N,N-dimethyl-aziridine-2-carboxamide (C-3384)
1-(2,4-Difluorophenyl)sulfonylaziridine-2-carboxamide (C-3402)
1-(2,4-Difluorophenyl)sulfonyl-N,N-dimethyl-aziridine-2-carboxamide (C-3403)
1-[[6-(Dimethylamino)-1-naphthyl]sulfonyl]aziridine-2-carboxamide (C-3459)
[1-(4-Hexylphenyl)sulfonylaziridin-2-yl]-pyrrolidin-1-yl-methanone (C-3511)
[1-(4-Hexylphenyl)sulfonylaziridin-2-yl]-morpholino-methanone (C-3517)
Methyl 1-(4-fluorophenyl)sulfonylaziridine-2-carboxylate (C-3576)
N,N-Diethyl-1-(4-hexylphenyl)sulfonyl-aziridine-2-carboxamide (C-3546)
1-(4-tert-Butylphenyl)sulfoirylaziridine-2-carboxamide (C-3591)
1-(4-tert-Butylphenyl)sulfonyl-N,N-dimethyl-aziridine-2-carboxamide (C-3594)
1-(4-tert-Butylphenyl)sulfoiryl-N-methylaziridine-2-carboxamide (C-3593).

Method for the preparation of N,N-disubstituted aromatic sulphonamides derivatives of formula (I), wherein that solution of appropriate aziridine derivative, selected from group, consisting of methyl-aziridin-2-carboxylate, 2-cyano-aziridine, aziridine-2-carboxamide, aziridine -2-carboxaldehyde, aziridine-2-methylketone and aziridine-2-N,N-dialkylcarboxamide aziridine-2-N,N-cycloalkyl or aziridine-2-N,N-cycloheteroalkylcarboxamide is treated with appropriate sufonylchloride in presence of base: 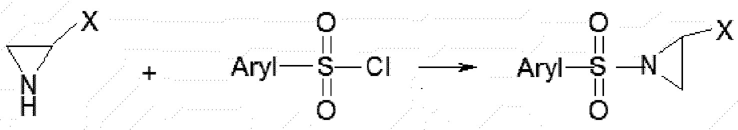 X=COOAlkyl, CONH₂, CONHAlkyl, CONAlkyl₂,CN, CHO, COAlkyl , 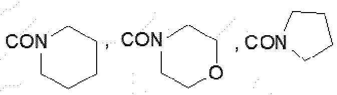 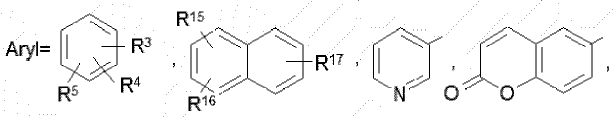 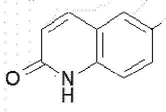
wherein R³, R⁴ and R⁵ are: H, linear alkyl group C₁-C₁₂, O-alkyl C₁-C₄, branched alkyl C₃-C4, cycloalkyl, phenyl, NO₂, halogen (Cl, F), trifluoromethyl, C₁-C₄ alkoxy,
C₁-C₄ dialkylamino, C₁-C₄ acylamino; and R¹⁵, R¹⁶, R¹⁷ are: H, alkyl C₁-C₄, Cl, O-alkyl C₁-C₄, -CHO and NR¹⁸R¹⁹, where R¹⁸ and R¹⁹ are H or alkyl C₁-C₄

The invention also relates to N,N-disubstituted aromatic sulphonamides or a pharmaceutically acceptable salt selected from the list for use in the treatment or prevention of diseases linked to PDI A1:
Methyl 1-(p-tolylsulfonyl)aziridine-2-carboxylate (C-3161);
Methyl 1-(4-nitrophenyl)sulfonylaziridine-2-carboxylate (C-3212);
1-(4-Nitrophenyl)sulfonylaziridine-2-carboxamide (C-3216);
1-(4-Aminophenyl)sulfonylaziridine-2-carboxamide (C-3218);
1-(p-Tolylsulfonyl)aziridine-2-carboxamide (C-3220);
Methyl 1-(benzenesulfonyl)aziridine-2-carboxylate (C-3251);
Methyl 1-[4-(trifluoromethyl)phenyl]sulfonylaziridine-2-carboxylate (C-3256);
Methyl 1-(4-butylphenyl)sulfonylaziridine-2-carboxylate (C-3257);
1-(p-Tolylsulfonyl)aziridine-2-carbaldehyde (C-3262);
1-[1-(p-Tolylsulfonyl)aziridin-2-yl]ethanone (C-3263);
Methyl 1-(4-dodecylphenyl)sulfonylaziridine-2-carboxylate (C-3270);
Methyl 1-(4-butoxyphenyl)sulfonylaziridine-2-carboxylate (C-3271);
1-[1-(4-Butylphenyl)sulfonylaziridin-2-yl]ethanone (C-3272);
1-(4-Butylphenyl)sulfonylaziridine-2-carbaldehyde (C-3273);
Methyl 1-(4-pentylphenyl)sulfonylaziridine-2-carboxylate (C-3281);
Methyl 1-(4-hexylphenyl)sulfonylaziridine-2-carboxylate (C-3287);
Methyl 1-(4-heptylphenyl)sulfonylaziridine-2-carboxylate (C-3288);
Methyl 1-(4-tert-butylphenyl)sulfonylaziridine-2-carboxylate (C-3290);
Methyl 1-(4-phenylphenyl)sulfonylaziridine-2-carboxylate (C-3291);
Methyl 1-(2-naphthylsulfonyl)aziridine-2-carboxylate (C-3292);
Methyl 1-(4-methoxyphenyl)sulfonylaziridine-2-carboxylate (C-3294);
Methyl 1-(4-isopropylphenyl)sulfonylaziridine-2-carboxylate (C-3295);
Methyl 1-(4-chlorophenyl)sulfonylaziridine-2-carboxylate (C-3296);
Methyl 1-(2-fluoro-4-methyl-phenyl)sulfonylaziridine-2-carboxylate (C-3297);
Methyl 1-(4-propylphenyl)sulfonylaziridine-2-carboxylate (C-3299);
Methyl 1-(m-tolylsulfonyl)aziridine-2-carboxylate (C-3303);
Methyl 1-(4-propylphenyl)sulfonylaziridine-2-carboxylate (C-3304);
Methyl 1-[3-(trifluoromethyl)phenyl]sulfonylaziridine-2-carboxylate (C-3305);
Methyl 1-(4-acetamidophenyl)sulfonylaziridine-2-carboxylate (C-3308);
Methyl 1-(1-naphthylsulfonyl)aziridine-2-carboxylate (C-3311);
1-(p-Tolylsulfonyl)aziridine-2-carbonitrile (C-3314);
Methyl 1-(4-cyclohexylphenyl)sulfonylaziridine-2-carboxylate (C-3316);
Methyl 1-(4-octylphenyl)sulfonylaziridine-2-carboxylate (C-3319);
Methyl 1-(o-tolylsulfonyl)aziridine-2-carboxylate (C-3320);
1-[4-(Trifluoromethyl)phenyl]sulfonylaziridine-2-carboxamide (C-3324);
Methyl 1-[[5-(dimethylamino)-1-naphthyl]sulfonyl]aziridine-2-carboxylate (C-3326);
Methyl 1-[(6-methoxy-2-naphthyl)sulfonyl]aziridine-2-carboxylate (C-3327);
Methyl 1-[(6-methyl-2-naphthyl)sulfonyl]aziridine-2-carboxylate (C-3329);
Methyl 1-(4-ethylphenyl)sulfonylaziridine-2-carboxylate (C-3332);
Methyl 1-[(4-methyl-1-naphthyl)sulfonyl]aziridine-2-carboxylate (C-3336);
N,N-Dimethyl-1-(p-tolylsulfonyl)aziridine-2-carboxamide (C-3342);
Methyl 1-[4-(dimethylamino)phenyl]sulfonylaziridine-2-carboxylate (C-3343);
Methyl 1-(2-furylsulfonyl)aziridine-2-carboxylate (C-3346);
Methyl 1-[(2-oxo-1H-quinolin-6-yl)sulfonyl]aziridine-2-carboxylate (C-3350);
Methyl 1-(2,4-difluorophenyl)sulfonylaziridine-2-carboxylate (C-3353);
Methyl 1-(2-oxochromen-6-yl)sulfonylaziridine-2-carboxylate (C-3355);
1-[4-(Dimethylamino)phenyl]sulfonyl-N,N-dimethyl-aziridine-2-carboxamide (C-3357);
1-(4-Butylphenyl)sulfonyl-N,N-dimethyl-aziridine-2-carboxamide (C-3362);
1-(4-Isopropylphenyl)sulfonyl-N,N-dimethyl-aziridine-2-carboxamide (C-3364);
N,N-Dimethyl-1-(1-naphthylsulfonyl)aziridine-2-carboxamide (C-3365);
N,N-Dimethyl-1-[(4-methyl-1-naphthyl)sulfonyl]aziridine-2-carboxamide (C-3366);
N,N-Dimethyl-1-(4-pentylphenyl)sulfonyl-aziridine-2-carboxamide (C-3368);
1-(4-Hexylphenyl)sulfonyl-N,N-dimethyl-aziridine-2-carboxamide (C-3369);
N,N-Dimethyl-1-[4-(trifluoromethyl)phenyl]sulfonyl-aziridine-2-carboxamide (C-3374);
1-(4-Hexylphenyl)sulfonyl-N-methyl-aziridine-2-carboxamide (C-3380);
1-(4-Cyclohexylphenyl)sulfonyl-N,N-dimethyl-aziridine-2-carboxamide (C-3384);
1-(4-Hexylphenyl)sulfonylaziridine-2-carboxamide (C-3389);
Methyl 1-[[5-chloro-6-(methylamino)-2-naphthyl]sulfonyl]aziridine-2-carboxylate (C-3393);
1-(2,4-Difluorophenyl)sulfonylaziridine-2-carboxamide (C-3402);
1-(2,4-Difluorophenyl)sulfonyl-N,N-dimethyl-aziridine-2-carboxamide (C-3403);
1-[[6-(Dimethylamino)-1-naphthyl]sulfonyl]aziridine-2-carboxamide (C-3459);
[1-(4-Hexylphenyl)sulfonylaziridin-2-yl]-pyrrolidin-1-yl-methanone (C-3511);
[1-(4-Hexylphenyl)sulfonylaziridin-2-yl]-morpholino-methanone (C-3517);
1-(Benzenesulfonyl)aziridine-2-carboxamide (C-3532);
Methyl (2S)-1-(p-tolylsulfonyl)aziridine-2-carboxylate (C-3535);
Ethyl 1-(p-tolylsulfonyl)aziridine-2-carboxylate (C-3537);
Methyl (2R)-1-(p-tolylsulfonyl)aziridine-2-carboxylate (C-3539);
N,N-Diethyl-1-(4-hexylphenyl)sulfonyl-aziridine-2-carboxamide (C-3546);
Methyl 1-(3-pyridylsulfonyl)aziridine-2-carboxylate (C-3559);
1-(3-pyridylsulfonyl)aziridine-2-carboxamide (C-3562);
Methyl (2R)-1-[[6-(dimethylamino)-1-naphthyl]sulfonyl]aziridine-2-carboxylate (C-3570);
Methyl 1-(4-fluorophenyl)sulfonylaziridine-2-carboxylate (C-3576);
1-(4-tert-Butylphenyl)sulfonylaziridine-2-carboxamide (C-3591);
1-(4-tert-Butylphenyl)sulfonyl-N-methylaziridine-2-carboxamide (C-3593);
1-(4-tert-Butylphenyl)sulfonyl-N,N-dimethyl-aziridine-2-carboxamide (C-3594);
Lithium 1-tosylaziridine-2-carboxylate (C-3612) .

Preferably, the compounds of the invention are for use in treatment and prevention of excessive platelet activation and thrombosis, in particular any disease from the list: disease or condition is thrombosis, thrombotic diseases, in particular the thrombotic disease is acute myocardial infarction, stable angina, unstable angina, aortocoronary bypass surgery, acute occlusion following coronary angioplasty and/or stent placement, transient ischemic attacks, cerebrovascular disease, peripheral vascular disease, placental insufficiency, prosthetic heart valves, atrial fibrillation, anticoagulation of tubing, deep vein thrombosis or pulmonary embolism and other pathologies linked with excessive activation of platelets and thrombosis including cancer-related thrombosis.

Also preferably, the compounds of the invention are for use in treatment and prevention of cancer in particular any disease from the list: gastrointestinal cancer, colorectal cancer, colon cancer, liver cancer, hepatocellular carcinoma, pancreatic cancer, biliary tract cancer, stomach cancer, genitourinary cancer, bladder cancer, testicular cancer, cervical cancer, malignant mesothelioma, osteogenic sarcoma, esophageal cancer, laryngeal cancer, prostate cancer, hormone-refractory prostate cancer, lung cancer, small cell lung cancer, non-small cell lung cancer, breast cancer, triple-negative breast cancer, breast cancer having a BRCA1 and/or BRCA2 gene mutation, hematological cancer, leukemia, acute lymphoblastic leukemia, acute myeloid leukemia, chronic lymphocytic leukemia, chronic myeloid leukemia, lymphoma, Hodgkin lymphoma, non-Hodgkin lymphoma, follicular lymphoma, diffuse large B-cell lymphoma, ovarian cancer, brain cancer, neuroblastoma, Ewing's sarcoma, kidney cancer, epidermoid cancer, skin cancer, melanoma, head and/or neck cancer, head and neck squamous cell carcinoma, and mouth cancer.

The invention has been described in embodiments and figures non-limiting of the scope of protection, where:
- Fig. 1: shows the influence of PDI A1 inhibitor C-3251 on the clonogenic potential, cell cycle and cell death of human colon cancer cell lines;
- Fig. 2: shows effect of bepristat and C-3380 and C-3389 inhibitor on MDA-MB-231 cell transendothelial migration across endothelial monolayer;

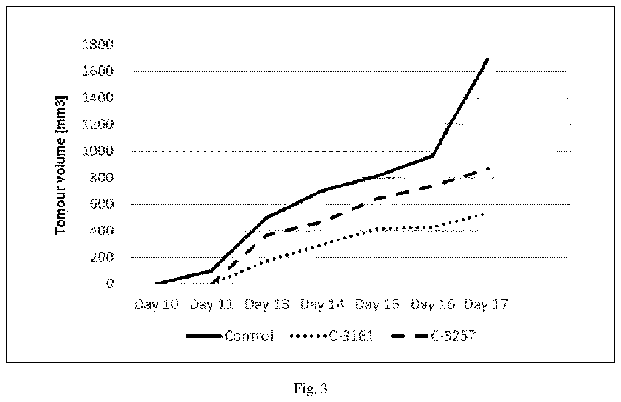

- Fig. 3: shows antiplatelet antithrombotic effects in in vivo model of thrombosis for selected PDI A1 inhibitor and reference compounds;
- Fig. 4: shows dose dependence of the anti-thrombotic effects for the selected PDIA1 inhibitor in in vivo model of thrombosis.
- Fig 5, 6: show anti-cancer activity of selected PDI-inhibitors *in vivo*

### Example 1. Chemical synthesis

It is described below the general method for the preparation of the aziridine aromatic N-sulphonamides of formula (I). 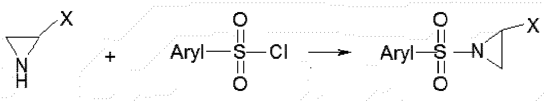 X=COOAlkyl, CONH₂, CONHAlkyl, CONAlkyl₂, CN, CHO, COAlkyl , 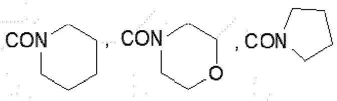 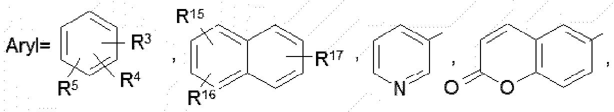 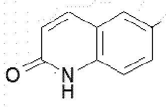 wherein R³, R⁴ and R⁵ are: H, linear alkyl group C₁-C₁₂, O-alkyl C₁-C₄, branched alkyl C₃-C₄, cycloalkyl, phenyl, NO₂, halogen (Cl, F), trifluoromethyl, C₁-C₄ alkoxy, C₁-C₄ dialkylamino, C₁-C₄ acylamino; and R¹⁵, R¹⁶, R¹⁷ are: H, alkyl C₁-C₄, Cl, O-alkyl C₁-C₄, -CHO and NR¹⁸R¹⁹ , where R¹⁸ and R¹⁹ are H or alkyl C₁-C₄

Aromatic or heteroaromatic sulphonic acid chloride (1 mmol) was added with stirring to the solution of the corresponding aziridine (1.1 mmol) and K₂CO₃ (2 mmol) in the mixture of 1 ml CHCl₃ + 1 ml water. The mixture was stirred for 24 h. at room temperature. Product was extracted with CHCl₃, and the solution dried over MgSO₄. The solvent was evaporated. The product was purified by chromatography (silica gel, petroleum ether/ethyl acetate 4:1=>1:2) to give corresponding aziridine aromatic N-sulfonamide.
1-(p-Tolylsulfonyl)aziridine-2-carbonitrile (C-3314) was prepared as described by Nadir, U. K. and Singh, A. Synthetic Communications, 34(7), 1337-1347; 2004.
1-(p-Tolylsulfonyl)aziridine-2-carbaldehyde (C-3262) was prepared as described by Lapinsky, D. J. and Bergmeier, S. C. Tetrahedron Letters, 42(49), 8583-8586; 2001.
1-(4-Butylphenyl)sulfonylaziridine-2-carbaldehyde (C-3273) was prepared using the same method.
1-[1-(p-Tolylsulfonyl)aziridin-2-yl]ethanone (C-3263) was prepared as described by Smith, A. B., and Kim, D.-S. Journal of Organic Chemistry, 71(7), 2547-2557; 2006.
1-[1-(4-Butylphenyl)sulfonylaziridin-2-yl]ethanone (C-3272) was prepared using the same method.
Methyl (2S)-1-(p-tolylsulfonyl)aziridine-2-carboxylate (C-3535) was prepared as described by Qian, G.; Bai, M.; Gao, S.; Chen, H.; Zhou, S.; Cheng, H-G.; Yan, W.; Zhou, Q. Angewandte Chemie, International Edition (2018), 57(34), 10980-10984.
Methyl (2R)-1-(p-tolylsulfonyl)aziridine-2-carboxylate (C-3539) was prepared as described by Smith, A. B. and Kim, D-S. Journal of Organic Chemistry (2006), 71(7), 2547-2557.
Methyl (2S)-1-[[6-(dimethylamino)-1-naphthyl]sulfonyl]aziridine-2-carboxylate (C-3548) and methyl (2R)-1-[[6-(dimethylamino)-1-naphthyl]sulfonyl]aziridine-2-carboxylate (C-3570) were prepared using methodology as described by Smith, A. B. and Kim, D-S. Journal of Organic Chemistry (2006), 71(7), 2547-2557.
Lithium 1-tosylaziridine-2-carboxylate (C-3612) was prepared as described by Baldwin, J. E.; Spivey, A. C.; Schofield, C. J.; Sweeney, J. B. Tetrahedron, 49(28), 6309-30; 1993

### Synthesis of 6-(dimethylamino)-5-formylnaphthalene-1-sulfonyl chloride.

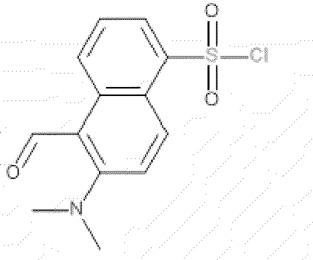

Into a 50 mL round-bottom flask, was placed 6-(dimethylamino)naphthalene-1-sulfonic acid (1.0 g, 3.98 mmol). To this was added CH₂Cl₂ (20 mL). To the mixture was added DMF (0.4 mL). To the above was added dropwise oxalyl dichloride (2.0 g, 15.74 mmol). The resulting solution was allowed to react with stirring for 24 h at room temperature. The reaction mixture was then quenched by the adding 50 mL of ice/salt. The resulting solution was extracted twice with 10 mL of CH₂Cl₂ and the organic layers combined and dried over Na₂SO₄. Solvent was evaporated under vacuum. The resulted 6-(dimethylamino)-5-formylnaphthalene-1-sulfonyl chloride (0.83 g 70%) was used on the next stage without additional purification. ¹H-NMR spectrum (400 MHz): (CDCl₃, HMDSO) δ: 10.30 (s, 1H), 9.55 (dt, *J*=8.7, 1.0 Hz, 1H), 8.87 (dd, *J*=9.7, 0.9 Hz, 1H), 8.18 (dd, *J*=7.6, 1.1 Hz, 1H), 7.66 (dd, *J*=8.7, 7.6 Hz, 1H), 7.60 (d, J=9.7 Hz, 1H), 3.22 (s, 6H).

The same method was used for the preparation of 6-(dimethylamino)-5-formylnaphthalene-2-sulfonyl chloride.

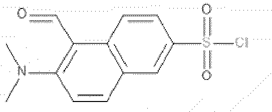

¹H-NMR spectrum (400 MHz): (CDCl₃, HMDSO) δ: 10.26 (s, 1H), 8.91 (d, *J*=9.1 Hz, 1H), 8.11 (d, *J*=9.1 Hz, 1H), 8.11 (m, 1H), 7.75 (dd, *J*=9.1, 2.0 Hz, 1H), 7.51 (d, *J*=9.1 Hz, 1H), 7.41 (d, *J*=9.1 Hz, 1H), 3.13 (s, 6H).

### Synthesis of 5-chloro-6-(methylamino)naphthalene-2-sulfonyl chloride.

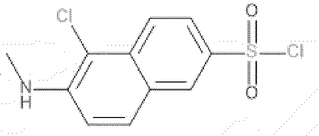

To a suspension of 6-(dimethylamino)naphthalene-2-sulfonic acid (1.0 g, 3.98 mmol). in POCl₃ (5 mL) was slowly added PCl₅ (3.7 g, 17.8 mmol). The resulting mixture was heated at 50 °C for 5 h before it was allowed to cool to room temperature and poured onto crushed ice. The aqueous mixture was stirred vigorously at 0 °C for 40 min. Product was extracted twice with 40 mL of CH₂Cl₂ and the organic layers combined and dried over Na₂SO₄. Solvent was evaporated under vacuum. The resulted 5-chloro-6-(methylamino)naphthalene-2-sulfonyl chloride was purified by chromatography (silica gel, petroleum ether/ethyl acetate 4:1). Yield 0.35 g (30%). ¹H-NMR spectrum (400 MHz): (CDCl₃, HMDSO) δ: 8.24 (d, *J=2.0* Hz, 1H), 8.15 (dt, *J*=9.2, 0.6 Hz, 1H), 7.95 (dd, *J*=9.2, 2.0 Hz, 1H), 7.86 (d, *J*=9.0 Hz, 1H), 7.22 (d, *J*=9.0 Hz, 1H), 3.11 (s, 3H).

**TABLE 1. Characteristics of aromatic sulfonamides**

| | |
|---|---|
| C-3161 | Methyl 1-(p-tolylsulfonyl)aziridine-2-carboxylate |
| | Molecular formula: C₁₁H₁₃NO₄S; Molecular weight: 255.29; Melting point: 56-58°C; ¹H-NMR spectrum (400 MHz): (CDCl₃, HMDSO) δ: 7.84 (d, J=8.3 Hz, 2H), 7.35 (d, J=8.3 Hz, 2H), 3.74 (s, 3H), 3.34 (dd, J=7.1, 4.1 Hz, 1H), 2.76 (d, J=7.1 Hz, 1H), 2.56 (d, J=4.1 Hz, 1H), 2.45 (s, 3H); ¹³C-NMR spectrum (100 MHz): (CDCl₃, HMDSO) δ: 167.2, 145.2, 134.0, 129.9, 128.2, 52.9, 35.7, 32.0, 21.7; LCMS ESI⁻ (m/z): 254.2 [M-H]⁻, LCMS purity 100.00% |
| C-3212 | Methyl 1-(4-nitrophenyl)sulfonylaziridine-2-carboxylate |
| | Molecular formula: C₁₀H₁₀N₂O₆S; Molecular weight: 286.26; Melting point: 106-108°C; ¹H-NMR spectrum (400 MHz): (CDCl₃, HMDSO) δ: 8.41 (d, *J*=9.0 Hz, 2H), 8.18 (d, *J*=9.0 Hz, 2H), 3.76 (s, 3H), 3.46 (dd, *J*=7.1, 4.3 Hz, 1H), 2.89 (d, *J*=7.1 Hz, 1H), 2.66 (d, *J*=4.3 Hz, 1H); ¹³C-NMR spectrum (100 MHz): (CDCl₃, HMDSO) δ: 166.6, 150.9, 142.9, 129.5, 124.5, 53.1, 35.2, 32.5; LCMS ESI⁻ (m/z): 285.1 [M-H]⁻, LCMS purity 100.00% |
| C-3216 | 1-(4-Nitrophenyl)sulfonylaziridine-2-carboxamide |
| | Molecular formula: C₉H₉N₃O₅S; Molecular weight: 271.25; Melting point: 179-181°C; ¹H-NMR spectrum (400 MHz): (DMSO-d6, HMDSO) δ: 8.47 (d, J=8.9 Hz, 2H), 8.24 (d, J=8.8 Hz, 2H), 7.87 (br s, 1H), 7.47 (br s, 1H), 3.39 (dd, J=7.2, 4.5 Hz, 1H), 2.79 (d, J=7.2 Hz, 1H), 2.58 (d, J=4.5 Hz, 1H); ¹³C-NMR spectrum (100 MHz): (DMSO-d6, HMDSO) δ: 166.5, 151.2, 142.6, 130.0, 125.3, 37.6, 32.2; LCMS ESI⁺ (m/z): 271.9 [M+H]⁺; Impurities: 3.67% (210 nm); 2.24% (254 nm) |
| C-3218 | 1-(4-Aminophenyl)sulfonylaziridine-2-carboxamide |
| | Molecular formula: C₉H₁₁N₃O₃S; Molecular weight: 241.27; Melting point: 139-141°C; ¹H-NMR spectrum (400 MHz): (CDCl₃, HMDSO) δ: 7.72 (s, 1H), 7.50 (d, *J*=8.7 Hz, 2H), 7.34 (s, 1H), 6.65 (d, *J*=8.7 Hz, 2H), 6.25 (s, 2H), 3.01 (dd, *J*=7.1, 4.1 Hz, 1H), 2.41 (d, *J*=7.1 Hz, 1H), 2.35 (d, *J*=4.1 Hz, 1H); ¹³C-NMR spectrum (100 MHz): (DMSO-d₆, HMDSO) δ: 167.5, 154.6, 130.5, 120.5, 113.2, 36.8, 31.2; LCMS ESI⁻ (m/z): 242.2 [M-H]⁻, LCMS purity 100.00% |
| C-3220 | 1-(p-Tolylsulfonyl)aziridine-2-carboxamide |
| | Molecular formula: C₁₀H₁₂N₂O₅S; Molecular weight: 240.28; Melting point: 118-120°C; ¹H-NMR spectrum (400 MHz): (CDCl₃, HMDSO) δ: 7.82 (d, *J*=8.2 Hz, 2H), 7.37 (d, *J*=8.2 Hz, 2H), 6.11 (s, 1H), 5.76 (s, 1H), 3.23 (dd, *J*=7.7, 4.2 Hz, 1H), 2.80 (d, *J*=7.7 Hz, 1H), 2.48 (s, 3H), 2.43 (d, *J*=4.2 Hz, 1H); ¹³C-NMR spectrum (100 MHz): (CDCl₃, HMDSO) δ: 168.3, 145.6, 133.4, 130.0, 128.2, 37.6, 33.1, 21.7; LCMS ESI⁻ (m/z): 241.2 [M-H]⁻, LCMS purity 100.00% |
| C-3251 | Methyl 1-(benzenesulfonyl)aziridine-2-carboxylate |
| | Molecular formula: C₁₀H₁₁NO₄S; Molecular weight: 241.26; Melting point: oil; ¹H-NMR spectrum (400 MHz): (CDCl₃) δ: 7.00-8.94 (m, 2H), 7.71-7.64 (m, 1H), 7.60-7.53 (m, 2H), 3.74 (s, 3H), 3.37 (dd, J=7.1, 4.1 Hz, 1H), 2.79 (d, J=7.1 Hz, 1H), 2.58 (d, J=4.1 Hz, 1H); ¹³C-NMR spectrum (100 MHz): (CDCl₃) δ: 167.3, 137.2, 134.3, 129.4, 128.3, 53.1, 35.9, 32.3; LCMS ESI⁺ (m/z): 242.1 [M+H]⁺, LCMS purity 100.00% |
| C-3256 | Methyl 1-[4-(trifluoromethyl)phenyl]sulfonylaziridine-2-carboxylate |
| | Molecular formula: C₁₁H₁₀F₃NO₄S; Molecular weight: 309.26; Melting point: 68-70°C; ¹H-NMR spectrum (400 MHz): (CDCl₃, HMDSO) δ: 8.12 (d, *J*=8.4 Hz, 2H), 7.84 (d, *J*=8.4 Hz, 2H), 3.76 (s, 3H), 3.43 (dd, *J*=7.1, 4.2 Hz, 1H), 2.85 (dd, *J*=7.1, 0.9 Hz, 1H), 2.63 (dd, *J*=4.2, 0.9 Hz, 1H); ¹³C-NMR spectrum (100 MHz): (CDCl₃, HMDSO) δ: 166.8, 140.8, 135.7 (q, *J*=33.1 Hz), 128.7, 126.4 (q, *J*=3.7 Hz), 123.0 (q, J=273.4 Hz), 53.0, 36.0, 32.3; LCMS ESI⁻ (m/z): 308.2 [M-H]⁻, LCMS purity 100.00% |
| C-3257 | Methyl 1-(4-butylphenyl)sulfonylaziridine-2-carboxylate |
| | Molecular formula: C₁₄H₁₉NO₄S; Molecular weight: 297.37; Melting point: oil; ¹H-NMR spectrum (400 MHz): (CDCl₃, HMDSO) δ: 7.86 (d, J=8.4 Hz, 2H), 7.36 (d, J=8.4 Hz, 2H), 3.74 (s, 3H), 3.35 (dd, J=7.1, 4.1 Hz, 1H), 2.76 (d, J=7.1 Hz, 1H), 2.70 (t, J=7.7 Hz, 2H), 2.56 (d, J=4.1 Hz, 1H), 1.58-1.67 (m, 2H), 1.36 (sextet, J=7.5 Hz, 2H), 0.94 (d, J=7.4 Hz, 3H); ¹³C-NMR spectrum (100 MHz): (CDCl₃, HMDSO) δ: 167.3, 150.1, 134.1, 129.2, 128.2, 52.9, 35.6, 35.6, 32.1, 32.1, 22.2, 13.8; LCMS ESI⁺ (m/z): 298.2 [M+H]⁺, LCMS purity 100% |
| C-3262 | 1-(p-Tolylsulfonyl)aziridine-2-carbaldehyde |
| | Molecular formula: C₁₀H₁₁NO₄S; Molecular weight: 225.26; Melting point: oil; ¹H-NMR spectrum (400 MHz): (CDCl₃, HMDSO) δ: 8.95 (d, *J*=5.9 Hz, 1H), 7.84 (d, *J*=8.3 Hz, 2H), 7.38 (d, *J*=8.3 Hz, 2H), 3.27 (ddd, *J*=7.4, 5.9, 4.0 Hz, 1H), 2.93 (d, *J*=7.4 Hz, 1H), 2.58 (d, *J*=4.0 Hz, 1H), 2.47 (s, 3H); ¹³C-NMR spectrum (100 MHz): (CDCl₃, HMDSO) δ: 194.3, 145.6, 133.7, 130.0, 128.2, 41.9, 30.1, 21.7; LCMS ESI⁺ (m/z): 226.2 [M+H]⁺, LCMS purity 94.70% |
| C-3263 | 1-[1-(p-Tolylsulfonyl)aziridin-2-yl]ethanone |
| | Molecular formula: C₁₁H₁₃NO₃S; Molecular weight: 239.29; Melting point: 90-92°C; ¹H-NMR spectrum (400 MHz): (CDCl₃, HMDSO) δ: 7.84 (d, J=8.3 Hz, 2H), 7.37 (d, J=8.3 Hz, 2H), 3.29 (dd, J=7.4, 4.1 Hz, 1H), 2.80 (d, J=7.1 Hz, 1H), 2.49 (d, J=4.1 Hz, 1H), 2.46 (s, 3H), 2.07 (s, 3H); ¹³C-NMR spectrum (100 MHz): (CDCl₃, HMDSO) δ: 201.4, 145.3, 133.9, 129.9, 128.1, 41.9, 31.8, 25.9, 21.7; LCMS ESI+ (m/z): 240.2 [M+H]+, LCMS purity 100%; Elemental analysis: Found [%]: C 55.07, H 5.35, N 5.74; Calculated [%]: C 55.21, H 5.48, N 5.85 |
| C-3270 | Methyl 1-(4-dodecylphenyl)sulfonylaziridine-2-carboxylate |
| | Molecular formula: C₂₂H₃₅NO₄S; Molecular weight: 409.58; Melting point: oil; ¹H-NMR spectrum (400 MHz): (CDCl₃, HMDSO) δ: 7.86 (d, *J*=8.4 Hz, 2H), 7.35 (d, *J*=8.4 Hz, 2H), 3.74 (s, 3H), 3.36 (dd, *J*=7.1, 4.1 Hz, 1H), 2.77 (d, *J*=7.1 Hz, 1H), 2.69 (t, *J*=7.7 Hz, 2H), 2.56 (d, *J*=4.1 Hz, 1H), 1.63 (quintet, *J*=7.5 Hz, 2H), 1.20-1.38 (m, 18H), 0.87 (d, *J*=6.9 Hz, 3H); ¹³C-NMR spectrum (100 MHz): (CDCl₃, HMDSO) δ: 167.3, 150.2, 134.1, 129.2, 128.2, 52.9, 36.0, 35.6, 32.1, 31.9, 29.6, 29.5, 29.4, 29.3, 29.2, 22.7, 14.1; LCMS ESI⁺ (m/z): 410.4 [M+H]⁺, LCMS purity 100.00% |
| C-3271 | Methyl 1-(4-butoxyphenyl)sulfonylaziridine-2-carboxylate |
| | Molecular formula: C₁₄H₁₉NO₃S; Molecular weight: 313.37; Melting point: oil; ¹H-NMR spectrum (400 MHz): (CDCl₃, HMDSO) δ: 7.87 (d, J=8.4 Hz, 2H), 7.00 (d, J=8.4 Hz, 2H), 4.04 (t, J=6.5 Hz, 2H), 3.74 (s, 3H), 3.32 (dd, J=7.1, 4.1 Hz, 1H), 2.74 (d, J=7.1 Hz, 1H), 2.55 (d, J=4.1 Hz, 1H), 1.80 (quintet, J=6.7 Hz, 2H), 1.50 (sextet, J=7.5 Hz, 2H), 0.99 (t, J=7.4 Hz, 3H); ¹³C-NMR spectrum (100 MHz): (CDCl₃, HMDSO) δ: 167.3, 163.7, 130.4, 127.9, 114.9, 68.3, 52.8, 35.6, 32.0, 30.9, 19.1, 13.7; LCMS ESI⁺ (m/z): 314.3 [M+H]⁺; LCMS purity 93.03% |
| C-3272 | 1-[1-(4-Butylphenyl)sulfonylaziridin-2-yl]ethanone |
| | Molecular formula: C₁₄H₁₉NO₃S; Molecular weight: 281.37; Melting point: oil; ¹H-NMR spectrum (400 MHz): (CDCl₃, HMDSO) δ: 7.84 (d, J=8.4 Hz, 2H), 7.36 (d, J=8.3 Hz, 2H), 3.29 (dd, J=7.4, 4.1 Hz, 1H), 2.70 (t, J=7.7 Hz, 2H), 2.79 (d, J=7.4 Hz, 1H), 2.49 (d, J=4.1 Hz, 1H), 2.46 (s, 3H), 2.07 (s, 3H), 1.57-1.67 (m, 2H), 1.36 (sextet, J=7.5 Hz, 2H), 0.93 (t, J=7.4 Hz, 3H); ¹³C-NMR spectrum (100 MHz): (CDCl₃, HMDSO) δ: 201.5, 150.2, 134.0, 129.3, 128.2, 41.9, 35.6, 33.0, 31.9, 25.9, 22.2, 13.8; LCMS ESI+ (m/z): 282.3 [M+H]⁺ , LCMS purity 96.65% |
| C-3273 | 1-(4-Butylphenyl)sulfonylaziridine-2-carbaldehyde |
| | Molecular formula: C₁₃H₁₇NO₃S; Molecular weight: 267.34; Melting point: oil; ¹H-NMR spectrum (400 MHz): (CDCl₃, HMDSO) δ: 8.96 (d, *J*=5.8 Hz, 1H), 7.85 (d, *J*=8.4 Hz, 2H), 7.38 (d, *J*=8.3 Hz, 2H), 3.28 (ddd, *J*=7.4, 5.8, 4.0 Hz, 1H), 2.93 (d, *J*=7.4 Hz, 1H), 2.71 (t, *J*=7.8 Hz, 2H), 2.58 (d, J=4.0 Hz, 1H), 1.57-1.68 (m, 2H), 1.37 (sextet, J=7.5 Hz, 2H), 0.94 (t, *J*=7.5 Hz, 3H); ¹³C-NMR spectrum (100 MHz): (CDCl₃, HMDSO) δ: 194.3, 150.4, 133.8, 129.4, 128.2, 41.8, 35.6, 33.0, 30.1, 22.2, 13.8; LCMS ESI⁺ (m/z): 268.4 [M+H]⁺, LCMS purity 100.00% |
| C-3281 | Methyl 1-(4-pentylphenyl)sulfonylaziridine-2-carboxylate |
| | Molecular formula: C₁₅H₂₁NO₄S; Molecular weight: 311.40; Melting point: oil; ¹H-NMR spectrum (400 MHz): (CDCl₃, HMDSO) δ: (CDCl₃, HMDSO) δ: 7.86 (d, *J*=8.4 Hz, 2H), 7.39 (d, J=8.4 Hz, 2H), 3.74 (s, 3H), 3.36 (dd, *J*=7.1, 4.1 Hz, 1H), 2.77 (d, *J*=7.1 Hz, 1H), 2.69 (t, *J*=7.8 Hz, 2H), 2.56 (d, *J*=4.1 Hz, 1H), 1.64 (quintet, *J*=7.5 Hz, 2H), 1.27-1.40 (m, 4H), 0.90 (t, *J*=7.0 Hz, 3H); ¹³C-NMR spectrum (100 MHz): (CDCl₃, HMDSO) δ: 167.2, 150.1, 134.1, 129.2, 128.2, 52.8, 35.8, 35.6, 32.1, 31.3, 30.6, 22.4, 13.9; LCMS ESI⁺ (m/z): 312.4 [M+H]⁺, LCMS purity 100.00% |
| C-3287 | Methyl 1-(4-hexylphenyl)sulfonylaziridine-2-carboxylate |
| | Molecular formula: C₁₆H₂₃NO₄S; Molecular weight: 325.42; Melting point: oil; ¹H-NMR spectrum (400 MHz): (CDCl₃) δ: 7.88-7.83 (m, 2H), 7.38-7.32 (m, 2H), 3.74 (s, 3H), 3.35 (dd, J=7.1, 4.1 Hz, 1H), 2.76 (d, J=7.1 Hz, 1H), 2.73-2.65 (m, 2H), 2.56 (d, J=4.1 Hz, 1H), 1.68-1.58 (m, 2H), 1.38-1.24 (m, 6H), 0.92-0.84 (m, 3H); ¹³C-NMR spectrum (100 MHz): (CDCl₃) δ: 167.4, 150.3, 134.3, 129.4, 128.4, 53.0, 36.1, 35.8, 32.2, 31.7, 31.1, 29.0, 22.7, 14.2; LCMS ESI+ (m/z): 326.2 [M+H]+, LCMS purity 99.43% |
| C-3288 | Methyl 1-(4-heptylphenyl)sulfonylaziridine-2-carboxylate |
| | Molecular formula: C₁₇H₂₅NO₄S; Molecular weight: 339.45; Melting point: oil; ¹H-NMR spectrum (400 MHz): (CDCl₃, HMDSO) δ: 7.86 (d, *J*=8.5 Hz, 2H), 7.36 (d, *J*=8.5 Hz, 2H), 3.74 (s, 3H), 3.36 (dd, *J*=7.1, 4.1 Hz, 1H), 2.77 (d, *J*=7.1 Hz, 1H), 2.69 (t, *J*=7.7 Hz, 2H), 2.56 (d, *J*=4.1 Hz, 1H), 1.63 (quintet, *J*=7.5 Hz, 2H), 1.22-1.37 (m, 8H), 0.88 (t, *J*=7.1 Hz, 3H); ¹³C-NMR spectrum (100 MHz): (CDCl₃, HMDSO) δ: 167.3, 150.1, 134.1, 129.2, 128.2, 52.9, 35.9, 35.6, 32.1, 31.7, 31.0, 29.1, 29.0, 22.6, 14.0; LCMS ESI⁺ (m/z): 340.4 [M+H]⁺, LCMS purity 100.00% |
| C-3290 | Methyl 1-(4-tert-butylphenyl)sulfonylaziridine-2-carboxylate |
| | Molecular formula: C₁₄H₁₉NO₄S; Molecular weight: 297.37; Melting point: oil; ¹H-NMR spectrum (400 MHz): (CDCl₃, HMDSO) δ: 7.88 (d, *J*=8.6 Hz, 2H), 7.57 (d, *J*=8.6 Hz, 2H), 3.74 (s, 3H), 3.37 (dd, *J*=7.1, 4.1 Hz, 1H), 2.76 (d, *J*=7.1 Hz, 1H), 2.56 (d, *J*=4.1 Hz, 1H), 1.35 (s, 9H); ¹³C-NMR spectrum (100 MHz): (CDCl₃, HMDSO) δ: 167.3, 158.1, 133.9, 128.0, 126.3, 52.9, 35.6, 35.3, 32.1, 31.0; LCMS ESI⁺ (m/z): 398.3 [M+H]⁺, LCMS purity 100.00% |
| C-3291 | Methyl 1-(4-phenylphenyl)sulfonylaziridine-2-carboxylate |
| | Molecular formula: C₁₆H₁₃NO₄S; Molecular weight: 317.36; Melting point: 90-92°C; ¹H-NMR spectrum (400 MHz): (CDCl₃, HMDSO) δ: 8.03 (d, *J*=8.8 Hz, 2H), 7.77 (d, *J*=8.8 Hz, 2H), 7.60-7.62 (m, 2H), 7.41-7.52 (m, 3H), 3.76 (s, 3H), 3.41 (dd, J=7.1, 4.1 Hz, 1H), 2.82 (d, J=7.1 Hz, 1H), 2.61 (d, *J*=4.1 Hz, 1H); ¹³C-NMR spectrum (100 MHz): (CDCl₃, HMDSO) δ: 167.2, 147.1, 139.0, 135.5, 129.1, 128.7, 128.7, 127.9, 127.4, 52.9, 35.7, 32.1; LCMS ESI⁺ (m/z): 318.3 [M+H]⁺, LCMS purity 100.00% |
| C-3292 | Methyl 1-(2-naphthylsulfonyl)aziridine-2-carboxylate |
| | Molecular formula: C₁₄H₁₃NO₄S; Molecular weight: 291.32; Melting point: oil; ¹H-NMR spectrum (400 MHz): (CDCl₃, HMDSO) δ: 8.52-8.54 (m, 1H), 7.99-8.03 (m, 2H), 7.93-7.97 (m, 2H), 7.67-7.72 (m, 1H), 7.62-7.67 (m, 1H), 3.73 (s, 3H), 3.44 (dd, J=7.1, 4.1 Hz, 1H), 2.85 (d, J=7.1 Hz, 1H), 2.60 (d, J=4.1 Hz, 1H); ¹³C-NMR spectrum (100 MHz): (CDCl₃, HMDSO) δ: 167.1, 135.5, 133.9, 132.0, 129.9, 129.6, 129.5, 129.5, 128.0, 127.8, 122.8, 52.9, 35.8, 32.1; LCMS ESI+ (m/z): 292.3 [M+H]+ , LCMS purity 97.48% |
| C-3294 | Methyl 1-(4-methoxyphenyl)sulfonylaziridine-2-caiboxylate |
| | Molecular formula: C₁₁H₁₃NO₅S; Molecular weight: 271.29; Melting point: 73-75 °C; ¹H-NMR spectrum (400 MHz): (CDCl₃, HMDSO) δ: 7.89 (d, J=9.1 Hz, 2H), 7.01 (d, J=9.1 Hz, 2H), 3.89 (s, 3H), 3.74 (s, 3H), 3.32 (dd, J=7.1, 4.1 Hz, 1H), 2.75 (d, J=7.1 Hz, 1H), 2.55 (d, J=4.1 Hz, 1H); ¹³C-NMR spectrum (100 MHz): (CDCl₃, HMDSO) δ: 167.3, 164.1, 130.4, 128.3, 114.5, 55.7, 52.8, 35.7, 32.0; LCMS ESI+ (m/z): 272.3 [M+H]+, LCMS purity 100% |
| C-3295 | Methyl 1-(4-isopropylphenyl)sulfonylaziridine-2-carboxylate |
| | Molecular formula: C₁₃H₁₇NO₄S; Molecular weight: 283.34; Melting point: oil; ¹H-NMR spectrum (400 MHz): (CDCl₃, HMDSO) δ: 7.88 (d, J=8.6 Hz, 2H), 7.40 (d, J=8.6 Hz, 2H), 3.75 (s, 3H), 3.36 (dd, J=7.1, 4.1 Hz, 1H), 3.00 (septet, J=6.9 Hz, 1H), 2.76 (d, J=7.1 Hz, 1H), 2.56 (d, J=4.1 Hz, 1H), 1.28 (d, J=6.9 Hz, 6H); ¹³C-NMR spectrum (100 MHz): (CDCl₃, HMDSO) δ: 167.3, 155.9, 134.2, 128.3, 127.4, 52.8, 35.6, 34.2, 32.1, 23.5; LCMS ESI+ (m/z): 284.3 [M+H]+, LCMS purity 100% |
| C-3296 | Methyl 1-(4-chlorophenyl)sulfonylaziridine-2-carboxylate |
| | Molecular formula: C₁₀H₁₀ClNO₄S; Molecular weight: 275.71; Melting point: oil; ¹H-NMR spectrum (400 MHz): (CDCl₃, HMDSO) δ: 7.91 (d, *J*=8.7 Hz, 2H), 7.55 (d, *J*=8.7 Hz, 2H), 3.75 (s, 3H), 3.38 (dd, *J*=7.1, 4.1 Hz, 1H), 2.80 (d, *J*=7.1 Hz, 1H), 2.60 (d, *J*=4.1 Hz, 1H); ¹³C-NMR spectrum (100 MHz): (CDCl₃, HMDSO) δ: 166.9, 140.9, 135.6, 129.6, 129.6, 53.0, 35.9, 32.2; LCMS ESI⁺ (m/z): 276.2 [M+H]⁺, LCMS purity 100.00% |
| C-3297 | Methyl 1-(2-fluoro-4-methyl-phenyl)sulfonylaziridine-2-carboxylate |
| | Molecular formula: C₁₁H₁₂FNO₄S; Molecular weight: 273.26; Melting point: oil; ¹H-NMR spectrum (400 MHz): (CDCl₃, HMDSO) δ: 7.82 (dd, *J*=8.0, 7.3 Hz, 1H), 7.10-7.13 (m, 1H), 7.04-7.08 (m, 1H), 3.77 (s, 3H), 3.50 (ddd, *J*=7.1, 4.3, 0.9 Hz, 1H), 2.95 (dd, *J*=7.1, 1.2 Hz, 1H), 2.66 (d, *J*=4.3 Hz, 1H), 2.44 (s, 3H); ¹³C-NMR spectrum (100 MHz): (CDCl₃, HMDSO) δ: 167.2, 159.3 (d, *J*=258.3 Hz,), 148.8 (d, *J*=8.5 Hz,), 130.3, 125.2 (d, *J*=3.0 Hz,), 122.4 (d, *J*=14.5 Hz,), 117.8 (d, *J*=20.9 Hz,),, 52.9, 35.9, 32.4, 21.6; LCMS ESI⁺ (m/z): 274.3 [M+H]⁺, LCMS purity 96.13% |
| C-3299 | Methyl 1-(4-propylphenyl)sulfonylaziridine-2-carboxylate |
| | Molecular formula: C₁₃H₁₇NO₄S; Molecular weight: 283.34; Melting point: oil; ¹H-NMR spectrum (400 MHz): (CDCl₃, HMDSO) δ: 7.86 (d, *J*=8.5 Hz, 2H), 7.36 (d, *J*=8.5 Hz, 2H), 3.74 (s, 3H), 3.36 (dd, *J*=7.1, 4.1 Hz, 1H), 2.77 (d, *J*=7.1 Hz, 1H), 2.68 (t, *J*=7.6 Hz, 2H), 2.56 (d, *J*=4.1 Hz, 1H), 1.68 (sextet, *J*=7.5 Hz, 2H), 0.95 (t, *J*=7.4 Hz, 3H); ¹³C-NMR spectrum (100 MHz): (CDCl₃, HMDSO) δ: (CDCl₃, HMDSO) δ: 167.2, 149.8, 134.2, 129.3, 128.2, 52.9, 37.9, 35.6, 32.1, 24.1, 13.6; LCMS ESI⁺ (m/z): 284.3 [M+H]⁺, LCMS purity 100.00% |
| C-3303 | Methyl 1-(m-tolylsulfonyl)aziridine-2-carboxylate |
| | Molecular formula: C₁₁H₁₃NO₄S; Molecular weight: 255.29; Melting point: oil; ¹H-NMR spectrum (400 MHz): (CDCl₃, HMDSO) δ: 7.88-7.80 (m, 1H), 7.74-7.77 (m, 1H), 7.46-7.50 (m, 1H), 7.42-7.46 (m, 1H), 3.74 (s, 3H), 3.37 (dd, *J*=7.1, 4.1 Hz, 1H), 2.78 (d, *J*=7.1 Hz, 1H), 2.57 (d, *J*=4.1 Hz, 1H), 2.45 (s, 3H); ¹³C-NMR spectrum (100 MHz): (CDCl₃, HMDSO) δ: 167.2, 139.6, 136.8, 135.0, 129.1, 128.5, 125.2, 52.9, 35.6, 32.1, 24.3; LCMS ESI⁺ (m/z): 256.2 [M+H]⁺, LCMS purity 97.26% |
| C-3304 | Methyl 1-(4-propylphenyl)sulfonylaziridine-2-carboxylate |
| | Molecular formula: C₁₃H₁₇NO₄S; Molecular weight: 283.34; Melting point:106-108°C ¹H-NMR spectrum (400 MHz): (CDCl₃, HMDSO) δ: 6.98 (s, 2H), 3.74 (s, 3H), 3.34 (dd, J=7.1, 4.1 Hz, 1H), 2.79 (d, J=7.1 Hz, 1H), 2.69 (s, 6H), 2.51 (d, J=4.1 Hz, 1H), 2.32 (s, 3H); ¹³C-NMR spectrum (100 MHz): (CDCl₃, HMDSO) δ: 167.5, 143.6, 140.4, 131.9, 131.7, 52.7, 34.7, 31.7, 22.9, 21.0 |
| | LCMS ESI+ (m/z): 284.3 [M+H]+ , LCMS purity 100% |
| C-3305 | Methyl 1-[3-(trifluoromethyl)phenyl]sulfonylaziridine-2-carboxylate |
| | Molecular formula: C₁₁H₁₀F₃NO₄S; Molecular weight: 309.26; Melting point: oil; ¹H-NMR spectrum (400 MHz): (CDCl₃, HMDSO) δ: 8.23-8.26 (m, 1H), 8.16-8.20 (m, 1H), 7.92-7.96 (m, 1H), 7.71-7.77 (m, 1H), 3.76 (s, 3H), 3.45 (dd, *J*=7.1, 4.2 Hz, 1H), 2.86 (dd, *J*=7.1, 0.9 Hz, 1H), 2.64 (dd, *J*=4.2, 0.9 Hz, 1H); ¹³C-NMR spectrum (100 MHz): (CDCl₃, HMDSO) δ: 166.8, 138.5, 132.0 (q, *J*=34.1 Hz), 131.4, 130.8 (q, *J*=3.6 Hz), 130.1, 125.2 (q, *J*=3.8 Hz), 123.0 (q, *J*=273.8 Hz), 53.0, 36.0, 32.4; LCMS ESI⁻ (m/z): 310.2 [M+H]⁺, LCMS purity 100.00% |
| C-3308 | Methyl 1-(4-acetamidophenyl)sulfonylaziridine-2-carboxylate |
| | Molecular formula: C₁₂H₁₄N₂O₅S; Molecular weight: 298.31; Melting point: oil; ¹H-NMR spectrum (400 MHz): (CDCl₃, HMDSO) δ: (CDCl₃, HMDSO) δ: 7.73-7.95 (m, 3H), 7.67 (d, *J*=8.9 Hz, 2H), 3.74 (s, 3H), 3.43 (dd, *J*=7.1, 4.1 Hz, 1H), 2.76 (d, *J*=7.1 Hz, 1H), 2.56 (d, *J=4.1* Hz, 1H), 2.21 (s, 3H); ¹³C-NMR spectrum (100 MHz): (CDCl₃, HMDSO) δ: 168.9, 167.4, 143.4, 131.0, 129.5, 119.3, 53.0, 35.7, 32.1, 24.7; LCMS ESI⁺ (m/z): 299.2 [M+H]⁺, LCMS purity 94.91% |
| C-3311 | Methyl 1-(1-naphthylsulfonyl)aziridine-2-carboxylate |
| | Molecular formula: C₁₄H₁₃NO₄S; Molecular weight: 291.32; Melting point: 102-104°C; ¹H-NMR spectrum (400 MHz): (CDCl₃, HMDSO) δ: 8.90 (ddd, J=8.6, 1.8, 0.8 Hz, 1H), 8.24 (dd, J=7.4, 1.3 Hz, 1H), 8.15 (dt, J=8.2, 1.2 Hz, 1H), 7.95 (ddd, J=8.2, 1.3, 0.6 Hz, 1H), 7.73 (ddd, J=8.2, 6.9, 1.3 Hz, 1H), 7.63 (ddd, J=8.2, 6.9, 1.2 Hz, 1H), 7.57 (dd, J=8.2, 7.4 Hz, 1H), 3.70 (s, 3H), 3.49 (dd, J=7.1, 4.1 Hz, 1H), 2.89 (d, J=7.1 Hz, 1H), 2.59 (d, J=4.1 Hz, 1H); ¹³C-NMR spectrum (100 MHz): (CDCl₃, HMDSO) δ: 167.1, 135.7, 134.2, 132.6, 130.0, 129.0, 128.7, 128.6, 127.2, 125.5, 124.0, 52.8, 35.9, 32.5; LCMS ESI+ (m/z): 292.3 [M+H]+, LCMS purity 98.48% |
| C-3314 | 1-(p-Tolylsulfonyl)aziridine-2-carbonitrile |
| | Molecular formula: C₁₀H₁₀N₂O₂S; Molecular weight: 222.26; Melting point: 83-85°C; ¹H-NMR spectrum (400 MHz): (CDCl₃, HMDSO) δ: 7.85 (d, J=8.4 Hz, 2H), 7.41 (d, J=8.4 Hz, 2H), 3.22 (dd, J=7.0, 4.0 Hz, 1H), 2.89 (d, J=7.0 Hz, 1H), 2.65 (d, J=4.0 Hz, 1H), 2.49 (s, 3H); ¹³C-NMR spectrum (100 MHz): (CDCl₃, HMDSO) δ: 146.1, 133.1, 130.2, 128.2, 115.0, 32.1, 23.4, 21.7; LCMS ESI- (m/z): 221.2 [M-H]-, LCMS purity 100.00% |
| C-3316 | Methyl 1-(4-cyclohexylphenyl)sulfonylaziridine-2-carboxylate |
| | Molecular formula: C₁₆H₂₁NO₄S; Molecular weight: 323.12; Melting point: oil; ¹H-NMR spectrum (400 MHz): (CDCl₃, HMDSO) δ: 7.87 (d, *J*=8.5 Hz, 2H), 7.38 (d, *J*=8.5 Hz, 2H), 3.74 (s, 3H), 3.36 (dd, *J*=7.1, 4.1 Hz, 1H), 2.76 (d, *J*=7.1 Hz, 1H), 2.56-2.65 (m, 1H), 2.56 (d, *J*=4.1 Hz, 1H), 1.82-1.94 (m, 4H), 1.73-1.82 (m, 1H), 1.34-1.50 (m, 4H), 1.20-1.33 (m, 1H); ¹³C-NMR spectrum (100 MHz): (CDCl₃, HMDSO) δ: 167.3, 155.0, 134.1, 128.3, 127.7, 52.8, 44.6, 35.5, 34.0, 32.1, 26.6, 25.9; LCMS ESI⁺ (m/z): 324.3 [M+H]⁺, LCMS purity 98.89% |
| C-3319 | Methyl 1-(4-octylphenyl)sulfonylaziridine-2-carboxylate |
| | Molecular formula: C₁₈H₂₇NO₄S; Molecular weight: 353.48; Melting point: oil; ¹H-NMR spectrum (400 MHz): (CDCl₃, HMDSO) δ: 7.86 (d, *J*=8.5 Hz, 2H), 7.36 (d, *J*=8.5 Hz, 2H), 3.74 (s, 3H), 3.36 (dd, *J*=7.1, 4.1 Hz, 1H), 2.77 (d, *J*=7.1 Hz, 1H), 2.69 (t, *J*=7.7 Hz, 2H), 2.56 (d, *J*=4.1 Hz, 1H), 1.63 (quintet, *J*=7.6 Hz, 2H), 1.22-1.36 (m, 10H), 0.88 (t, *J*=7.1 Hz, 3H); ¹³C-NMR spectrum (100 MHz): (CDCl₃, HMDSO) δ: 167.3, 150.1, 134.1, 129.2, 128.2, 52.8, 35.9, 35.6, 32.1, 31.8, 31.0, 29.3, 29.1, 29.0, 22.6, 14.0; LCMS ESI⁺ (m/z): 354.4 [M+H]⁺, LCMS purity 100.00% |
| C-3320 | Methyl 1-(o-tolylsulfonyl)aziridine-2-carboxylate |
| | Molecular formula: C₁₁H₁₃NO₄S; Molecular weight: 255.29; Melting point: oil; ¹H-NMR spectrum (400 MHz): (CDCl₃, HMDSO) δ: 7.94 (dd, *J*=7.8, 1.4 Hz, 1H), 7.53 (dt, *J*=7.5, 1.4 Hz, 1H), 7.32-7.39 (m, 2H), 3.75 (s, 3H), 3.38 (dd, *J*=7.1, 4.1 Hz, 1H), 2.83 (d, *J*=7.1 Hz, 1H), 2.79 (s, 3H), 2.55 (d, *J*=4.1 Hz, 1H); ¹³C-NMR spectrum (100 MHz): (CDCl₃, HMDSO) δ: 167.3, 139.4, 135.6, 134.0, 132.7, 129.5, 126.1, 52.8, 35.4, 32.2, 20.6; LCMS ESI⁺ (m/z): 256.2 [M+H]⁺, LCMS purity 100.00% |
| C-3324 | 1-[4-(Trifluoromethyl)phenyl]sulfonylaziridine-2-carboxamide |
| | Molecular formula: C₁₀H₉F₃N₂O₅S; Molecular weight: 294.25; Melting point: 138-140°C; ¹H-NMR spectrum (400 MHz): (DMSO-d₆, HMDSO) δ: 8.19 (d, *J*=8.3 Hz, 2H), 8.08 (d, *J*=8.3 Hz, 2H), 7.86 (br s, 1H), 7.45 (br s, 1H), 3.36 (dd, *J*=7.2, 4.5 Hz, 1H), 2.76 (d, *J*=7.2*,* 1H), 2.56 (d, *J*=4.5*,* 1H); ¹³C-NMR spectrum (100 MHz): (DMSO-d₆, HMDSO) δ: 166.6, 141.2, 133.9 (q, *J*=32.7 Hz), 129.2, 127.3 (q, *J*=3.6 Hz), 123.6 (q, *J*=273.4 Hz), 37.5, 32.0; LCMS ESI⁺ (m/z): 295.2 [M+H]⁺, LCMS purity 100.00% |
| C-3326 | Methyl 1-[[5-(dimethylamino)-1-naphthyl]sulfonyl]aziridine-2-carboxylate |
| | Molecular formula: C₁₆H₁₈N₂O₄S; Molecular weight: 334.39; Melting point: 117-119°C; ¹H-NMR spectrum (400 MHz): (CDCl₃, HMDSO) δ: 8.63 (dt, *J*=8.5, 1.1 Hz, 1H), 8.55 (dt, *J*=8.7, 0.9 Hz, 1H), 8.22 (dd, *J*=7.4, 1.3 Hz, 1H), 7.61 (dd, *J*=8.7, 7.6 Hz, 1H), 7.54 (dd, *J*=8.5, 7.4 Hz, 1H), 7.21 (dd, *J*=7.6, 0.9 Hz, 1H), 3.74 (s, 3H), 3.49 (dd, *J=7*.1*,* 4.2 Hz, 1H), ), 2.89 (s, 6H), 2.87 (d, *J*=7.1 Hz, 1H), 2.58 (d, *J*=4.2 Hz, 1H); ¹³C-NMR spectrum (100 MHz): (CDCl₃, HMDSO) δ: 167.2, 151.7, 132.7, 131.9, 130.4, 129.9, 129.9, 128.7, 123.0, 120.0, 115.5, 52.8, 45.4, 35.8, 32.5; LCMS ESI⁺ (m/z): 335.1 [M+H]⁺, LCMS purity 100.00% |
| C-3327 | Methyl 1-[(6-methoxy-2-naphthyl)sulfonyl]aziridine-2-carboxylate |
| | Molecular formula: C₁₃H₁₃NO₃S; Molecular weight: 321.35; Melting point: 97-99°C; ¹H-NMR spectrum (400 MHz): (CDCl₃, HMDSO) δ: 8.42-8.44 (m, 1H), 7.85-7.93 (m, 3H), 7.28 (dd, *J*=9.0, 2.5 Hz, 1H), 7.20 (d, *J*=2.5 Hz, 1H), 3.97 (s, 3H), 3.73 (s, 3H), 3.41 (dd, *J*=7.1, 4.1 Hz, 1H), 2.82 (d, J=7.1 Hz, 1H), 2.58 (d, *J*=4.1 Hz, 1H); ¹³C-NMR spectrum (100 MHz): (CDCl₃, HMDSO) δ: 167.2, 160.4, 137.4, 131.3, 131.0, 129.8, 128.1, 127.3, 123.6, 120.9, 105.9, 55.5, 52.9, 35.7, 32.1; LCMS ESI⁺ (m/z): 322.2 [M+H]⁺, LCMS purity 95.29% |
| C-3329 | Methyl 1-[(6-methyl-2-naphthyl)sulfonyl]aziridine-2-carboxylate |
| | Molecular formula: C₁₅H₁₅NO₄S; Molecular weight: 305.07; Melting point: 91-93°C; ¹H-NMR spectrum (400 MHz): (CDCl₃, HMDSO) δ: 8.63-8.66 (m, 1H), 8.21 (dd, *J*=7.4, 1.3 Hz, 1H), 8.9 (dt, *J*=8.2, 1.2 Hz, 1H), 7.84 (d, *J*=1.4 Hz, 1H), 7.48 (dd, *J*=8.2, 7.4 Hz, 1H), 7.46 (dd, *J*=8.4, 1.6 Hz, 1H), 3.71 (s, 3H), 3.50 (dd, *J*=7.1, 4.1 Hz, 1H), 2.88 (d, *J*=7.1 Hz, 1H), 2.61 (s, 3H), 2.59 (d, *J*=4.1 Hz, 1H); ¹³C-NMR spectrum (100 MHz): (CDCl₃, HMDSO) δ: 167.2, 138.9, 135.4, 132.5, 131.7, 130.0, 129.4, 129.2, 128.5, 124.4, 123.0, 52.8, 35.9, 32.5, 22.4; LCMS ESI⁺ (m/z): 306.3 [M+H]⁺, LCMS purity 98.42% |
| C-3332 | Methyl 1-(4-ethylphenyl)sulfonylaziridine-2-carboxylate |
| | Molecular formula: C₁₂H₁₃NO₄S; Molecular weight: 269.07; Melting point: oil; ¹H-NMR spectrum (400 MHz): (CDCl₃, HMDSO) δ: 7.87 (d, *J*=8.4 Hz, 2H), 7.38 (d, *J*=8.4 Hz, 2H), 3.74 (s, 3H), 3.35 (dd, *J*=7.1, 4.1 Hz, 1H), 2.71-2.78 (m, 3H), 2.56 (d, *J*=4.1 Hz, 1H), 1.27 (t,*J*=7.6 Hz, 3H); ¹³C-NMR spectrum (100 MHz): (CDCl₃, HMDSO) δ: 167.2, 151.3, 134.1, 128.7, 128.3, 52.9, 35.6, 32.0, 28.9, 15.0; LCMS ESI⁺ (m/z): 270.3 [M+H]⁺, LCMS purity 97.57% |
| C-3336 | Methyl 1-[(4-methyl-1-naphthyl)sulfonyl]aziridine-2-carboxylate |
| | Molecular formula: C₁₅H₁₅NO₄S; Molecular weight: 305.07; Melting point: 96-98°C; ¹H-NMR spectrum (400 MHz): (CDCl₃, HMDSO) δ: 8.93 (d, *J*=8.8 Hz, 1H), 8.13 (d, *J*=7.6 Hz, 1H), 8.11 (dd, *J*=8.0, 1.1 Hz, 1H), 7.72 (ddd, *J*=8.3, 6.9 Hz, 1.4 Hz, 1H), 7.66 (ddd, *J*=8.3, 6.9 Hz, 1.4 Hz, 1H), 7.41 (dd, *J*=7.6, 0.9 Hz, 1H), 3.70 (s, 3H), 3.46 (dd, *J*=7.1, 4.1 Hz, 1H), 2.86 (d, *J*=7.1 Hz, 1H), 2.79 (s, 3H), 2.57 (d, *J*=4.1 Hz, 1H); ¹³C-NMR spectrum (100 MHz): (CDCl₃, HMDSO) δ: 167.2, 143.2, 133.3, 130.7, 129.8, 129.0, 128.2, 127.0, 126.1, 124.8, 124.7, 52.8, 35.8, 32.4, 20.2; LCMS ESI⁺ (m/z): 306.3 [M+H]⁺, LCMS purity 100.00% |
| C-3342 | N,N-Dimethyl-1-(p-tolylsulfonyl)aziridine-2-carboxamide |
| | Molecular formula: C₁₂H₁₆N₂O₃S; Molecular weight: 268.33; Melting point: oil; ¹H-NMR spectrum (400 MHz): (CDCl₃, HMDSO) δ: 7.85 (d, *J*=8.4 Hz, 2H), 7.35 (d, *J*=8.4 Hz, 2H), 3.59 (dd, *J*=6.9, 4.2 Hz, 1H), 3.22 (s, 3H), 2.97 (s, 3H), 2.68 (d, *J*=4.2 Hz, 1H), 2.64 (d, *J*=6.9 Hz, 1H), 2.45 (s, 3H); ¹³C-NMR spectrum (100 MHz): (CDCl₃, HMDSO) δ: 164.9, 145.1, 134.2, 129.8, 128.1, 37.2, 36.0, 35.2, 32.4, 21.6; LCMS ESI⁺ (m/z): 269.2 [M+H]⁺, LCMS purity 100.00% |
| C-3343 | Methyl 1-[4-(dimethylamino)phenyl]sulfonylaziridine-2-carboxylate |
| | Molecular formula: C₁₂H₁₆N₂O₄S; Molecular weight: 284.33; Melting point: 84-86°C; ¹H-NMR spectrum (400 MHz): (CDCl₃, HMDSO) δ: 7.74 (d, *J*=9.1 Hz, 2H), 6.68 (d, *J*=9.1 Hz, 2H), 3.73 (s, 3H), 3.25 (dd, *J*=7.1, 4.1 Hz, 1H), 3.07 (s, 6H), 2.67 (d, *J*=7.1 Hz, 1H), 2.50 (d, *J*=4.1 Hz, 1H); ¹³C-NMR spectrum (100 MHz): (CDCl₃, HMDSO) δ: 167.6, 153.7, 130.1, 121.1, 110.9, 52.7, 40.0, 35.5, 31.8; LCMS ESI⁺ (m/z): 285.2 [M+H]⁺, LCMS purity 100.00% |
| C-3346 | Methyl 1-(2-furylsulfonyl)aziridine-2-carboxylate |
| | Molecular formula: C₈H₉NO₅S; Molecular weight: 231.02; Melting point: oil; ¹H-NMR spectrum (400 MHz): (CDCl₃, HMDSO) δ: 7.67 (dd, *J*=1.8, 1.0 Hz, 1H), 7.23 (dd, *J*=3.5, 1.0 Hz, 1H), 6.58 (dd, *J*=3.5, 1.8 Hz, 1H), 3.78 (s, 3H), 3.42 (dd, *J*=7.1, 4.2 Hz, 1H), 2.85 (d, *J*=7.1 Hz, 1H), 2.68 (d, *J*=4.2 Hz, 1H); ¹³C-NMR spectrum (100 MHz): (CDCl₃, HMDSO) δ: 166.8, 147.9, 145.5, 111.6, 53.0, 35.9, 32.3; LCMS ESI⁺ (m/z): 232.1 [M+H]⁺, LCMS purity 99.01% |
| C-3350 | Methyl 1-[(2-oxo-1H-quinolin-6-yl)sulfonyl]aziridine-2-carboxylate |
| | Molecular formula: C₁₃H₁₂N₃O₃S; Molecular weight: 308.31; Melting point: 139-141°C; ¹H-NMR spectrum (400 MHz): (CDCl₃, HMDSO) δ: 12.78 (br s, 1H), 8.24 (d, *J*=2.0 Hz, 1H), 8.05 (dd, *J*=8.7, 2.0 Hz, 1H), 7.90 (d, *J*=9.6 Hz, 1H), 7.67 (d, *J*=8.7 Hz, 1H), 6.84 (d, *J*=9.6 Hz, 1H), 3.74 (s, 3H), 3.41 (dd, *J*=7.1, 4.1 Hz, 1H), 2.84 (d, *J*=7.1 Hz, 1H), 2.61 (d, *J*=4.1 Hz, 1H); ¹³C-NMR spectrum (100 MHz): (CDCl₃, HMDSO) δ: 167.0, 164.6, 142.0, 140.7, 131.1, 129.4, 129.1, 123.4, 119.4, 117.1, 53.0, 35.9, 32.2; LCMS ESI⁺ (m/z): 309.1 [M+H]⁺, LCMS purity 96.88% |
| C-3353 | Methyl 1-(2,4-difluorophenyl)sulfonylaziridine-2-carboxylate |
| | Molecular formula: C₁₀H₉F₂NO₄S; Molecular weight: 277.24; Melting point: 40-42°C; ¹H-NMR spectrum (400 MHz): (CDCl₃, HMDSO) δ: 7.99 (ddd, *J*=7.9, 7.9, 7.9 Hz, 1H), 7.03-7.08 (m, 1H), 6.97-7.03 (m, 1H), 3.77 (s, 3H), 3.52 (ddd, *J*=7.2, 4.3, 0.9 Hz, 1H), 2.97 (dd, *J*=7.2, 1.2 Hz, 1H), 2.69 (d, *J*=4.3 Hz, 1H); ¹³C-NMR spectrum (100 MHz): (CDCl₃, HMDSO) δ: 166.9, 166.7 (dd, J=260.0, 11.6 Hz,), 160.5 (dd, *J*=261.0, 12.9 Hz,), 132.4 (d, J=10.8 Hz,), 122.1 (dd, J=14.5, 3.4 Hz,), 112.2 (dd, J=22.3, 3.5 Hz,), 106.0 (t, *J*=25.5 Hz,), 53.0, 36.1, 32.6; LCMS ESI⁺ (m/z): 276.3 [M+H]⁺, LCMS purity 98.27% |
| C-3355 | Methyl 1-(2-oxochromen-6-yl)sulfonylaziridine-2-carboxylate |
| | Molecular formula: C₁₃H₁₁NO₆S; Molecular weight: 309.29; Melting point: 42-44°C; ¹H-NMR spectrum (400 MHz): (CDCl₃, HMDSO) δ: 12.78 (br s, 1H), 8.24 (d, *J*=2.0 Hz, 1H), 8.05 (dd, *J*=8.7, 2.0 Hz, 1H), 7.90 (d, *J*=9.6 Hz, 1H), 7.67 (d, *J*=8.7 Hz, 1H), 6.84 (d, *J*=9.6 Hz, 1H), 3.74 (s, 3H), 3.41 (dd, *J*=7.1, 4.1 Hz, 1H), 2.84 (d, *J*=7.1 Hz, 1H), 2.61 (d, *J*=4.1 Hz, 1H); ¹³C-NMR spectrum (100 MHz): (CDCl₃, HMDSO) δ: 166.8, 159.0, 157.3, 142.1, 133.4, 131.0, 128.7, 119.1, 118.7, 118.2, 53.0, 36.0, 32.3; LCMS ESI⁺ (m/z): 310.1 [M+H]⁺, LCMS purity 100.00% |
| C-3357 | 1-[4-(Dimethylamino)phenyl]sulfonyl-N,N-dimethyl-aziridine-2-carboxamide |
| | Molecular formula: C₁₃H₁₉N₃O₃S; Molecular weight: 297.37; Melting point: 144-146°C; ¹H-NMR spectrum (400 MHz): (CDCl₃, HMDSO) δ: 7.74 (d, *J*=9.2 Hz, 2H), 6.68 (d, *J*=9.2 Hz, 2H), 3.49 (dd, *J*=6.9, 4.2 Hz, 1H), 3.23 (s, 3H), 3.06 (s, 6H), 2.97 (s, 3H), 2.61 (d, *J*=4.2 Hz, 1H), 2.55 (d, *J*=6.9 Hz, 1H); ¹³C-NMR spectrum (100 MHz): (CDCl₃, HMDSO) δ: 165.3, 153.6, 130.0, 121.4, 110.8, 40.0, 37.3, 36.0, 35.4, 31.9; LCMS ESI⁺ (m/z): 298.2 [M+H]⁺, LCMS purity 100.00% |
| C-3362 | 1-(4-Butylphenyl)sulfonyl-N,N-dimethyl-aziridine-2-carboxamide |
| | Molecular formula: C₁₅H₂₂N₂O₃S; Molecular weight: 310.41; Melting point: oil; ¹H-NMR spectrum (400 MHz): (CDCl₃, HMDSO) δ: 7.86 (d, *J*=8.4 Hz, 2H), 7.35 (d, *J*=8.4 Hz, 2H), 3.59 (dd, *J*=6.9, 4.2 Hz, 1H), 3.22 (s, 3H), 2.98 (s, 3H), 2.69 (t, *J*=7.6 Hz, 2H), 2.68 (d, *J*=4.2 Hz, 1H), 2.65 (d, *J*=6.9 Hz, 1H), 1.57-1.66 (m, 2H), 1.35 (sextet, *J*=7.5 Hz, 2H), 0.93 (d, *J*=7.4 Hz, 3H); ¹³C-NMR spectrum (100 MHz): (CDCl₃, HMDSO) δ: 164.9, 150.0, 134.3, 129.2, 128.2, 37.2, 36.0, 35.6, 35.2, 33.1, 32.5, 22.2, 13.8; LCMS ESI⁺ (m/z): 311.2 [M+H]⁺, LCMS purity 100.00% |
| C-3364 | 1-(4-Isopropylphenyl)sulfonyl-N,N-dimethyl-aziridine-2-carboxamide |
| | Molecular formula: C₁₄H₂₀N₂O₃S; Molecular weight: 296.38; Melting point: oil; ¹H-NMR spectrum (400 MHz): (CDCl₃, HMDSO) δ: 7.88 (d, *J*=8.5 Hz, 2H), 7.40 (d, *J*=8.5 Hz, 2H), 3.55 (dd, *J*=6.9, 4.2 Hz, 1H), 3.23 (s, 3H), 3.00 (septet, *J*=6.9 Hz, 1H), 2.98 (s, 3H), 2.69 (d, *J*=4.2 Hz, 1H), 2.65 (d, *J*=6.9 Hz, 1H), 1.28 (d, *J*=6.9 Hz, 6H); ¹³C-NMR spectrum (100 MHz): (CDCl₃, HMDSO) δ: 164.9, 155.8, 134.5, 128.3, 127.3, 37.2, 36.0, 35.2, 34.3, 32.6, 23.6; LCMS ESI⁺ (m/z): 297.2 [M+H]⁺, LCMS purity 100.00% |
| C-3365 | N,N-Dimethyl-1-(1-naphthylsulfonyl)aziridine-2-carboxamide |
| | Molecular formula: C₁₅H₁₆N₂O₃S; Molecular weight: 304.36; Melting point: 110-112°C; ¹H-NMR spectrum (400 MHz): (CDCl₃, HMDSO) δ: 8.87 (ddd, *J*=8.5, 1.8, 0.9 Hz, 1H), 8.25 (dd, *J*=7.4, 1.3 Hz, 1H), 8.15 (dt, *J*=8.2, 1.2 Hz, 1H), 7.95 (ddd, *J*=8.2, 1.3, 0.6 Hz, 1H), 7.72 (ddd, *J*=8.5, 6.9, 1.4 Hz, 1H), 7.63 (ddd, *J*=8.2, 6.9, 1.2 Hz, 1H), 7.56 (dd, *J*=8.2, 7.4 Hz, 1H), 3.75 (dd, *J*=6.9, 4.3 Hz, 1H), 3.20 (s, 3H), 2.97 (s, 3H), 2.74 (d, *J*=6.9 Hz, 1H), 2.69 (d, *J*=4.3 Hz, 1H); ¹³C-NMR spectrum (100 MHz): (CDCl₃, HMDSO) δ: 164.9, 135.6, 134.2, 132.8, 129.7, 129.0, 128.7, 128.7, 127.2, 125.7, 123.9, 37.2, 36.0, 35.8, 32.7; LCMS ESI⁺ (m/z): 305.2 [M+H]⁺, LCMS purity 96.57% |
| C-3366 | N,N-Dimethyl-1-[(4-methyl-1-naphthyl)sulfonyl]aziridine-2-carboxamide |
| | Molecular formula: C₁₆H₁₈N₂O₃S; Molecular weight: 318.39; Melting point: 120-122°C; ¹H-NMR spectrum (400 MHz): (CDCl₃, HMDSO) δ: 8.92 (ddd, *J*=8.3, 1.4, 0.7 Hz, 1H), 8.14 (d, *J*=7.6 Hz, 1H), 8.10 (ddd, *J*=8.3, 1.3, 0.7 Hz, 1H), 7.71 (ddd, *J*=8.3, 6.8, 1.5 Hz, 1H), 7.66 (ddd, *J*=8.3, 6.9, 1.5 Hz, 1H), 7.41 (ddd, *J*=7.6, 1.9, 0.9 Hz, 1H), 3.73 (dd, *J*=6.9, 4.3 Hz, 1H), 3.20 (s, 3H), 2.97 (s, 3H), 2.79 (s, 3H), 2.70 (d, *J*=6.9 Hz, 1H), 2.66 (d, *J*=4.3 Hz, 1H); ¹³C-NMR spectrum (100 MHz): (CDCl₃, HMDSO) δ: 164.9, 143.1, 133.3, 130.9, 129.7, 129.0, 128.2, 127.0, 126.2, 124.8, 124.7, 37.3, 35.9, 35.9, 32.6, 20.2; LCMS ESI⁺ (m/z): 319.2 [M+H]⁺, LCMS purity 98.41% |
| C-3368 | N,N-Dimethyl-1-(4-pentylphenyl)sulfonyl-aziridine-2-carboxamide |
| | Molecular formula: C₁₆H₂₄N₂O₃S; Molecular weight: 324.44; Melting point: oil; ¹H-NMR spectrum (400 MHz): (CDCl₃, HMDSO) δ: 7.85 (d, *J*=8.5 Hz, 2H), 7.30 (d, *J*=8.5 Hz, 2H), 3.59 (dd, *J*=6.9, 4.3 Hz, 1H), 3.21 (s, 3H), 2.97 (s, 3H), 2.68 (t, *J*=7.8 Hz, 2H), 2.68 (d, *J*=4.3 Hz, 1H), 2.65 (d, *J*=6.9 Hz, 1H), 1.63 (quintet, *J*=7.5 Hz, 2H), 1.23-1.39 (m, 4H), 0.89 (t, *J*=7.0 Hz, 3H); ¹³C-NMR spectrum (100 MHz): (CDCl₃, HMDSO) δ: 164.9, 150.0, 134.4, 129.2, 128.2, 37.2, 36.0, 35.9, 35.2, 32.5, 31.3, 30.7, 22.4, 13.9; LCMS ESI⁺ (m/z): 325.2 [M+H]⁺, LCMS purity 100.00% |
| C-3369 | 1-(4-Hexylphenyl)sulfonyl-N,N-dimethyl-aziridine-2-carboxamide |
| | Molecular formula: C₁₇H₂₆N₂O₃S; Molecular weight: 338.46; Melting point: oil; ¹H-NMR spectrum (400 MHz): (CDCl₃, HMDSO) δ: 7.86 (d, *J*=8.5 Hz, 2H), 7.34 (d, *J*=8.5 Hz, 2H), 3.59 (dd, *J*=6.9, 4.2 Hz, 1H), 3.21 (s, 3H), 2.97 (s, 3H), 2.68 (t, *J*=7.8 Hz, 2H), 2.68 (d, *J*=4.2 Hz, 1H), 2.65 (d, *J*=6.9 Hz, 1H), 1.62 (quintet, *J*=7.5 Hz, 2H), 1.23-1.37 (m, 6H), 0.88 (t, *J*=7.0 Hz, 3H); ¹³C-NMR spectrum (100 MHz): (CDCl₃, HMDSO) δ: 165.0, 150.0, 134.3, 129.2, 128.2, 37.2, 36.0, 35.9, 35.2, 32.5, 31.6, 30.9, 28.8, 22.5, 14.0; LCMS ESI⁺ (m/z): 339.2 [M+H]⁺, LCMS purity 95.86% |
| C-3371 | 1-[[5-(Dimethylamino)-1-naphthyl]sulfonyl]-N,N-dimethylaziridine-2-carboxamide |
| | Molecular formula: C₁₇H₂₁N₃O₃S; Molecular weight: 347.43; Melting point: oil; ¹H-NMR spectrum (400 MHz): (CDCl₃, HMDSO) δ: 8.62 (dt, *J*=8.5, 1.1 Hz, 1H), 8.55 (dt, *J*=8.7, 0.9 Hz, 1H), 8.23 (dd, *J*=7.4, 1.3 Hz, 1H), 7.60 (dd, *J*=8.7, 7.6 Hz, 1H), 7.53 (dd, *J*=8.5, 7.4 Hz, 1H), 7.21 (dd, *J*=7.6, 0.9 Hz, 1H), 3.74 (dd, *J*=6.9, 4.3 Hz, 1H), 3.20 (s, 3H), 2.96 (s, 3H), 2.88 (s, 6H), 2.73 (d, *J*=6.9 Hz, 1H), 2.68 (d, *J*=4.3 Hz, 1H); ¹³C-NMR spectrum (100 MHz): (CDCl₃, HMDSO) δ: 164.9, 151.6, 132.9, 131.8, 130.4, 129.9, 129.7, 128.7, 122.9, 120.1, 115.5, 45.4, 37.3, 35.9, 35.8, 32.7; LCMS ESI⁺ (m/z): 348.2 [M+H]⁺, LCMS purity 95.88 % |
| C-3373 | N,N-Dimethyl-1-(2-naphthylsulfonyl)aziridine-2-carboxamide |
| | Molecular formula: C₁₅H₁₆N₂O₃S; Molecular weight: 304.36; Melting point: oil ¹H-NMR spectrum (400 MHz): (CDCl₃) δ: 8.54-8.52 (m, 1H), 8.04-7.90 (m, 4H), 7.73-7.59 (m, 2H), 3.67 (dd, J=6.9, 4.3 Hz, 1H), 3.24 (s, 3H), 2.98 (s, 3H), 2.74 (d, *J*=6.9 Hz, 1H), 2.72 (d, J=4.3 Hz, 1H); ¹³C-NMR spectrum (100 MHz): (CDCl₃, HMDSO) δ: 165.0, 135.6, 134.3, 132.1, 129.9, 129.7, 129.6, 128.2, 127.9, 123.1, 37.4, 36.2, 35.5, 32.8 |
| | LCMS ESI+ (m/z): 305.2 [M+H]+ , LCMS purity 97.08% |
| C-3374 | N,N-Dimethyl-1-[4-(trifluoromethyl)phenyl]sulfonyl-aziridine-2-carboxamide |
| | Molecular formula: C₁₂H₁₃N₂O₃S; Molecular weight: 322.30; Melting point: oil; ¹H-NMR spectrum (400 MHz): 8.12 (d, *J*=8.4 Hz, 2H), 7.83 (d, *J*=8.4 Hz, 2H), 3.68 (dd, *J*=6.9, 4.3 Hz, 1H), 3.25 (s, 3H), 3.00 (s, 3H), 2.75 (d, *J*=4.3 Hz, 1H), 2.73 (d, *J*=6.9 Hz, 1H); ¹³C-NMR spectrum (100 MHz): (CDCl₃, HMDSO) δ: 164.4, 141.1, 135.6 (q, *J*=33.3 Hz), 128.6, 126.3 (q, *J*=3.7 Hz), 123.0 (q, *J*=273.2 Hz), 37.2, 36.0, 35.3, 33.0; LCMS ESI⁺ (m/z): 323.1 [M+H]⁺, LCMS purity 95.04.00% |
| C-3375 | Methyl 1-[[6-(dimethylamino)-1-naphthyl]sulfonyl]aziridine-2-carboxylate |
| | Molecular formula: C₁₆H₁₈N₂O₄S; Molecular weight: 334.39; Melting point: 106-108°C ¹H-NMR spectrum (400 MHz): (CDCl₃, HMDSO) δ: 8.73 (dt, J=9.5, 0.6 Hz, 1H), 7.93 (d, J=8.3 Hz, 1H), 7.89 (dd, J=7.3, 1.2 Hz, 1H), 7.40 (dd, J=8.3, 7.3 Hz, 1H), 7.34 (dd, J=9.5, 2.6 Hz, 1H), 6.94 (d, J=2.6 Hz, 1H), 3.70 (s, 3H), 3.44 (dd, J=7.1, 4.1 Hz, 1H), ), 3.09 (s, 6H), 2.83 (d, J=7.1 Hz, 1H), 2.55 (d, J=4.2 Hz, 1H); ¹³C-NMR spectrum (100 MHz): (CDCl₃, HMDSO) δ: 167.3, 148.9, 136.2, 133.8, 132.1, 126.2, 125.6, 124.1, 121.6, 118.2, 106.2, 52.7, 40.4, 35.7, 32.3 |
| | LCMS ESI⁺ (m/z): 335.1 [M+H]⁺, LCMS purity 100% |
| C-3376 | Methyl 1-[[6-(dimethylamino)-5-formyl-1-naphthyl]sulfonyl]aziridine-2-carboxylate |
| | Molecular formula: C₁₇H₁₈N₂O₅S; Molecular weight: 362.40; Melting point: 116-117°C; ¹H-NMR spectrum (400 MHz): (CDCl₃, HMDSO) δ: 10.30 (s, 1H), 9.49 (dt, *J*=8.7, 1.1 Hz, 1H), 9.02 (dd, *J*=9.7, 0.9 Hz, 1H), 8.05 (dd, *J*=7.4, 1.1 Hz, 1H), 7.62 (dd, *J*=8.7, 7.4 Hz, 1H), 7.53 (d, *J*=9.7 Hz, 1H), 3.72 (s, 3H), 3.47 (dd, *J*=7.1, 4.1 Hz, 1H), 3.19 (s, 6H), 2.85 (d, *J*=7.1 Hz, 1H), 2.55 (d, *J*=4.2 Hz, 1H); ¹³C-NMR spectrum (100 MHz): (CDCl₃, HMDSO) δ: 190.8, 167.1, 158.1, 133.9, 132.6, 132.0, 131.3, 127.2, 127.0, 123.7, 120.4, 116.4, 52.8, 45.9, 35.8, 32.4; LCMS ESI⁺ (m/z): 363.1 [M+H]⁺, LCMS purity 100.00% |
| C-3377 | 1-[[6-(Dimethylamino)-1-naphthyl]sulfonyl]-N,N-dimethylaziridine-2-carboxamide |
| | Molecular formula: C₁₇H₂₁N₃O₃S; Molecular weight: 347.43; Melting point: 147-149°C ¹H-NMR spectrum (400 MHz): (CDCl₃, HMDSO) δ: 8.72 (d, J=9.5 Hz, 1H), 7.90 (d, J=8.4 Hz, 1H), 7.89 (dd, J=7.3, 1.2 Hz, 1H), 7.49 (dd, J=8.2, 7.5 Hz, 1H), 7.32 (dd, J=9.5, 2.7 Hz, 1H), 6.93 (d, J=2.7 Hz, 1H), 3.67 (dd, J=6.9, 4.3 Hz, 1H), 3.14 (s, 3H), 3.08 (s, 6H), 2.94 (s, 3H), 2.71 (d, J=6.9 Hz, 1H), 2.67 (d, J=4.3 Hz, 1H); ¹³C-NMR spectrum (100 MHz): (CDCl₃, HMDSO) δ: 165.0, 148.9, 136.2, 133.7, 132.3, 126.3, 125.3, 124.1, 121.5, 118.2, 106.1, 40.3, 37.1, 35.9, 35.9, 32.3 |
| | LCMS ESI+ (m/z): 348.1 [M+H]+ , LCMS purity 100% |
| C-3380 | 1-(4-Hexylphenyl)sulfonyl-N-methyl-aziridine-2-carboxamide |
| | Molecular formula: C₁₆H₂₄N₂O₃S; Molecular weight: 324.44; Melting point: 84-86°C; ¹H-NMR spectrum (400 MHz): (CDCl₃, HMDSO) δ: 7.83 (d, *J*=8.5 Hz, 2H), 7.38 (d, *J*=8.5 Hz, 2H), 6.15 (br s, 1H), 3.28 (dd, *J*=7.7, 4.2 Hz, 1H), 2.75 (d, *J*=4.9 Hz, 3H), 2.74 (d, *J*=7.7 Hz, 1H), 2.71 (t, *J*=7.8 Hz, 2H), 2.36 (d, *J*=4.2 Hz, 1H), 1.64 (quintet, *J*=7.5 Hz, 2H), 1.25-1.39 (m, 6H), 0.89 (t, *J*=7.0 Hz, 3H); ¹³C-NMR spectrum (100 MHz): (CDCl₃, HMDSO) δ: 166.2, 150.4, 133.4, 129.4, 128.2, 37.9, 36.0, 33.4, 31.5, 30.8, 28.8, 25.9, 22.5, 14.0; LCMS ESI⁺ (m/z): 325.1 [M+H]⁺, LCMS purity 97.38% |
| C-3383 | Methyl 1-[[4-(dimethylamino)-1-naphthyl]sulfonyl]aziridine-2-carboxylate |
| | Molecular formula: C₁₆H₁₈N₂O₄S; Molecular weight: 334.39; Melting point: oil; ¹H-NMR spectrum (400 MHz): (CDCl₃, HMDSO) δ: 8.86 (ddd, *J*=8.6, 1.3, 0.7 Hz, 1H), 8.23 (ddd, *J*=8.6, 1.4, 0.7 Hz, 1H), 8.11 (d, *J*=8.3 Hz, 1H), 7.67 (ddd, *J*=8.6, 6.9, 1.4 Hz, 1H), 7.56 (ddd, *J*=8.6, 6.9, 1.3 Hz, 1H), 6.97 (d, *J*=8.3 Hz, 1H), 3.70 (s, 3H), 3.44 (dd, *J*=7.1, 4.1 Hz, 1H), 3.01 (s, 6H), 2.83 (d, *J*=7.1 Hz, 1H), 2.54 (d, *J*=4.1 Hz, 1H); ¹³C-NMR spectrum (100 MHz): (CDCl₃, HMDSO) δ: 167.4, 157.5, 131.3, 130.8, 128.3, 128.2, 125.9, 125.7, 125.3, 124.3, 110.7, 52.7, 44.6, 35.7, 32.4; LCMS ESI⁺ (m/z): 335.1 [M+H]⁺, LCMS purity 100.00% |
| C-3384 | 1-(4-Cyclohexylphenyl)sulfonyl-N,N-dimethyl-aziridine-2-carboxamide |
| | Molecular formula: C₁₇H₂₄N₂O₃S; Molecular weight: 336.45; Melting point: oil; ¹H-NMR spectrum (400 MHz): (CDCl₃, HMDSO) δ: 7.86 (d, *J*=8.5 Hz, 2H), 7.37 (d, *J*=8.5 Hz, 2H), 3.58 (dd, *J*=6.9, 4.3 Hz, 1H), 3.21 (s, 3H), 2.97 (s, 3H), 2.68 (d, *J*=4.3 Hz, 1H), 2.65 (d, *J*=6.9 Hz, 1H), 2.54-2.63 (m, 1H), 1.80-1.92 (m, 4H), 1.72-1.80 (m, 1H), 1.32-1.48 (m, 4H), 1.19-1.32 (m, 1H); ¹³C-NMR spectrum (100 MHz): (CDCl₃, HMDSO) δ: 164.9, 154.9, 134.4, 128.2, 127.7, 44.6, 37.3, 36.0, 35.1, 34.0, 32.5, 26.6, 25.9; LCMS ESI⁺ (m/z): 337.2 [M+H]⁺, LCMS purity 95.84% |
| C-3385 | 1-[[4-(Dimethylamino)-1-naphthyl]sulfonyl]-N,N-dimethylaziridine-2-carboxamide |
| | Molecular formula: C₁₇H₂₁N₃O₃S; Molecular weight: 347.43; Melting point: 120-122°C ¹H-NMR spectrum (400 MHz): (CDCl₃, HMDSO) δ: 8.85 (ddd, *J*=8.6, 1.3, 0.6 Hz, 1H), 8.22 (ddd, *J*=8.6, 1.3, 0.6 Hz, 1H), 8.12 (d, *J*=8.2 Hz, 1H), 7.65 (ddd, *J*=8.6, 6.9, 1.4 Hz, 1H), 7.56 (ddd, *J=*8.6, 6.9, 1.4 Hz, 1H), 6.97 (d, *J*=8.2 Hz, 1H), 3.69 (dd, *J*=6.9, 4.3 Hz, 1H), 3.20 (s, 3H), 3.01 (s, 6H), 2.96 (s, 3H), 2.68 (d, *J*=6.9 Hz, 1H), 2.64 (d, *J*=4.3 Hz, 1H); ¹³C-NMR spectrum (100 MHz): (CDCl₃, HMDSO) δ: 165.2, 157.4, 131.0, 130.8, 128.3, 128.2, 126.0, 125.7, 125.3, 124.6, 110.6, 44.6, 37.3, 35.9, 35.8, 32.5 |
| | LCMS ESI⁺ (m/z): 348.1 [M+H]⁺, LCMS purity 100% |
| C-3389 | 1-(4-Hexylphenyl)sulfonylaziridine-2-carboxamide |
| | Molecular formula: C₁₅H₂₂N₂O₃S; Molecular weight: 310.41; Melting point: 103-105°C; ¹H-NMR spectrum (400 MHz): (DMSO-d₆, HMDSO) δ: 7.84 (d, *J*=8.4 Hz, 2H), 7.81 (br s, 1H), 7.50 (d, *J*=8.4 Hz, 2H), 7.41 (br s, 1H), 3.23 (dd, *J*=7.2, 4.3 Hz, 1H), 2.69 (t, *J*=7.7 Hz, 2H), 2.62 (d, *J*=7.2 Hz, 1H), 2.46 (d, *J*=4.3 Hz, 1H), 1.60 (quintet, *J*=7.4 Hz, 2H), 1.22-1.33 (m, 6H), 0.85 (t, *J*=7.0 Hz, 3H); ¹³C-NMR spectrum (100 MHz): (DMSO-d₆, HMDSO) δ: 167.0, 150.0, 134.5, 129.8, 128.2, 37.1, 35.4, 31.6, 31.4, 30.9, 28.7, 22.4, 14.3; LCMS ESI⁺ (m/z): 311.1 [M+H]⁺, LCMS purity 100.00% |
| C-3390 | Methyl 1-[[6-(dimethylamino)-5-formyl-2-naphthyl]sulfonyl]aziridine-2-carboxylate |
| | Molecular formula: C₁₇H₁₈N₂O₅S; Molecular weight: 362.40; Melting point: 147-149°C ¹H-NMR spectrum (400 MHz): (CDCl3, HMDSO) δ: 10.27 (s, 1H), 9.22 (d, J=9.1 Hz, 1H), 8.35 (d, J=2.0 Hz, 1H), 7.96 (dd, J=9.1, 2.0 Hz, 1H), 7.94 (d, J=9.1 Hz, 1H), 7.41 (d, J=9.1 Hz, 1H), 3.72 (s, 3H), 3.39 (dd, J=7.1, 4.1 Hz, 1H), 3.24 (s, 6H), 2.81 (d, J=7.1 Hz, 1H), 2.58 (d, J=4.1 Hz, 1H); ¹³C-NMR spectrum (100 MHz): (CDC13, HMDSO) δ: 190.1, 167.2, 159.5, 135.9, 135.7, 131.1, 129.7, 126.5, 126.1, 125.1, 119.6, 115.2, 52.9, 45.7, 35.8, 32.0 LCMS ESI⁺ (m/z): 363.1 [M+H]⁺, LCMS purity 96.10% |
| C-3391 | 1-[[6-(Dimethylamino)-5-formyl-2-naphthyl]sulfonyl]-N,N-dimethylaziridine-2-carboxamide |
| | Molecular formula: C₁₈H₂₁N₃O₄S; Molecular weight: 375.44; Melting point: 58-60°C; ¹H-NMR spectrum (400 MHz): (CDCl₃, HMDSO) δ: 10.27 (s, 1H), 9.21 (dt, *J*=9.2, 0.6 Hz, 1H), 8.35 (d, *J*=2.1 Hz, 1H), 7.97 (dd, *J*=9.2, 2.1 Hz, 1H), 7.94 (d, *J*=9.2 Hz, 1H), 7.41 (d, *J*=9.2 Hz, 1H), 3.64 (dd, *J*=6.9, 4.3 Hz, 1H), 3.24 (s, 3H), 3.23 (s, 6H), 2.97 (s, 3H), 2.70 (d, *J*=6.9 Hz, 1H), 2.69 (d, *J*=4.3 Hz, 1H); ¹³C-NMR spectrum (100 MHz): (CDCl₃, HMDSO) δ: 190.1, 164.9, 159.5, 135.8, 135.6, 131.5, 129.5, 126.5, 126.1, 125.0, 119.6, 115,2, 45.7, 37.2, 36.0, 35.3, 32.5; LCMS ESI⁺ (m/z): 376.1 [M+H]⁺, LCMS purity 100.00% |
| C-3393 | Methyl 1-[[5-chloro-6-(methylamino)-2-naphthyl]sulfonyl]aziridine-2-carboxylate |
| | Molecular formula: C₁₅H₁₅ClN₂O₄S; Molecular weight: 354.81; Melting point: 170-172°C; ¹H-NMR spectrum (400 MHz): (CDCl₃, HMDSO) δ: 8.36 (d, *J*=1.9 Hz, 1H), 8.12 (dt, *J*=9.1, 0.6 Hz, 1H), 7.90 (dd, *J*=9.1, 2.0 Hz, 1H), 7.83 (d, *J*=9.0 Hz, 1H), 7.19 (d, *J*=9.0 Hz, 1H), 5.01 (q, *J*=5.1 Hz, 1H), 3.72 (s, 3H), 3.39 (dd, *J*=7.1, 4.1 Hz, 1H), 3.09 (d, *J*=5.1 Hz, 3H), 2.81 (d, *J*=7.1 Hz, 1H), 2.56 (d, *J*=4.1 Hz, 1H); ¹³C-NMR spectrum (100 MHz): (CDCl₃, HMDSO) δ: 167.3, 145.4, 133.9, 130.5, 129.7, 129.1, 125.3, 124.4, 123.3, 114.3, 110.5, 52.8, 35.7, 32.0, 30.3; LCMS ESI⁺ (m/z): 355.0 [M+H]⁺, LCMS purity 100.00% |
| C-3397 | Methyl 1-[[6-(dimethylamino)-2-naphthyl]sulfonyl]aziridine-2-carboxylate |
| | Molecular formula: C₁₆H₁₈N₂O₄S; Molecular weight: 334.39; Melting point: 156-158°C; ¹H-NMR spectrum (400 MHz): (CDCl₃, HMDSO) δ: 8.31 (d, *J*=1.9 Hz, 1H), 7.92 (d, *J*=9.2 Hz, 1H), 7.77 (dd, *J*=8.8, 1.9 Hz, 1H), 7.71 (d, *J*=8.8 Hz, 1H), 7.22 (dd, *J*=9.2, 2.6 Hz, 1H), 6.88 (d, *J*=2.6 Hz, 1H), 3.72 (s, 3H), 3.36 (dd, *J*=7.1, 4.1 Hz, 1H), 3.13 (s, 6H), 2.78 (d, *J*=7.1 Hz, 1H), 2.56 (d, *J*=4.1 Hz, 1H); ¹³C-NMR spectrum (100 MHz): (CDCl₃, HMDSO) δ: 167.4, 150.7, 137.7, 130.5, 130.0, 128.1, 127.0, 124.4, 123.4, 116.3, 104.9, 52.8, 40.3, 35.6, 32.0; LCMS ESI⁺ (m/z): 335.1 [M+H]⁺, LCMS purity 100.00% |
| C-3398 | 1-[[6-(Dimethylamino)-2-naphthyl]sulfonyl]-N,N-dimethylaziridine-2-carboxamide |
| | Molecular formula: C₁₇H₂₁N₃O₃S; Molecular weight: 347.43; Melting point: foam; ¹H-NMR spectrum (400 MHz): (CDCl₃, HMDSO) δ: 8.31 (d, *J*=2.0 Hz, 1H), 7.79 (d, *J*=9.2 Hz, 1H), 7.78 (dd, *J*=8.7, 2.0 Hz, 1H), 7.71 (d, *J*=8.7 Hz, 1H), 7.21 (dd, *J*=9.2, 2.5 Hz, 1H), 6.87 (d, *J*=2.5 Hz, 1H), 3.59 (dd, *J*=6.9, 4.3 Hz, 1H), 3.21 (s, 3H), 3.12 (s, 6H), 2.96 (s, 3H), 2.68 (d, *J*=4.3 Hz, 1H), 2.67 (d, *J*=6.9 Hz, 1H); ¹³C-NMR spectrum (100 MHz): (CDCl₃, HMDSO) δ: 165.1, 150.6, 137.6, 130.4, 129.8, 128.5, 127.0, 124.4, 123.4, 117.0, 105.0, 40.3, 37.3, 36.0, 35.4, 32.3; LCMS ESI⁺ (m/z): 348.1 [M+H]⁺, LCMS purity 96.37% |
| C-3399 | Methyl 1-[[5-(dimethylamino)-2-naphthyl]sulfonyl]aziridine-2-carboxylate |
| | Molecular formula: C₁₆H₁₈N₂O₄S; Molecular weight: 334.39; Melting point: 85-87°C ¹H-NMR spectrum (400 MHz): (CDCl3, HMDSO) δ: 8.47 (d, J=2.0 Hz, 1H), 8.40 (dt, J=9.0, 0.7 Hz, 1H), 7.93 (dd, J=9.0, 2.0 Hz, 1H), 7.63 (dt, J=8.2, 1.0 Hz, 1H), 7.54 (dd, J=8.2, 7.6 Hz, 1H), 7.25 (dd, J=7.6, 1.0 Hz, 1H), 3.73 (s, 3H), 3.43 (dd, J=7.1, 4.1 Hz, 1H), 2.90 (s, 6H), 2.84 (d, J=7.1 Hz, 1H), 2.60 (d, J=4.1 Hz, 1H); ¹³C-NMR spectrum (100 MHz): (CDCl3, HMDSO) δ: 167.2, 151.1, 133.6, 133.5, 131.1, 130.2, 127.8, 126.2, 123.9, 121.9, 117.4, 52.9, 45.1, 35.8, 32.1 |
| | LCMS ESI⁺ (m/z): 335.1 [M+H]⁺, LCMS purity 100% |
| C-3400 | 1-[[5-(Dimethylamino)-2-naphthyl]sulfonyl]-N,N-dimethylaziridine-2-carboxamide |
| | Molecular formula: C₁₇H₂₁N₃O₃S; Molecular weight: 347.43; Melting point: 96-98°C; ¹H-NMR spectrum (400 MHz): (CDCl₃, HMDSO) δ: 8.47 (d, *J*=1.9 Hz, 1H), 8.39 (dt, *J*=9.0, 0.7 Hz, 1H), 7.94 (dd, *J*=9.0, 1.9 Hz, 1H), 7.62 (dt, J=8.2, 1.0 Hz, 1H), 7.53 (dd, *J*=8.2, 7.6 Hz, 1H), 7.24 (dd, *J*=7.6, 1.0 Hz, 1H), 3.67 (dd, *J*=6.9, 4.3 Hz, 1H), 3.25 (s, 3H), 2.98 (s, 3H), 2.90 (s, 6H), 2.73 (d, *J*=6.9 Hz, 1H), 2.72 (d, *J*=4.3 Hz, 1H); ¹³C-NMR spectrum (100 MHz): (CDCl₃, HMDSO) δ: 167.9, 151.1, 133.9, 133.4, 131.0, 130.0, 127.8, 126.1, 123.8, 122.0, 117.3, 45.1, 37.3, 36.0, 35.3, 32.6; LCMS ESI⁺ (m/z): 348.2 [M+H]⁺, LCMS purity 100.00% |
| C-3402 | 1-(2,4-Difluorophenyl)sulfonylaziridine-2-carboxamide |
| | Molecular formula: C₉H₈F₂NO₃S; Molecular weight: 262.23; Melting point: 141-143°C; ¹H-NMR spectrum (400 MHz): (CDCl₃, HMDSO) δ: 7.99 (dt, *J*=8.5, 6.2 Hz, 1H), 7.87 (s, 1H), 7.66-7.74 (m, 1H), 7.47 (s, 1H), 7.36-7.43 (m, 1H), 3.38 (dd, *J*=7.2, 4.5 Hz, 1H), 2.77 (d, *J*=7.2 Hz, 1H), 2.59 (d, *J*=4.5 Hz, 1H); ¹³C-NMR spectrum (100 MHz): (CDCl₃, HMDSO) δ: 166.6, 166.7 (dd, *J*=256.9, 12.1 Hz,), 160.5 (dd, *J*=258.8, 13.7 Hz,), 132.8 (d, *J*=11.1 Hz,), 122.2 (dd, *J*=14.6, 3.7 Hz,), 113.3 (dd, *J*=22.4, 3.4 Hz,), 107.0 (t, *J*=26.4 Hz,), 37.3, 32.3; LCMS ESI⁺ (m/z): 263.1 [M+H]⁺, LCMS purity 100.00% |
| C-3403 | 1-(2,4-Difluorophenyl)sulfonyl-N,N-dimethyl-aziridine-2-carboxamide |
| | Molecular formula: C₁₁H₁₂F₂NO₃S; Molecular weight: 290.29; Melting point: oil; ¹H-NMR spectrum (400 MHz): (CDCl₃, HMDSO) δ: 7.99 (ddd, *J*=8.8, 7.8, 6.0 Hz, 1H), 6.96-7.06 (m, 2H), 3.26 (s, 3H), 2.99 (s, 3H), 3.71 (dd, *J*=7.0, 4.4 Hz, 1H), 2.88 (d, *J*=7.0 Hz, 1H), 2.81 (d, *J*=4.4 Hz, 1H); ¹³C-NMR spectrum (100 MHz): (CDCl₃, HMDSO) δ: 166.7 (dd, *J*=259.4, 11.6 Hz,), 164.5, 160.5 (dd, *J*=260.8, 13.1 Hz,), 132.3 (d, *J*=10.9 Hz,), 122.3 (dd, *J*=14.6, 3.8 Hz,), 112.1 (dd, *J*=22.2, 3.4 Hz,), 106.0 (t, *J*=25.6 Hz,), 37.2, 36.1, 35.1, 33.4; LCMS ESI⁺ (m/z): 291.1 [M+H]⁺, LCMS purity 94.31% |
| C-3427 | Methyl 1-[[8-(dimethylamino)-2-naphthyl]sulfonyl]aziridine-2-carboxylate |
| | Molecular formula: C₁₆H₁₈N₂O₄S; Molecular weight: 334.39; Melting point: oil; |
| | ¹H-NMR spectrum (400 MHz): (CDCl₃, HMDSO) δ: 8.88-8.89 (m, 1H), 7.96 (dd, *J*=8.8, 0.7 Hz, 1H), 7.92 (dd, *J*=8.8, 1.8 Hz, 1H), 7.58 (dd, *J*=8.2, 6.7 Hz, 1H), 7.55 (ddd, *J*=8.2, 1.8, 0.7 Hz, 1H), 7.17 (dd, *J*=6.7, 1.8 Hz, 1H), 3.72 (s, 3H), 3.42 (dd, *J*=7.1, 4.1 Hz, 1H), 2.92 (s, 6H), 2.84 (d, *J*=7.1 Hz, 1H), 2.59 (d, *J*=4.1 Hz, 1H); ¹³C-NMR spectrum (100 MHz): (CDCl₃, HMDSO) δ: 167.2, 152.6, 137.1, 132.8, 129.9, 129.7, 127.6, 126.8, 122.8, 122.2, 115.7, 52.8, 45.2, 35.8, 32.1; LCMS ESI⁺ (m/z): 335.2 [M+H]⁺, LCMS purity 100.00% |
| C-3459 | 1-[[6-(Dimethylamino)-1-naphthyl]sulfonyl]aziridine-2-carboxamide |
| | Molecular formula: C₁₅H₁₇N₃O₃S; Molecular weight: 319.38; Melting point: 197-199°C |
| | ¹H-NMR spectrum (300 MHz): (DMSO-D₆, HMDSO) δ: 8.55 (d, *J*=9.6 Hz, 1H), 8.07 (d, *J*=8.3 Hz, 1H), 7.80 (dd, *J*=7.3, 1.0 Hz, 1H), 7.76 (br s, 1H), 7.51 (dd, *J*=8.3, 7.3 Hz, 1H), 7.45 (dd, *J*=9.6, 2.7 Hz, 1H), 7.37 (br s, 1H), 7.11 (d, *J*=2.7 Hz, 1H), 3.28-3.38 (m, overlapped with water, 1H), 3.09 (s, 6H), 2.68 (d, J=7.2 Hz, 1H), 2.47 (d, J=4.4 Hz, 1H); ¹³C-NMR spectrum (100 MHz): (DMSO-D₆, HMDSO) δ: 171.8, 154.0, 141.2, 138.8, 137.3, 130.9, 130.0, 129.7, 125.7, 123.3, 111.3, 45.8, 41.2, 36.9; LCMS ESI⁺ (m/z): 320.3 [M+H]⁺, LCMS purity 100% |
| C-3511 | [1-(4-Hexylphenyl)sulfonylaziridin-2-yl]-pyrrolidin-1-yl-methanone |
| | Molecular formula: C₁₉H₂₈N₂O₃S; Molecular weight: 364.50; Melting point: oil |
| | ¹H-NMR spectrum (400 MHz): (CDCl₃, HMDSO) δ: 7.85 (d, *J*=8.3 Hz, 2H), 7.34 (d, *J*=8.3 Hz, 2H), 3.75 (dt, *J*=10.1, 6.7 Hz, 1H), 3.63 (dt, *J*=10.1, 7.0 Hz, 1H), 3.42-3.54 (m, 3H), 2.70 (d, *J*=4.2 Hz, 1H), 2.68 (t, *J*=7.9 Hz, 2H), 2.64 (d, *J*=6.9 Hz, 1H), 1.93-2.05 (m, 2H), 1.81-1.93 (m, 2H), 1.57-1.69 (m, 2H), 1.23-1.38 (m, 6H), 0.88 (t, *J*=7.0 Hz, 3H); ¹³C-NMR spectrum (100 MHz): (CDCl₃, HMDSO) δ: 163.4, 150.0, 134.4, 129.2, 128.3, 46.8, 46.5, 36.0, 35.8, 32.4, 31.6, 31.0, 28.9, 26.1, 24.2, 22.6, 14.1; LCMS ESI⁺ (m/z): 365.3 [M+H]⁺, LCMS purity 100% |
| C-3517 | [1-(4-Hexylphenyl)sulfonylaziridin-2-yl]-morpholino-methanone |
| | Molecular formula: C₁₉H₂₈N₂O₄S; Molecular weight: 380.18; Melting point: oil |
| | ¹H-NMR spectrum (400 MHz): (CDCl₃, HMDSO) δ: 7.85 (d, *J*=8.4 Hz, 2H), 7.36 (d, *J*=8.4 Hz, 2H), 3.75-3.86 (m, 2H), 3.57-3.75 (m, 5H), 3.55 (dd, *J*=6.8, 4.2 Hz, 1H), 3.46 (ddd, *J*=13.3, 7.7, 3.3 Hz, 1H), 2.71 (d, *J*=4.2 Hz, 1H), 2.69 (t, *J*=7.9 Hz, 2H), 2.67 (d, *J*=6.8 Hz, 1H), 1.57-1.68 (m, 2H), 1.23-1.39 (m, 6H), 0.88 (t, *J*=7.0 Hz, 3H); ¹³C-NMR spectrum (100 MHz): (CDCl₃, HMDSO) δ: 163.7, 150.3, 134.3, 129.3, 128.2, 66.7, 66.6, 42.8, 36.0, 35.3, 32.4, 31.6, 31.0, 28.9, 22.7, 14.1; LCMS ESI⁺ (m/z): 381.3 [M+H]⁺, LCMS purity 100% |
| C-3520 | [1-(4-hexylphenyl)sulfonylaziridin-2-yl]-(1-piperidyl)methanone |
| | Molecular formula: C₂₀H₃₀N₂O₃S; Molecular weight: 378.53; Melting point: oil |
| | ¹H-NMR spectrum (400 MHz): (CDCl₃, HMDSO) δ: 7.86 (d, *J*=8.4 Hz, 2H), 7.35 (d, *J*=8.4 Hz, 2H), 3.62-3.73 (m, 2H), 3.54 (dd, *J*=6.8, 4.2 Hz, 1H), 3.52-3.60 (m, 1H), 3.38-3.46 (m, 1H), 2.69 (t, *J*=7.3 Hz, 2H), 2.70 (d, *J*=4.2 Hz, 1H), 2.66 (d, *J*=6.8 Hz, 1H), 1.60-1.70 (m, 4H), 1.49-1.60 (m, 4H), 1.24-1.38 (m, 6H), 0.88 (t, *J*=7.0 Hz, 3H); ¹³C-NMR spectrum (100 MHz): (CDCl₃, HMDSO) δ: 163.3, 150.0, 134.6, 129.2, 128.2, 46.9, 43.6, 36.0, 35.7, 32.3, 31.6, 31.0, 28.9, 26.4, 25.4, 24.4, 22.6, 14.1; LCMS ESI⁺ (m/z): 379.3 [M+H]⁺, LCMS purity 100% |
| C-3532 | 1-(Benzenesulfonyl)aziridine-2-carboxamide |
| | Molecular formula: C₉H₁₀N₂O₃S; Molecular weight: 226.25; Melting point: 108-110 °C. ¹H-NMR spectrum (400 MHz): (CDCl₃, HMDSO) δ: 7.93-7.98 (m, 2H), 7.67-7.73 (m, 1H), 7.55-7.63 (m, 2H), 6.11 (s, 1H), 5.82 (s, 1H), 3.28 (dd, *J*=7.7, 4.2 Hz, 1H), 2.81 (d, *J*=7.7 Hz, 1H), 2.45 (d, *J*=4.2 Hz, 1H); ¹³C-NMR spectrum (100 MHz): (CDCl₃, HMDSO) δ: 168.3, 136.6, 134.4, 129.5, 128.2, 37.7, 33.2; LCMS ESI⁺ (m/z): 227.2 [M+H]⁺, LCMS purity 100.00% |
| C-3535 | Methyl (2S)-1-(p-tolylsulfonyl)aziridine-2-carboxylate |
| | Molecular formula: C₁₁H₁₃NO₄S; Molecular weight: 255.29. Melting point: oil. ¹H-NMR spectrum (400 MHz): (CDCl₃, HMDSO) δ: 7.84 (d, *J*=8.3 Hz, 2H), 7.36 (d, *J*=8.3 Hz, 2H), 3.74 (s, 3H), 3.34 (dd, *J*=7.1, 4.1 Hz, 1H), 2.76 (d, *J*=7.1 Hz, 1H), 2.56 (d, *J*=4.1 Hz, 1H), 2.45 (s, 3H). ¹³C-NMR spectrum (100 MHz (CDCl₃, HMDSO) δ: 167.2, 145.3, 134.0, 129.9, 128.2, 52.9, 35.7, 32.0, 21.7. LCMS ESI⁺ (m/z): 256.1 [M+H]⁺, LCMS purity 100% |
| C-3537 | Ethyl 1-(p-tolylsulfonyl)aziridine-2-carboxylate |
| | Molecular formula: C₁₂H₁₃NO₄S. Molecular weight: 269.32. Melting point: oil. ¹H-NMR spectrum (400 MHz): (CDCl₃, HMDSO) δ: 7.85 (d, *J*=8.3 Hz, 2H), 7.35 (d, *J*=8.3 Hz, 2H), 4.12-4.24 (m, 2H), 3.32 (dd, *J*=7.1, 4.1 Hz, 1H), 2.75 (d, *J*=7.1 Hz, 1H), 2.55 (d, *J*=4.1 Hz, 1H), 2.45 (s, 3H), 1.25 (t, *J*=7.1 Hz, 3H). ¹³C-NMR spectrum (100 MHz (CDCl₃, HMDSO) δ: 166.8, 145.2, 134.1, 130.0, 128.3, 62.1, 35.9, 32.0, 21.7, 14.0. LCMS ESI⁺ (m/z): 270.2 [M+H]⁺, LCMS purity 100% |
| C-3539 | Methyl (2R)-1-(p-tolylsulfonyl)aziridine-2-carboxylate |
| | Molecular formula: C₁₁H₁₃NO₄S; Molecular weight: 255.29. Melting point: oil. ¹H-NMR spectrum (400 MHz): (CDCl₃, HMDSO) δ: 7.84 (d, *J*=8.3 Hz, 2H), 7.36 (d, *J*=8.3 Hz, 2H), 3.74 (s, 3H), 3.34 (dd, *J*=7.1, 4.1 Hz, 1H), 2.76 (d, *J*=7.1 Hz, 1H), 2.56 (d, *J*=4.1 Hz, 1H), 2.45 (s, 3H). ¹³C-NMR spectrum (100 MHz (CDCl₃, HMDSO) δ: 167.2, 145.3, 134.0, 129.9, 128.2, 52.9, 35.7, 32.0, 21.7. LCMS ESI⁺ (m/z): 256.1 [M+H]⁺, LCMS purity 100%. ¹³C-NMR spectrum (100 MHz (CDCl₃, HMDSO) δ: 167.3, 145.3, 134.0, 130.0, 128.3, 53.0, 35.7, 32.1, 21.7. LCMS ESI⁺ (m/z): 256.1 [M+H]⁺, LCMS purity 97.75% |
| C-3546 | N,N-Diethyl-1-(4-hexylphenyl)sulfonyl-aziridine-2-carboxamide |
| | Molecular formula: C₁₉H₃₀N₂O₃S; Molecular weight: 366.52; Melting point: oil ¹H-NMR spectrum (400 MHz): (CDCl₃, HMDSO) δ: 7.86 (d, J=8.4 Hz, 2H), 7.34 (d, J=8.4 Hz, 2H), 3.56 (dq, *J*=15.0, 7.2 Hz, 1H), 3.54 (dd, *J*=6.8, 4.2 Hz, 1H), 3.49 (dq, *J*=15.0, 7.2 Hz, 1H), 3.32-3.44 (m, 2H), 2.69 (d, *J*=4.2 Hz, 1H), 2.69 (t, *J*=7.9 Hz, 2H), 2.65 (d, *J*=6.9 Hz, 1H), 1.56-1.67 (m, 2H), 1.26-1.38 (m, 6H), 1.26 (t, *J*=7.2 Hz, 3H), 1.13 (t, *J*=7.2 Hz, 3H), 0.89 (t, *J*=7.0 Hz, 3H); ¹³C-NMR spectrum (100 MHz): (CDCl₃, HMDSO) δ: 164.3, 150.0, 134.5, 129.2, 128.2, 42.1, 41.1, 36.0, 34.9, 32.5, 31.6, 31.0, 28.8, 22.6, 14.7, 14.1, 12.8; LCMS ESI⁺ (m/z): 367.3 [M+H]⁺, LCMS purity 100% |
| C-3548 | Methyl (2S)-1-[[6-(dimethylamino)-1-naphthyl]sulfonyl]aziridine-2-carboxylate |
| | Molecular formula: C₁₆H₁₈N₂O₄S; Molecular weight: 334.39; Melting point: 118-120°C. |
| | ¹H-NMR spectrum (400 MHz): (CDCl₃, HMDSO) δ: 8.73 (d, *J*=9.6 Hz, 1H), 7.91 (d, *J*=8.3 Hz, 1H), 7.87 (dd, *J*=7.3, 1.2 Hz, 1H), 7.39 (dd, *J*=8.3, 7.4 Hz, 1H), 7.33 (dd, *J*=9.6, 2.7 Hz, 1H), 6.93 (d, *J*=2.7 Hz, 1H), 3.70 (s, 3H), 3.44 (dd, *J*=7.1, 4.1 Hz, 1H), ), 3.08 (s, 6H), 2.83 (d, *J*=7.1 Hz, 1H), 2.55 (d, *J*=4.2 Hz, 1H); ¹³C-NMR spectrum (100 MHz): (CDCl₃, HMDSO) δ: 167.3, 148.9, 136.2, 133.8, 132.0, 126.2, 125.6, 124.1, 121.5, 118.2, 106.2, 52.8, 40.4, 35.7, 32.3. LCMS ESI⁺ (m/z): 335.2 [M+H]⁺, LCMS purity 100% |
| C-3559 | Methyl 1-(3-pyridylsulfonyl)aziridine-2-carboxylate |
| | Molecular formula: C₉H₁₀N₂O₄S; Molecular weight: 242.25; Melting point: oil |
| | ¹H-NMR spectrum (400 MHz): (CDCl₃, HMDSO) δ: 9.17 (dd, *J*=2.3, 0.7 Hz, 1H), 8.90 (dd, *J*=4.9, 1.6 Hz, 1H), 8.28 (ddd, *J*=8.1, 2.3, 1.7 Hz, 1H), 7.53 (ddd, *J*=8.1, 4.9, 0.7 Hz, 1H), 3.76 (s, 3H), 3.45 (dd, *J*=7.1, 4.2 Hz, 1H), 2.87 (d, *J*=7.1 Hz, 1H), 2.65 (d, *J*=4.2 Hz, 1H); ¹³C-NMR spectrum (100 MHz): (CDCl₃, HMDSO) δ: 166.88, 154.7, 148.8, 135.9, 134.2, 124.1, 53.1, 36.0, 32.4; LCMS ESI⁺ (m/z): 243.1 [M+H]⁺, LCMS purity 92.21% |
| C-3562 | 1-(3-pyridylsulfonyl)aziridine-2-carboxamide |
| | Molecular formula: C₈H₉N₃O₃S; Molecular weight: 227.24; Melting point: 147-149°C |
| | ¹H-NMR spectrum (400 MHz): (DMSO-D₆, HMDSO) δ: 9.11 (dd, *J*=2.3, 0.7 Hz, 1H), 8.96 (dd, *J*=4.9, 1.6 Hz, 1H), 8.38 (ddd, *J*=8.1, 2.3, 1.6 Hz, 1H), 7.87 (s, 1H), 7.53 (ddd, *J*=8.1, 4.9, 0.7 Hz, 1H), 7.46 (s, 1H), 3.34-3.38 (m, 1H, overlapped with H₂O), 2.77 (d, *J*=7.2 Hz, 1H), 2.56 (d, *J*=4.5 Hz, 1H); ¹³C-NMR spectrum (100 MHz): (DMSO-D₆, HMDSO) δ: 166.69, 155.3, 148.5, 136.4, 134.1, 125.1, 37.6, 32.0; LCMS ESI⁺ (m/z): 228.1 [M+H]⁺, LCMS purity 100% |
| C-3570 | Methyl (2R)-1-[[6-(dimethylamino)-1-naphthyl]sulfonyl]aziridine-2-carboxylate |
| | Molecular formula: C₁₆H₁₈N₂O₄S; Molecular weight: 334.39; Melting point: 117-119°C |
| | ¹H-NMR spectrum (400 MHz): (CDCl₃, HMDSO) δ: 8.73 (d, *J*=9.5 Hz, 1H), 7.91 (d, *J*=8.3 Hz, 1H), 7.87 (dd, *J*=7.3, 1.2 Hz, 1H), 7.39 (dd, *J*=8.3, 7.4 Hz, 1H), 7.33 (dd, *J*=9.5, 2.7 Hz, 1H), 6.93 (d, *J*=2.7 Hz, 1H), 3.70 (s, 3H), 3.44 (dd, *J*=7.1, 4.1 Hz, 1H), ), 3.08 (s, 6H), 2.83 (d, *J*=7.1 Hz, 1H), 2.55 (d, *J*=4.2 Hz, 1H); ¹³C-NMR spectrum (100 MHz): (CDCl₃, HMDSO) δ: 167.4, 148.9, 136.3, 133.8, 132.0, 126.2, 125.6, 124.2, 121.6, 118.2, 106.3, 52.8, 40.4, 35.7, 32.4; LCMS ESI⁺ (m/z): 335.3 [M+H]⁺, LCMS purity 100% |
| C-3576 | Methyl 1-(4-fluorophenyl)sulfonylaziridine-2-carboxylate |
| | Molecular formula: C₁₀H₁₀FNO₄S; Molecular weight: 259.25; Melting point: oil |
| | ¹H-NMR spectrum (400 MHz): (CDCl₃, HMDSO) δ: 7.96-8.02 (m, 2H), 7.21-7.27 (m, 2H), 3.74 (s, 3H), 3.37 (dd, *J*=7.1, 4.1 Hz, 1H), 2.79 (d, *J*=7.1 Hz, 1H), 2.59 (d, *J*=4.1 Hz, 1H); ¹³C-NMR spectrum (100 MHz): (CDCl₃, HMDSO) δ: 167.1, 166.1 (d, *J*=257.1 Hz, 1H), 133.2 (d, *J*=3.1 Hz, 1H), 131.1 (d, *J*=9.9 Hz, 1H), 116.7 (d, *J*=22.9 Hz, 1H), 53.0, 35.9, 32.2; LCMS ESI⁺ (m/z): 260.2 [M+H]⁺, LCMS purity 100% |
| C-3591 | 1-(4-tert-Butylphenyl)sulfonylaziridine-2-carboxamide |
| | Molecular formula: C₁₃H₁₈N₂O₃S. Molecular weight: 282.36. Melting point: 151-153°C. ¹H-NMR spectrum (400 MHz): (CDCl₃, HMDSO) δ: 7.86 (d, *J*=8.8 Hz, 2H), 7.59 (d, *J*=8.8 Hz, 2H), 6.07 (br s, 1H), 5.42 (br s, 1H), 3.24 (dd, *J*=7.7, 4.2 Hz, 1H), 2.81 (d, *J*=7.7 Hz, 1H), 2.44 (d, *J*=4.2 Hz, 1H), 1.36 (s, 9H). ¹³C-NMR spectrum (100 MHz (CDCl₃, HMDSO) δ: 168.4, 158.5, 133.2, 128.1, 126.5, 37.6, 35.4, 33.2, 31.4. LCMS ESI⁺ (m/z): 283.3 [M+H]⁺, LCMS purity 100% |
| C-3593 | 1-(4-tert-Butylphenyl)sulfonyl-N-methylaziridine-2-carboxamide |
| | Molecular formula: C₁₄H₂₀N₂O₃S. Molecular weight: 296.38. Melting point: 146-148°C. ¹H-NMR spectrum (400 MHz): (CDCl₃, HMDSO) δ: 7.85 (d, *J*=8.7 Hz, 2H), 7.59 (d, *J*=8.7 Hz, 2H), 6.16 (br s, 1H), 3.28 (dd, *J*=7.7, 4.2 Hz, 1H), 2.76 (d, *J*=4.9 Hz, 3H), 2.73 (d, *J*=7.7 Hz, 1H), 2.36 (d, *J*=4.2 Hz, 1H), 1.36 (s, 9H). ¹³C-NMR spectrum (100 MHz (CDCl₃, HMDSO) δ: 166.3, 158.5, 133.1, 128.2, 126.5, 37.9, 35.4, 33.5, 31.0, 26.0. LCMS ESI⁺ (m/z): 297.3 [M+H]⁺ , LCMS purity 100% |
| C-3594 | 1-(4-tert-Butylphenyl)sulfonyl-N,N-dimethyl-aziridine-2-carboxamide |
| | Molecular formula: C₁₅H₂₂N₂O₃S. Molecular weight: 310.41. Melting point: 104-106°C. ¹H-NMR spectrum (400 MHz): (CDCl₃, HMDSO) δ: 7.88 (d, *J*=8.6 Hz, 2H), 7.56 (d, *J*=8.6 Hz, 2H), 3.23 (s, 3H), 3.60 (dd, *J*=6.8, 4.2 Hz, 1H), 3.23 (s, 3H), 2.98 (s, 3H), 2.69 (d, *J*=6.8 Hz, 1H), 2.66 (d, *J*=4.2 Hz, 1H), 1.35 (s, 9H). ¹³C-NMR spectrum (100 MHz (CDCl₃, HMDSO) δ: 165.0, 159.1, 134.2, 128.0, 126.3, 37.3, 36.0, 35.3, 35.2, 31.1. LCMS ESI⁺ (m/z): 311.3 [M+H]⁺ , LCMS purity 100% |
| C-3612 | Lithium 1-tosylaziridine-2-carboxylate |
| | Molecular formula: C₁₀H₁₀LiNO₄S; Molecular weight: 247.20; Melting point: 222-224 °C (decomp.) |
| | ¹H-NMR spectrum (400 MHz): (D₂O, HMDSO) d: 7.84 (d, *J*=8.3 Hz, 2H), 7.49 (d, *J*=8.3 Hz, 2H), 3.10 (dd, *J*=7.1, 4.1 Hz, 1H), 2.74 (d, *J*=7.1 Hz, 1H), 2.51 (d, *J*=4.1 Hz, 1H), 2.44 (s, 3H); ¹³C-NMR spectrum (100 MHz): (D₂O, HMDSO) d: 173.2, 146.7, 131.6, 130.2, 127.9, 39.0, 32.3, 20.8.LCMS ESI⁻ (m/z): 240.2 [M-Li]⁻, LCMS purity 95.83% |

### Example 2. Inhibition of PDI A1.

The inhibitory effects compounds of invention on activity of PDI A1 was assess based on the insulin turbidometric assay. Enzymatic activity of PDIA1 was confirmed by measuring the turbidity increase at 650 nm due to insulin reduction. The assay mixture was prepared by addition 10ug/ml PDIA1 (E.coli recombinant protein; Mybiosource), 0.1 mM phosphate buffer (pH7.6), 1mM EDTA, 0.087 mM DTT and with or without tested compound and was incubated for 60 min, at 37°C. Reaction was started by addition insulin and DTT. Final concentration of insulin and DTT in assay mixture was 0.15 mM and 0.174 mM, respectively. Turbidity was detected at 650 nm against reference samples without PDI A1s. The measurements were performed at 650 nm using 120-s recordings.

**TABLE 2. Inhibition of PDI A1**

| Compound Number | Inhibition of PDI A1 (EC ₅₀, µM) | Compound Number | Inhibition of PDI A1 (EC ₅₀, µM) | Compound Number | Inhibition of PDI A1 (EC ₅₀, µM) |
|---|---|---|---|---|---|
| C-3389 | 0,03 | C-3385 | 0,7 | C-3294 | 8,4 |
| C-3380 | 0,033 | C-3270 | 0,8 | C-3403 | 12,3 |
| C-3369 | 0,04 | C-3427 | 0,94 | C-3402 | 12,3 |
| C-3287 | 0,05 | C-3373 | 0,95 | C-3303 | 12,6 |
| C-3257 | 0,08 | C-3271 | 1 | C-3343 | 16 |
| C-3368 | 0,085 | C-3329 | 1,1 | C-3308 | 18 |
| C-3399 | 0,12 | C-3316 | 1,2 | C-3520 | 18,5 |
| C-3384 | 0,12 | C-3398 | 1,4 | C-3297 | 18,6 |
| C-3281 | 0,14 | C-3332 | 1,4 | C-3324 | 21,5 |
| C-3376 | 0,24 | C-3366 | 1,5 | C-3320 | 22,8 |
| C-3400 | 0,25 | C-3326 | 1,5 | C-3537 | 25 |
| C-3383 | 0,25 | C-3327 | 2,2 | C-3594 | 26 |
| C-3377 | 0,26 | C-3319 | 2,4 | C-3593 | 29 |
| C-3375 | 0,29 | C-3365 | 2,6 | C-3576 | 29 |
| C-3393 | 0,34 | C-3273 | 3 | C-3562 | 29 |
| C-3391 | 0,35 | C-3314 | 4,1 | C-3539 | 29 |
| C-3295 | 0,35 | C-3296 | 4,4 | C-3357 | 30 |
| C-3290 | 0,4 | C-3161 | 5 | C-3532 | 31 |
| C-3291 | 0,4 | C-3311 | 5 | C-3459 | 33 |
| C-3288 | 0,4 | C-3353 | 5 | C-3342 | 33,4 |
| C-3371 | 0,48 | C-3350 | 5,5 | C-3212 | 34 |
| C-3362 | 0,5 | C-3305 | 5,6 | C-3612 | 54,4 |
| C-3272 | 0,5 | C-3548 | 6 | C-3216 | 56 |
| C-3292 | 0,5 | C-3251 | 6 | C-3511 | 72,5 |
| C-3256 | 0,5 | C-3304 | 6,8 | C-3263 | 140 |
| C-3336 | 0,53 | C-3355 | 7,7 | C-3262 | 160 |
| C-3397 | 0,57 | C-3559 | 10,6 | C-3346 | >200 |
| C-3390 | 0,6 | C-3570 | 11 | C-3220 | >200 |
| C-3374 | 0,65 | C-3591 | 11 | C-3218 | >200 |
| C-3364 | 0,7 | C-3535 | 11,2 | | |
| C-3299 | 0,8 | C-3517 | 12,1 | | |

### Example 3. The in vitro antiproliferative effect of PDI A1 inhibitors toward panel of cancer cells (48-hour exposition).

Anticancer activity of compounds of invention, PDI A1-inhibitors has been tested *in vitro* in classical antiproliferative assay in various cancer cells lines Monolayer tumor cell lines MDA-MB-231 (human mammary breast adenocarcinoma), MCF-7 (human breast adenocarcinoma, estrogen-positive), HT-1080 (human fibrosarcoma) and Caco-2 (human colon adenocarcinoma) were cultured in standard medium DMEM (Dulbecco's modified Eagle's medium) ("Sigma") supplemented with 10% fetal bovine serum ("Sigma"). About 2000-4000 cells per well (depending on line nature) were placed in 96-well plates and after 24h compounds were added to the wells. Untreated cells were used as a control. The plates were incubated for 48 h, 37°C, 5% CO₂. The number of surviving cells was determined using 3-(4,5-dimethylthiazol-2-yl)-2,5-diphenyltetrazolinium bromide (MTT). MTT-test: after incubating culture medium was removed and 200 µL fresh medium with 20 µL MTT (2mg/mL in HBSS) was added in each well of the plate. After incubation (3 hr., 37°C, 5% CO₂), the medium with MTT was removed and 200 µL DMSO were added at once to each sample. The samples were tested at 540 nm on Thermo Scientific Multiskan EX microplate photometer. The half-maximal inhibitory concentration (IC₅₀) of each compound was calculated using Graph Pad Prism^{®} 3.0. The results are presented in Table 5.

**TABLE 5. The in vitro antiproliferative effect of PDI A1 inhibitors towards panel of cancer cells (48-hour exposition)**

| Comp. | Cytotoxicity, IC₅₀, µM | | | |
|---|---|---|---|---|
| | HT-1080 | CaCo-2 | MDA-MB-231 | MCF-7 |
| C-3212 | 56±9 | 45±4 | 46±8 | 54±4 |
| C-3216 | 15±2 | 40±5 | 22±1 | 16±0.2 |
| C-3218 | 200±3 | 200±1 | 64±5 | 137±14 |
| C-3220 | 5.9±1.1 | 56±4 | 45±9 | 35±6 |
| C-3256 | 215±5 | 205±29 | 8.9±1.6 | 2.8±3.2 |
| C-3262 | 3.8±0.3 | 13.5±0.8 | 6.8±0.8 | 7.2±0.8 |
| C-3263 | 7.6±0.2 | 29±0.8 | 9.1±0.5 | 17±0.2 |
| C-3270 | 21±3 | 87±4 | 52±5 | 33±4 |
| C-3271 | 49±6 | 138±11 | 13±2 | 54±4 |
| C-3272 | 4.5±0.9 | 57±6 | 8.4±0.9 | 26±0.4 |
| C-3273 | 31±0.9 | 56±12 | 35±3 | 92±2 |
| C-3281 | 164±3.2 | 125±6.4 | 15±1 | 118±5 |
| C-3287 | 92±9.2 | 144±6 | 12±2 | 123±17 |
| C-3288 | 70±2.9 | 118±3 | 10±1 | 113±4 |
| C-3290 | 9.5±1.8 | 6.9±0.2 | 7.8±1.6 | 29±4 |
| C-3291 | 140±3 | 140±5 | 32±4 | 34±1 |
| C-3292 | 34±3.4 | 14.4±1.4 | 16±1 | 25±1 |
| C-3294 | 88.5±11 | 8.1±0.7 | 40±6 | 49±6 |
| C-3295 | 74.1±2.5 | 12±1.1 | 17±1 | 32±2 |
| C-3296 | 210±15 | 37±1.5 | 30±1 | 32±1 |
| C-3297 | 154±11 | 15.4±0.7 | 5.6±0.6 | 7.5±0.5 |
| C-3299 | 56±3 | 53±7 | 18±2 | 41±2 |
| C-3303 | 70±2 | 27±2 | 102±9 | 170±35 |
| C-3304 | 17±3 | 10±1 | 13±1 | 49±1 |
| C-3305 | 190±3 | 21±1 | 12±1 | 25±1 |
| C-3308 | 120±6 | 100±5 | >500 | 485±32 |
| C-3311 | 5.1±0.2 | 7.2±0.2 | 33±4 | 124±5 |
| C-3314 | 4.5±1 | 14±0.8 | 104±3 | 206±21 |
| C-3316 | 76±3 | 81±4 | 78±12 | 137±18 |
| C-3319 | 44±4 | 31±6 | 25±5 | 28±8 |
| C-3320 | 180±8 | 78±19 | 380±15 | >500 |
| C-3324 | 510±3 | 27±3 | 102±12 | 156±9 |
| C-3326 | 100±5 | 170±20 | 169±20 | 349±13 |
| C-3327 | 130±10 | 150±10 | 161±2 | 168±23 |
| C-3329 | 33±4 | 19±2 | 6.5±0.5 | 8.2±0.8 |
| C-3332 | 140±20 | 5.9±0.3 | 44±7 | 28±3 |
| C-3336 | 4.6±0.4 | 0.5±0.1 | 7.6±1.9 | 16±3 |
| C-3342 | 6.7±2 | 6±0.5 | 17±2 | 33±2 |
| C-3343 | 6.5±0.6 | 68±4 | 26±8 | 46±5 |
| C-3346 | 169±11 | 17±2 | >200 | 458±6 |
| C-3350 | 16±5 | 49±5 | 136±6 | 80±8 |
| C-3353 | 31±1 | >500 | 92±8 | 53±9 |
| C-3355 | 20±1 | 39±1 | 247±14 | 276±9 |
| C-3357 | 30±1 | 61±3 | 25±6 | 295±9 |
| C-3362 | 32±5 | 25±6 | 19.1±1 | 40±9 |
| C-3364 | 29±4 | 11±3 | 21±0.3 | 22±2 |
| C-3365 | 28±4 | 21±4 | 4.6±0.1 | 7.3±0.5 |
| C-3366 | 11±2 | 11±2 | 20±0.2 | 9.1±0.6 |
| C-3368 | 28±1 | 28±5 | 26±3 | 16±4 |
| C-3369 | 10±2 | 22±1 | 22±2 | 21±1 |
| C-3371 | 15±3 | 25±1 | 36±7 | 11±1 |
| C-3373 | 4.8±0.8 | 43±6 | 19±1 | 19±2 |
| C-3374 | 4.6±0.3 | 25±5 | 6.7±0.2 | 4.9±0.6 |
| C-3375 | 22±4 | 55±5 | 75±4 | 12±2 |
| C-3376 | 1.3±0.1 | 11±1 | 2.3±0.2 | 27±2 |
| C-3377 | 6.1±0.3 | 16±1 | 7.8±1.4 | 4.8±0.1 |
| C-3380 | 28±4 | 157±25 | 32±7 | 44±10 |
| C-3383 | 12±1 | 50±7 | 7.3±0.5 | 80±6 |
| C-3384 | 14±3 | 56±6 | 5.5±0.6 | 40±5 |
| C-3385 | 8.0±0.7 | 20±2 | 7.0±0.7 | 16±3 |
| C-3389 | 36±3 | 49±2 | 32±3 | 47±6 |
| C-3390 | 7.9±0.9 | 11±2 | 18±0.1 | 20±0.3 |
| C-3391 | 7.8±1.0 | 38±4 | 20±0.3 | 22±2 |
| C-3393 | 0.46±0.04 | 35±1 | 32±4 | 29±3 |
| C-3397 | 9.3±1.7 | 36±5 | 38±2 | 62±4 |
| C-3398 | 7.4±0.8 | 34±6 | 17±3 | 23±3 |
| C-3399 | 7.9±0.7 | 55±7 | 17±1 | 41±6 |
| C-3400 | 11±1 | 24±2 | 24±5 | 33±1 |
| C-3402 | 4.6±0.1 | 14±2 | 39±5 | 43±8 |
| C-3403 | 6,7±0,9 | 38±5 | 16±1 | 11±3 |
| C-3427 | 5.9±0.5 | 20±5 | 9.3±1 | 13±2 |
| C-3459 | >500 | n.e. | n.e. | n.e. |
| C-3511 | n.t. | 61±4 | 5.4±0.3 | 28±3 |
| C-3517 | n.t. | 74±4 | 12±1 | 18±1 |
| C-3520 | n.t. | 45±6 | 16±2 | 30±1 |
| C-3532 | 74±1 | 181±33 | 27±2 | 103±2 |
| C-3535 | 84±3 | 35±7 | 107±11 | 81±0 |
| C-3537 | 17±1 | 27±2 | 28±3 | 35±1 |
| C-3539 | 20±1 | 23±1 | 40±5 | 113±6 |
| C-3546 | 0.13±0.02 | 73±10 | 14±1 | 32±7 |
| C-3548 | 9.2±1.0 | 45±13 | 57±9 | 37±3 |
| C-3559 | 372±23 | 260±55 | 128±8 | 154±25 |
| C-3562 | 31±2 | 36±6 | 24±2 | 57±5 |
| C-3570 | 12±1 | 13±1 | 14±3 | 29±2 |
| C-3591 | 9.9±0.9 | 27±4 | 12±3 | 16±2 |
| C-3593 | 16±1 | 25±4 | 20±1 | 9±2 |
| C-3594 | 15±1 | 7.2±0.6 | 6.1±2.4 | 10±2 |

### Example 4. Antiproliferative effect of PDIA1-inhibitors in vitro in hypoxic conditions and in cancer cells stimulated with estrogen.

Anticancer activity of PDI A1-inhibitors has been also tested *in vitro* in antiproliferative essay in normoxic and hypoxic conditions as well in estrogen-stimulated cancer cells. In the experiment, cells were seeded on 96-well plates (Sarstedt, Germany) in appropriate culture medium at a density of 10⁵ cells/mL 24h before adding the tested compounds. Cells were treated with each compound in four concentrations in the range 0,1-100 µg/mL. Cisplatin (Ebewe, Austria) in the range 0,01 -10 µg/mL was used as a reference drug. Dimethyl sulfoxide (DMSO), used as a stock solution solvent, was tested for antiproliferative activity and it did not affect cell proliferation at 0.1% (v/v) - a highest concentration used in compound solutions. After 72 h of compound treatment at 37 °C, 5 % CO₂ humid atmosphere and in wo different oxygen level conditions: 21% - normal and <1% - hypoxia. In some experiments, the MDA-MB-231 and MCF-7 cells were seeded with or without 200nM estradiol and after 24 h the tested compounds were added. A previously described sulforhodamine B antiproliferative assay was used with minor modifications (Skehan P et al., 1990). Briefly, cells were fixed with 50 µL/well of 50% (w/v) trichloroacetic acid (Avantor Performance Materials, Gliwice, Poland). After 1 h incubation, plates were washed several times with tap water and 50 µL of 0.4% (w/v) solution of sulforhodamine B (Sigma-Aldrich, Germany) in 1% (v/v) acetic acid (Avantor Performance Materials, Gliwice, Poland) was added to each well. After 30 min incubation at room temperature, unbound dye was washed out with 1% (v/v) acetic acid, whereas bound dye was solubilized with 10 mM unbuffered TRIS (Avantor Performance Materials, Gliwice, Poland) solution. The entire procedure was performed using a BioTek EL-406 washing station (BioTek Instruments, USA). After additional 30 min, absorbance was read using a Biotek Hybrid H4 reader (BioTek Instruments, USA) at 540 nm wavelength. MTT assay was used alternatively for HL-60 cell line and in the experiment in which estrogens are added (specified in the table legend). Absorbance was measured using a Biotek Hybrid H4 reader at 570 nm wavelength for MTT assay.

Compounds at each concentration were tested in triplicate in a single experiment and each experiment was repeated at least three times independently.
Results of *in vitro* antiproliferative effects are shown in Table 3 and Table 4 and are presented as mean IC₅₀ ± SD calculated using the Prolab-3 system based on Cheburator 0.4 software.

**TABLE 3. Antiproliferative effect of PDI A1 inhibitors in normal (21% of oxygen) and hypoxia (<1% of oxygen) conditions against human cancer cell lines (72- hour exposition).**

| Compd | Cell line/condition IC50 [µg/ml] | | | | | |
|---|---|---|---|---|---|---|
| | **MCF-7** | | **MDA-MB-231** | | **Caco-2** | **HT-29** |
| | normal | hypoxia | normal | hypoxia | normal | normal |
| C-3161 | 3.6 ± 0.3 | 3.6 ± 0.3 | 9.9 ± 8.2 | 11.3 ± 10.6 | 3.5 ± 0.3 | 28.38 ± 5.0 |
| C-3251 | 8.8. ± 8.2 | 7.9 ± 6.5 | 24.0 ± 6.4 | 26.5 ± 1.3 | 3.4 ± 0.2 | 30.2 ± 4.7 |
| C-3257 | 20.8 ± 6.6 | 17.1 ± 7.4 | 9.9 ± 2.8 | 13.5 ± 4.0 | 9.8 ± 3.8 | 23.8 ± 4.8 |
| CCF642 | 0.7 ± 0.5 | 0.6 ± 0.6 | 0.3 ± 0.1 | 0.3 ± 0.2 | 0.4 ± 0.1 | 0.4 ± 0.05 |
| LOC14 | 4.4 ± 0.9 | 3.9 ± 0.3 | 2.8 ± 0.4 | 2.6 ± 0.5 | 9.1 ± 3.4 | 12.6 ± 8.4 |
| Cisplatin | 1.8 ± 0.3 | 1.3 ± 0.5 | 3.2 ± 0.5 | 3.1 ± 0.9 | 2.3 ± 0.5 | 2.82 ± 0.6 |

**TABLE 4. The antiproliferative effect of PDI A1 inhibitors towards human breast cancer cell lines pretreated or not with estradiol and for comparison towards human colon cancer and leukemia cell line (72 hours exposition).**

| Cmpd | Cell line/condition IC50 [µg/ml] | | | | | | Selectivity index (SI): IC50 of MCF-10A/ IC50 of MCF-7 |
|---|---|---|---|---|---|---|---|
| | **MDA-MB-231** | | **MCF-7** | | **HT-29** | **HL-60** | |
| | **normal** | **+ estradiol** | **normal** | **+ estradiol** | **normal** | **normal** | |
| C-3380 | 26.1 ± 1.0 | 26.3 ± 2.5 | 25.5 ± 2.3 | 26.3 ± 4.3 | 34.6 ± 2.2 | 5.3 ± 2.8 | 0.75 |
| C-3389 | 23.2 ± 1.0 | 28.0 ± 1.7* | 26.7 ± 4.5 | 18.4 ± 4.3 | 31.2 ± 6.7 | 2.8 ± 0.8 | 0.23 |
| C-3353 | 24.2 ± 2.6 | 25.7 ± 1.9 | 6.2 ± 1.8 | 5.8 ± 1.3 | 35.9 ± 1.3 | 2.0 ± 0.1 | 3.36 |
| C-3287 | 6.4 ± 0.8 | 23.8 ± 2.3* | 31.3 ± 0.6 | 29.3 ± 4.1 | 31.5 ± 4.9 | 0.5 ± 0.3 | 1.10 |
| C-3326 | 15.7 ± 3.1 | 17.7 ± 2.1 | 5.4 ± 1.7 | 10.4 ± 2.4 | 39.2 ± 6.5 | 1.9 ± 0.6 | 4.84 |
| C-3251 | 26.6 ± 2.3 | 29.7 ± 1.9 | 3.9 ± 0.5 | 4.0 ± 0.2 | 37.2 ± 8.4 | 1.5 ± 0.1 | 6.62 |

| | | | | | | | |
|---|---|---|---|---|---|---|---|
| *Statistical analysis: Unpaired t test. *p<0.05 as compared to appropriate cells not pretreated with estradiol. MTT assay was used for antiproliferative activity assessment.* | | | | | | | |

In reference to Table 3 and Table 4, among all PDI A1 inhibitors tested in normal condition, the most active towards all cell lines were C-3161 and C-3251. The activity of these compounds was comparable to reference LOC14. In reference to Table 3, the antiproliferative effect of several PDIA1 inhibitors was compared at different oxygen supplies, which showed that their antiproliferative effect on human breast cancer cells MCF-7 and MDA-MB-231 is not dependent on oxygen access. Therefore, it seems that these compounds may also be active in the hypoxic environment of the tumor. In reference to Table 4, a slight decrease of antiproliferative activity towards MDA-MB-231 cell line of PDI A1 inhibitors C-3389 and C-3287 has been demonstrated during simultaneous incubation with estradiol. For the MCF-7 cell line, a slight decrease in sensitivity after addition of estradiol was observed with the inhibitor C-3326. These results suggest modulation of PDIA1 expression in cancer cells by estradiol.

### Example 5. The influence of PDI A1 inhibitors on the clonogenic potential, cell cycle and cell death of human colon cancer cell lines

To confirm, anticancer effects, selected compounds of invention, PDIA1 inhibitors were tested in long-term colony formation assay. For long-term colony formation assay the human colorectal carcinoma cell lines Caco-2 and HT-29 were maintained as follows: HT-29 in RPMI-1640 with HEPES + OPTI-MEM (1:1) and Caco-2 in Eagle's medium (all from IIET, Wroclaw, Poland) both culture media were supplemented with 2 mM L-glutamine, 1 mM sodium pyruvate (both from Sigma-Aldrich Chemie GmbH, Steinheim, Germany), fetal bovine serum: 5% HT-29 (GE Healthcare), 20% Caco-2 (Sigma-Aldrich Chemie GmbH, Steinheim, Germany), 100 U/ml penicillin, 100 µg/ml streptomycin (both from Polfa Tarchomin S.A., Warsaw, Poland). All cell lines were grown at 37°C in a humidified atmosphere with 5% CO₂. 24 h before adding the tested compounds, cells were seeded on 24-well plates (Corning, Germany) in appropriate culture medium (described for each cell line in details below) at a density of 1.5× 10⁵ cells/mL. The cells were treated with tested compounds in the doses of IC50 for each compound and collected after three more days. The viable cells were counted using a hemocytometer (trypan blue exclusion method) and seeded in triplicates at a density of 5 × 10² cells/3 ml and 2,5 × 10² cells/3 ml (9,5 cm²) for Caco-2 and HT-29 respectively. The dishes had been pre-coated with poly-L-lysine/PBS (0.001%; Sigma-Aldrich) and washed twice with PBS (with Ca²⁺ and Mg²⁺). After 2 weeks, the colonies were fixed and stained with 1% crystal violet/ethanol (Sigma-Aldrich), documented with Sony Alpha 300 camera (Sony), and counted manually using ImageJ 1.47 software (National Institutes of Health, Bethesda, MD, USA). Surviving fraction (SF) was calculated. The concentrations of disclosed compounds were chosen on the basis of IC50 values CCF642: 0.4 µg/ml, LOC14: 10 µg/ml on both cell lines; C-3251: 30 µg/ml on HT-29 and 3 µg/ml on Caco-2.

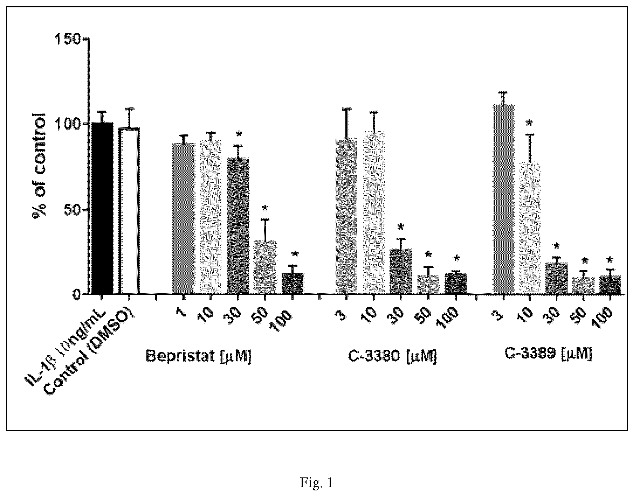

In reference to fig. 1A and 1B, the ability of C-3251 (as the representative for PDI A1 inhibitors) to inhibit long-term colonies formation was much potent towards HT-29 than CaCo-2 cell line, opposite to the results of proliferation inhibition test. Reference compounds inhibit colonies formation only of Caco-2 cell line (Fig. 1A and 1B). Fig. 1B shows representative plates with the use of disclosed compounds from clonogenic assay.

Cell cycle and cell death analysis has been performed as previously described (Milczarek M et al. , 2015). Compounds were tested 5 times independently. Obtained results were analyzed using BD FACSDiva 6.2 software. In reference to fig. 1C, C-3251 increased the percentage of CaCo-2 cells in S cell cycle phase. This compound decreased HT-29 cells in G0/G1 and increased in G2/M phase. In reference to fig 1D, C-3251 also increased significantly the percentage of death HT-29 cells.

Caspase-3/7 activity assay has been performed as previously described (Milczarek M et al. , 2015) after 24 and 48 h of incubation with disclosed compounds. Compounds were tested in triplicate in a single experiment and each experiment was repeated three times independently. Results were normalized to the protein content using the SRB method and reported as mean relative caspase-3/7 activity compared to the control sample ± SD.

In reference to fig. 1E and fig 1F, caspase 3/7 induction was higher on HT-29 cell line after incubation with all compounds as compare to Caco-2 cell line. C-3251 significantly increase caspase 3/7 activity towards Caco-2 cells after 48h treatment and towards HT-29 cells after 24 and 48h of incubation. Dashed line designated control level. Statistical analysis: Dunn's or Dunett's multiple comparison tests. *p<0.05 as compared to control.7

### Example 6. Evaluation of effects of PDI A1 inhibitors on transendothelial cancer cells migration in in vitro model

To assess whether compounds of invention, PDI A1-inhibitors are also effective as inhibitors of cancer cell transmigration through endothelium the transmigration assay with MDA-MB-231/lung microvascular endothelium was used as described previously (Stojak et al., 2018). Cell migration was assayed in 24-well, 6.5-mm internal-diameter Transwell plates (8.0 µM pore size; BD Pharmingen). Human lung microvascular endothelial cells (hLMVECs) were seeded into 24-well plates (seeding density 5×10⁴ cells/insert) on the upper side of the filter and left to grow to confluence. After confluent monolayer formation, hLMVECs were pre-treated with 10 ng/mL IL-1β for 6 h. Prior to use in transmigration assay, cancer cells were pre-incubated with various concentrations (3, 10, 30, 50, 100 µM) of tested inhibitors of PDIA1, C-3380, C-3389 for 30 min. Then, MDA-MB-231 cells (each 5×10⁴ per well) were placed into upper chambers and tested PDI A1 inhibitors (see above) or bepristat, a reference pharmacological inhibitor of PDIA1, at various concentrations (1, 10, 30, 50, 100 µM) were given. Lower chambers were filled with medium containing chemoattractant (20% FBS or 100 ng/mL SDF-1α). After 24 h of co-culture, hLMVEC monolayers and non-migrating cancer cells on the upper surface of the membrane were removed. Migrated cancer cells on the undersides of the Transwell membranes were detached and stained by Calcein-AM-Accutase solution for 60 minutes. The cell number was determined by measuring the fluorescence using plate reader. Experiments were performed in triplicates and repeated three times.

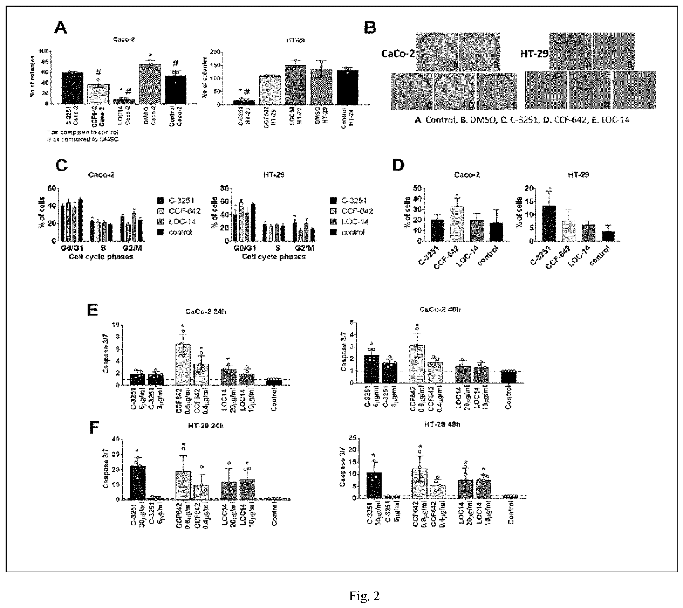

In reference to fig. 2A, it is shown the influence of bepristat 2a and C-3380 on MDA-MB-231 cell transmigration across IL-1β 10 ng/mL (6 hours) - stimulated hLMVECs. The number of migrating MDA-MB-231 cells through the hLMVEC monolayer was quantified by measuring the fluorescence, as described in *Methodology.* Data represent mean ± SD of three independent experiments. Statistical analysis was performed using one-way ANOVA. Symbols mark the statistical significance levels as follows: (*) indicates p<0.05 as compared to IL-1β 10 ng/mL stimulated group.

In reference to fig. 2B, it is shown the influence of bepristat and C-3389 on MDA-MB-231 cell transmigration across IL-1β 10 ng/mL (6 hours) - stimulated hLMVECs. The number of migrating MDA-MB-231 cells through the hLMVEC monolayer was quantified by measuring the fluorescence, as described in *Methodology.* Data represent mean ± SD of three independent experiments. Statistical analysis was performed using one-way ANOVA. Symbols mark the statistical significance levels as follows: (*) indicates p<0.05 as compared to IL-1β 10 ng/mL stimulated group.

Tested compounds inhibited transmigration of breast cancer cells across hLMVEC monolayer in a concentration -dependent manner.

### Example 7. Anticancer effects of PDI A1-inhibitors in vivo

To confirm anticancer activity of selected compounds of invention, PDI A1-inhibitors in vivo, selected compounds of invention were tested in the murine model of Lewis Lung Carcinoma (LLC). The Lewis Lung Carcinoma (LLC) cell line was obtained from the ATCC collection and was cultured in a humidified atmosphere of 5% CO₂ at 37⁰ using Dulbecco's modified Eagle's medium (DMEM) that contained 10% bovine serum (Sigma). Female (6-8 weeks old) C57BL/6 mice (purchased from Envigo RMS. BV., Nederland) were acclimated for one week and were fed with animal chow and water *ad libitum.* The C57BL/6 mice were injected s.c. with 0.5 × 10⁵ LLC cells per mice in 100 µL PBS. Compounds were dissolved in DMSO and then 0.9% NaCl (DMSO final concentration 1%) were added. In each treatment group, i.p. injections of 100 µl compound were started on 1 day after tumor inoculation. Control animals injected with equal volume of 0.9% NaCl with 1% DMSO administered in the same schedule as other groups. Tumor larger radius (a) and smaller radius (b) were measured by calipers. The tumor volume (Tv) was calculated according the following formulaTv = V=4· ab2/3. The mice in control group were sacrificed 17 days after tumor establishment. Tumor tissues were dissected, weighed and frozen in OCT compound.

**TABLE 6. The individual tumor volumes (mm3) of mice in group treated with C-3161 and control group**

| Nr.of animal treated with C-3161 (50 mg/kg) | Day of experiment | | | | |
|---|---|---|---|---|---|
| | 10 | 12 | 14 | 16 | 17 |
| 1 | 258 | 496 | 829 | 980 | 946 |
| 2 | 0 | 0 | 254 | 199 | 476 |
| 3 | 0 | 0 | 0 | 0 | 0 |
| 4 | 0 | 0 | 0 | 0 | 0 |
| 5 | 266 | 365 | 642 | 719 | 419 |
| average±stdev | 105±144 | 172±240 | 345±377 | 380±446 | 368±393 |
| | | | | | |

| Nr.of animal (control group) | | | | | |
|---|---|---|---|---|---|
| 1 | 0 | 617 | 875 | 1206 | 1255 |
| 2 | 201 | 305 | 435 | 576 | 548 |
| 3 | 151 | 389 | 1110 | 1338 | 1354 |
| 4 | 0 | 429 | 1119 | 1182 | 1176 |
| 5 | 60 | 412 | 979 | 1486 | 1714 |
| average±stdev | 82±91 | 430±115 | 904±281 | 1158±347 | 1209±423 |
| TTEST^{a} | 0.78 | 0.06 | 0.03 | 0.02 | 0.01 |

**TABLE 7. The individual tumor volumes (mm3) of mice in group C-3257 and control group**

| Nr.of animal treated with C-3257 (75mg/kg) | Day of experiment | | | | |
|---|---|---|---|---|---|
| | 10 | 12 | 14 | 16 | 17 |
| 1 | 0 | 572 | 594 | 794 | 674 |
| 2 | 0 | 0 | 0 | 548 | 882 |
| 3 | 329 | 468 | 537 | 601 | 1016 |
| 4 | 0 | 0 | 657 | 772 | 1016 |
| 5 | 0 | 0 | 523 | 717 | 986 |
| average±stdev | 66±147 | 208±287 | 462±264 | 686±108 | 1097±482 |
| | | | | | |

| Nr.of animal (control group) | | | | | |
|---|---|---|---|---|---|
| 1 | 0 | 617 | 875 | 1206 | 1255 |
| 2 | 201 | 305 | 435 | 576 | 548 |
| 3 | 151 | 389 | 1110 | 1338 | 1354 |
| 4 | 0 | 429 | 1119 | 1182 | 1176 |
| 5 | 60 | 412 | 979 | 1486 | 1714 |
| average±stdev | 82±91 | 430±115 | 904±281 | 1158±347 | 1209±423 |
| TTEST^{a} | 0.36 | 0.25 | 0.13 | 0.04 | 0.03 |

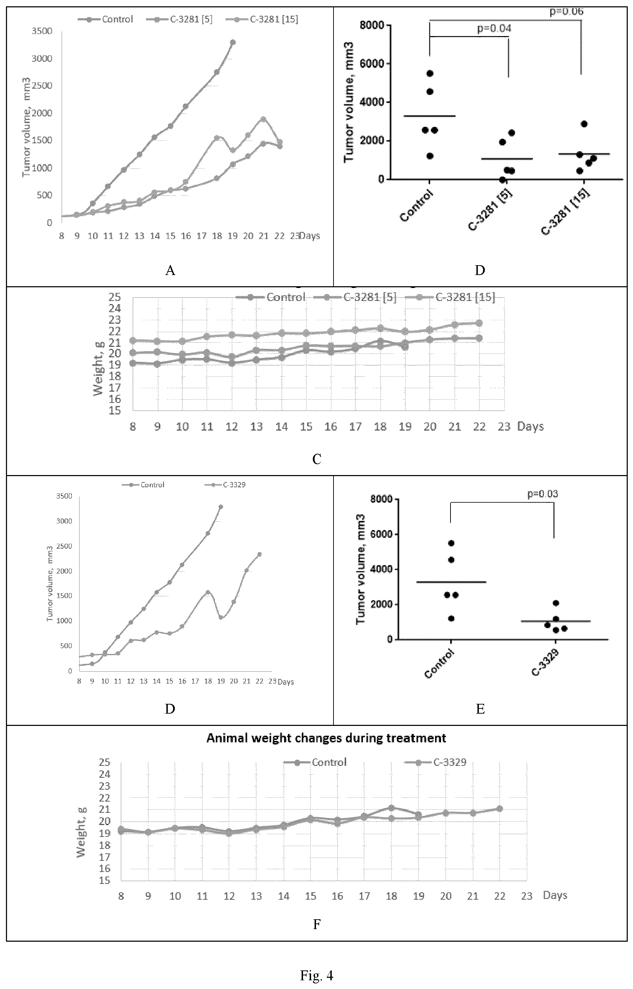

In reference to Tables 6,and 7, it was established that compounds of invention: C-3161, C-3257 possess anti-cancer activity in vivo. Results of anticancer activities of C-3161, C-3257 are also shown in Figure 4.

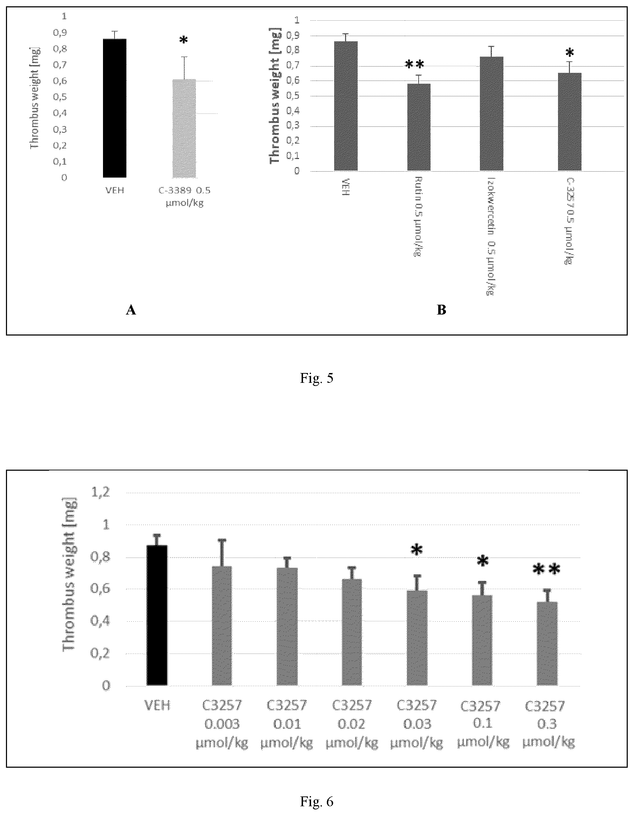

In reference to figure 5, anti-cancer effects were also shown for the compounds C-3281 and C-3329. These compounds diminished tumour volume but did not modified animal weight suggesting that they display clear-cut anticancer activity *in vivo,* without evident toxicity as evidenced by lack of the effects on the animal weight.

### Example 8. Evaluation of anti-thrombotic effects of PDIA1 inhibitors in vivo

To confirm antithrombotic activity of compounds of invention, inhibitors of PDIA1, pharmacological activity of selected compounds was tested *in vivo* in the rat model of arterial thrombosis. Wistar rats were anaesthetized with pentobarbital (40 mg/kg, i.p.) and placed in a supine position on a heated (37 °C) operating table. Arterial thrombosis was induced by electrical stimulation of the right common carotid artery, as previously described (Kramkowski et al., 2012). Briefly, the anode, a stainless steel L-shaped wire, was inserted under the artery and connected to a constant current generator. The cathode was attached subcutaneously to the hind limb. The artery was stimulated (1 mA) for 10 min. Fifty-five minutes after the beginning of stimulation, the segment of the common carotid artery containing the formed thrombus was dissected and opened lengthwise, and the thrombus was completely removed and air-dried at room temperature for 24 h. Thrombus was then weighed in a blinded manner. As shown in fig 5, reference PDI inhibitors rutin inhibited thrombus formation in vivo in the rat model of arterial thrombosis while isoquercetin was less effective. Fig 5 also shows anti-thrombotic effects of C3989 and C-3257 two among compounds of invention in *in vivo* rat model of arterial thrombosis. Fig. 6 shows dose-dependent effects induced by C-3257 on thrombus formation in *in vivo* rat model of arterial thrombosis. As can be noted in fig. 6 effects of C-3257 on thrombus formation in vivo was significant and pronounced at a dose as low as 0.03 µmol/kg.

### Bibliography

Atkin et al. (2008) Neurobiol Dis 30(3):400-407.
Barbouche et al. (2003) J Biol Chem 278(5):3131-3136.
Bennett et al., (2000) J. Immunol. 164(8), 4120-4129
Burgess et al. (2000) J. Biol Chem. 275(13):9758-9766;
Cho et al. (2008) J Clin Invest 118(3): 1123-1131.
Colla et al. (2012) J Neurosci 32(10):3306-3320.
Darby N J and Creighton T E (1995) Biochemistry. 34(37):11725-11735.
Essex et al. (1999) Br J. Haematol. 104(3):448-454;
Ge et al. (2013) ACS Chem Biol 8(11):2577-2585.
Hashida et al. (2011) J Toxicol Sci 36(1):1-7.
Hatahet et al. (2009) Antioxid Redox Signal. 11(11), 2807-2850
Hoffstrom et al. (2010) Nat Chem Biol 6(12):900-906.
Hotchkiss et al., (1998) Biochim Biophys Acta. 1388(2), 478-488;
Jasuja et al. (2010) Blood 116(22), 4665-4674
Jasuja et al. (2012) J Clin Invest 122(6):2104-2113.
Karala et al. (2010) FEBS J. 277(11), 2454-2462
Khan et al. (2011) ACS Chem. Biol. 6(3), 245-251
Koivu et al. (1987) J Biol Chem 262(14):6447-6449.
Kramkowski et al. (2012) Arterioscler Thromb Vasc Biol. 32:2149-2157.
Lovat et al. (2008) Cancer Res 68(13):5363-5369.
Lee et al., (2017) BMB Rep. 2017;50(8):401-410
Manickam et al. (2008) Br J Haematol. 140(2), 223-229.
Milczarek M et al. (2014) Oncol Rep. 32:491-504.
Milczarek M et al. (2019) J Steroid Biochem Mol Biol. 190:139-51.
Pawlak A et al. (2016) BMC Cancer. 16:893.
Reinhardt et al. (2008) J. Clin Invest. 118(3), 1110-1122
Rossowska J et al. (2017) Front Immunol. 8:713. 3.
Skehan P et al. (1990) JNCI J Natl Cancer Inst. 82:1107-12.
Stojak et al., (2018), Pharmacol Res. 2018;136:160-171
Wetterau et al. (1990) J Biol Chem 265(17):9800-9807.
Xu et al. (2012) Proc Natl Acad Sci USA 109(40):16348-16353.
Yoo et al. (2002) Neurosci Lett 334(3):196-200.

## Claims

1. N,N-disubstituted aromatic sulphonamides in form of racemates or enantiomers, or a pharmaceutically acceptable salt, wherein the compounds are chosen from the list:
1-(4-Nitrophenyl)sulfonylaziridine-2-carboxamide(C-3216)
1-(4-Aminopheny1)sulfonylaziridine-2-carboxamide (C-3218)
Methyl 1-[4-(trifluoromethyl)phenyl]sulfonylaziridine-2-carboxylate (C-3256)
Methyl 1-(4-butylphenyl)sulfonylaziridine-2-carboxylate (C-3257)
Methyl 1-(4-dodecy1pheny1)sulfonylaziridine-2-carboxylate (C-3270)
Methyl 1-(4-butoxyphenyl)sulfonylaziridine-2-carboxylate (C-3271)
1-[1-(4-Butylphenyl)sulfonylaziridin-2-yl]ethanone (C-3272)
1-(4-Butylpheny1)sulfonylaziridine-2-carbaldehyde (C-3273)
Methyl 1-(4-pentylpheny1)sulfonylaziridine-2-carboxylate (C-3281)
Methyl 1-(4-hexylphenyl)sulfonylaziridine-2-carboxylate (C-3287)
Methyl 1-(4-heptylphenyl)sulfonylaziridine-2-carboxylate (C-3288)
Methyl 1-(4-tert-butylphenyl)sulfonylaziridine-2-carboxylate (C-3290)
Methyl 1-(4-phenylphenyl)sulfonylaziridine-2-carboxylate (C-3291)
Methyl 1-(2-naphthylsulfonyl)aziridine-2-carboxylate (C-3292)
Methyl 1-(4-isopropylphenyl)sulfonylaziridine-2-carboxylate (C-3295)
Methyl 1-(2-fluoro-4-methyl-phenyl)sulfonylaziridine-2-carboxylate (C-3297)
Methyl 1-(4-propylpheny1)sulfonylaziridine-2-carboxylate (C-3299)
Methyl 1-(m-tolylsulfonyl)aziridine-2-carboxylate (C-3303)
Methyl 1-[3-(trifluoromethy1)phenyl]sulfonylaziridine-2-carboxylate (C-3305)
Methyl 1-(4-acetamidophenyl)sulfonylaziridine-2-carboxylate (C-3308)
Methyl 1-(1-naphthylsulfonyl)aziridine-2-carboxylate (C-3311)
Methyl 1-(4-cyclohexylphenyl)sulfonylaziridine-2-carboxylate (C-3316)
Methyl 1-(4-octylphenyl)sulfonylaziridine-2-carboxylate (C-3319)
Methyl 1-(o-tolylsulfonyl)aziridine-2-carboxylate (C-3320)
1-[4-(Trifluoromethyl)phenyl]sulfonylaziridine-2-carboxamide (C-3324)
Methyl 1-[[5-(dimethylamino)-1-naphthyl]sulfonyl]aziridine-2-carboxylate (C-3326)
Methyl 1-[(6-methoxy-2-naphthyl)sulfonyl]aziridine-2-carboxylate (C-3327)
Methyl 1-[(6-methyl-2-naphthyl)sulfonyl]aziridine-2-carboxylate (C-3329)
Methyl 1-(4-ethylphenyl)sulfonylaziridine-2-carboxylate (C-3332)
Methyl 1-[4-(dimethylamino)phenyl]sulfonylaziridine-2-carboxylate (C-3343)
Methyl 1-(2,4-difluorophenyl)sulfonylaziridine-2-carboxylate (C-3353)
1-[4-(Dimethylamino)pheny1]sulfonyl-N,N-dimethyl-aziridine-2-carboxamide (C-3357)
1-(4-Butylpheny1)sulfonyl-N,N-dimethyl-aziridine-2-carboxamide (C-3362)
1-(4-Isopropylpheny1)sulfonyl-N,N-dimethyl-aziridine-2-carboxamide (C-3364)
N,N-Dimethy1-1-(1-naphthylsulfony1)aziridine-2-carboxamide (C-3365)
N,N-Dimethyl-1-[(4-methyl-1-naphthy1)sulfonyl]aziridine-2-carboxamide (C-3366)
N,N-Dimethyl-1-(4-pentylphenyl)sulfonyl-aziridine-2-carboxamide (C-3368)
1-(4-Hexylpheny1)sulfonyl-N,N-dimethyl-aziridine-2-carboxamide (C-3369)
N,N-Dimethyl-1-[4-(trifluoromethyl)phenyl]sulfonyl-aziridine-2-carboxamide (C-3374)
1-(4-Hexylphenyl)sulfonyl-N-methyl-aziridine-2-carboxamide (C-3380)
Methyl 1-[[5-(dimethylamino)-2-naphthyl]sulfonyl]aziridine-2-carboxylate (C-3399)
1-(4-Hexylpheny1)sulfonylaziridine-2-carboxamide (C-3389)
1-(4-Cyclohexylphenyl)sulfonyl-N,N-dimethyl-aziridine-2-carboxamide (C-3384)
1-(2,4-Difluorophenyl)sulfonylaziridine-2-carboxamide (C-3402)
1-(2,4-Difluorophenyl)sulfonyl-N,N-dimethyl-aziridine-2-carboxamide (C-3403)
1-[[6-(Dimethylamino)-1-naphthyl]sulfonyl]aziridine-2-carboxamide (C-3459)
[1-(4-Hexylpheny1)sulfonylaziridin-2-yl]-pyrrolidin-1-yl-methanone (C-3511)
[1-(4-Hexylpheny1)sulfonylaziridin-2-y1]-morpholino-methanone (C-3517)
Methyl 1-(4-fluorophenyl)sulfonylaziridine-2-carboxylate (C-3576)
N,N-Diethyl-1-(4-hexylpheny1)sulfonyl-aziridine-2-carboxamide (C-3546)
1-(4-tert-Butylpheny1)sulfonylaziridine-2-carboxamide (C-3591)
1-(4-tert-Butylphenyl)sulfonyl-N,N-dimethyl-aziridine-2-carboxamide (C-3594)
1-(4-tert-Butylpheny1)sulfonyl-N-methylaziridine-2-carboxamide (C-3593).

2. N,N-disubstituted aromatic sulphonamides or a pharmaceutically acceptable salt selected from the list for use in the treatment or prevention of diseases linked to PDI A1:
Methyl 1-(p-tolylsulfonyl)aziridine-2-carboxylate (C-3161);
Methyl 1-(4-nitrophenyl)sulfonylaziridine-2-caiboxylate (C-3212);
1-(4-Nitrophenyl)sulfonylaziridine-2-carboxamide (C-3216);
1-(4-Aminophenyl)sulfonylaziridine-2-carboxamide (C-3218);
1-(p-Tolylsulfonyl)aziridine-2-carboxamide (C-3220);
Methyl 1-(benzenesulfonyl)aziridine-2-carboxylate (C-3251);
Methyl 1-[4-(trifluoromethyl)phenyl]sulfonylaziridine-2-carboxylate (C-3256);
Methyl 1-(4-butylphenyl)sulfonylaziridine-2-carboxylate (C-3257);
1-(p-Tolylsulfonyl)aziridine-2-carbaldehyde (C-3262);
1-[1-(p-Tolylsulfonyl)aziridin-2-yl]ethanone (C-3263);
Methyl 1-(4-dodecylphenyl)sulfonylaziridine-2-carboxylate (C-3270);
Methyl 1-(4-butoxyphenyl)sulfonylaziridine-2-carboxylate (C-3271);
1-[1-(4-Butylphenyl)sulfonylaziridin-2-yl]ethanone (C-3272);
1-(4-Butylphenyl)sulfonylaziridine-2-carbaldehyde (C-3273);
Methyl 1-(4-pentylphenyl)sulfonylaziridine-2-carboxylate (C-3281);
Methyl 1-(4-hexylphenyl)sulfonylaziridine-2-carboxylate (C-3287);
Methyl 1-(4-heptylphenyl)sulfonylaziridine-2-carboxylate (C-3288);
Methyl 1-(4-tert-butylphenyl)sulfonylaziridine-2-carboxylate (C-3290);
Methyl 1-(4-phenylphenyl)sulfonylaziridine-2-carboxylate (C-3291);
Methyl 1-(2-naphthylsulfonyl)aziridine-2-carboxylate (C-3292);
Methyl 1-(4-methoxyphenyl)sulfonylaziridine-2-carboxylate (C-3294);
Methyl 1-(4-isopropylphenyl)sulfonylaziridine-2-carboxylate (C-3295);
Methyl 1-(4-chlorophenyl)sulfonylaziridine-2-carboxylate (C-3296);
Methyl 1-(2-fluoro-4-methyl-phenyl)sulfonylaziridine-2-carboxylate (C-3297);
Methyl 1-(4-propylphenyl)sulfonylaziridine-2-carboxylate (C-3299);
Methyl 1-(m-tolylsulfonyl)aziridine-2-carboxylate (C-3303);
Methyl 1-(4-propylphenyl)sulfonylaziridine-2-carboxylate (C-3304);
Methyl 1-[3-(trifluoromethyl)phenyl]sulfonylaziridine-2-carboxylate (C-3305);
Methyl 1-(4-acetamidophenyl)sulfonylaziridine-2-carboxylate (C-3308);
Methyl 1-(1-naphthylsulfonyl)aziridine-2-carboxylate (C-3311);
1-(p-Tolylsulfonyl)aziridine-2-carbonitrile (C-3314);
Methyl 1-(4-cyclohexylphenyl)sulfonylaziridine-2-carboxylate (C-3316);
Methyl 1-(4-octylphenyl)sulfonylaziridine-2-carboxylate (C-3319);
Methyl 1-(o-tolylsulfonyl)aziridine-2-carboxylate (C-3320);
1-[4-(Trifluoromethyl)phenyl]sulfonylaziridine-2-carboxamide (C-3324);
Methyl 1-[[5-(dimethylamino)-1-naphthyl]sulfonyl]aziridine-2-carboxylate (C-3326);
Methyl 1-[(6-methoxy-2-naphthyl)sulfonyl]aziridine-2-carboxylate (C-3327);
Methyl 1-[(6-methyl-2-naphthyl)sulfonyl]aziridine-2-carboxylate (C-3329);
Methyl 1-(4-ethylphenyl)sulfonylaziridine-2-carboxylate (C-3332);
Methyl 1-[(4-methyl-1-naphthyl)sulfonyl]aziridine-2-carboxylate (C-3336);
N,N-Dimethyl-1-(p-tolylsulfonyl)aziridine-2-carboxamide (C-3342);
Methyl 1-[4-(dimethylamino)phenyl]sulfonylaziridine-2-carboxylate (C-3343);
Methyl 1-(2-furylsulfonyl)aziridine-2-carboxylate (C-3346);
Methyl 1-[(2-oxo-1H-quinolin-6-yl)sulfonyl]aziridine-2-carboxylate (C-3350);
Methyl 1-(2,4-difluorophenyl)sulfonylaziridine-2-carboxylate (C-3353);
Methyl 1-(2-oxochromen-6-yl)sulfonylaziridine-2-carboxylate (C-3355);
1-[4-(Dimethylamino)phenyl]sulfonyl-N,N-dimethyl-aziridine-2-carboxamide (C-3357);
1-(4-Butylphenyl)sulfonyl-N,N-dimethyl-aziridine-2-carboxamide (C-3362);
1-(4-Isopropylphenyl)sulfonyl-N,N-dimethyl-aziridine-2-carboxamide (C-3364);
N,N-Dimethyl-1-(1-naphthylsulfonyl)aziridine-2-carboxamide (C-3365);
N,N-Dimethyl-1-[(4-methyl-1-naphthyl)sulfonyl]aziridine-2-carboxamide (C-3366);
N,N-Dimethyl-1-(4-pentylphenyl)sulfonyl-aziridine-2-carboxamide (C-3368);
1-(4-Hexylphenyl)sulfonyl-N,N-dimethyl-aziridine-2-carboxamide (C-3369);
N,N-Dimethyl-1-[4-(trifluoromethyl)phenyl]sulfonyl-aziridine-2-carboxamide (C-3374);
1-(4-Hexylphenyl)sulfonyl-N-methyl-aziridine-2-carboxamide (C-3380);
1-(4-Cyclohexylphenyl)sulfonyl-N,N-dimethyl-aziridine-2-caiboxamide (C-3384);
1-(4-Hexylphenyl)sulfonylaziridine-2-carboxamide (C-3389);
Methyl 1-[[5-chloro-6-(methylamino)-2-naphthyl]sulfonyl]aziridine-2-carboxylate (C-3393);
1-(2,4-Difluorophenyl)sulfonylaziridine-2-carboxamide (C-3402);
1-(2,4-Difluorophenyl)sulfonyl-N,N-dimethyl-aziridine-2-carboxamide (C-3403);
1-[[6-(Dimethylamino)-1-naphthyl]sulfonyl]aziridine-2-carboxamide (C-3459);
[1-(4-Hexylphenyl)sulfonylaziridin-2-yl]-pyrrolidin-1-yl-methanone (C-3511);
[1-(4-Hexylphenyl)sulfonylaziridin-2-yl]-morpholino-methanone (C-3517);
1-(Benzenesulfonyl)aziridine-2-carboxamide (C-3532);
Methyl (2S)-1-(p-tolylsulfonyl)aziridine-2-carboxylate (C-3535);
Ethyl 1-(p-tolylsulfonyl)aziridine-2-carboxylate (C-3537);
Methyl (2R)-1-(p-tolylsulfonyl)aziridine-2-carboxylate (C-3539);
N,N-Diethyl-1-(4-hexylphenyl)sulfonyl-aziridine-2-carboxamide (C-3546);
Methyl 1-(3-pyridylsulfonyl)aziridine-2-carboxylate (C-3559);
1-(3-pyridylsulfonyl)aziridine-2-carboxamide (C-3562);
Methyl (2R)-1-[[6-(dimethylamino)-1-naphthyl]sulfonyl]aziridine-2-carboxylate (C-3570);
Methyl 1-(4-fluorophenyl)sulfonylaziridine-2-carboxylate (C-3576);
1-(4-tert-Butylphenyl)sulfonylaziridine-2-carboxamide (C-3591);
1-(4-tert-Butylphenyl)sulfonyl-N-methylaziridine-2-carboxamide (C-3593);
1-(4-tert-Butylphenyl)sulfonyl-N,N-dimethyl-aziridine-2-carboxamide (C-3594);
Lithium 1-tosylaziridine-2-carboxylate (C-3612).

3. N,N-disubstituted aromatic sulphonamides for use according to claim 2, in the treatment and prevention of excessive platelet activation and thrombosis, in particular any disease from the list:
disease or condition is thrombosis, thrombotic diseases, in particular the thrombotic disease is acute myocardial infarction, stable angina, unstable angina, acute occlusion following coronary angioplasty and/or stent placement, transient ischemic attacks, cerebrovascular disease, peripheral vascular disease, placental insufficiency, atrial fibrillation, deep vein thrombosis or pulmonary embolism and other pathologies linked with excessive activation of platelets.

4. N,N-disubstituted aromatic sulphonamides for use according to claim 2, in the treatment and prevention of cancer, in particular any disease from the list:
gastrointestinal cancer, colorectal cancer, colon cancer, liver cancer, hepatocellular carcinoma, pancreatic cancer, biliary tract cancer, stomach cancer, genitourinary cancer, bladder cancer, testicular cancer, cervical cancer, malignant mesothelioma, osteogenic sarcoma, esophageal cancer, laryngeal cancer, prostate cancer, hormone-refractory prostate cancer, lung cancer, small cell lung cancer, non-small cell lung cancer, breast cancer, triple-negative breast cancer, breast cancer having a BRCA1 and/or BRCA2 gene mutation, hematological cancer, leukemia, acute lymphoblastic leukemia, acute myeloid leukemia, chronic lymphocytic leukemia, chronic myeloid leukemia, lymphoma, Hodgkin lymphoma, non-Hodgkin lymphoma, follicular lymphoma, diffuse large B-cell lymphoma, ovarian cancer, brain cancer, neuroblastoma, Ewing's sarcoma, kidney cancer, epidermoid cancer, skin cancer, melanoma, head and/or neck cancer, head and neck squamous cell carcinoma, and mouth cancer.

## Patentansprüche

1. N,N-disubstituierte aromatische Sulfonamide in Form von Racematen oder Enantiomeren oder ein pharmazeutisch akzeptables Salz davon, wobei die Verbindungen aus der folgenden Liste ausgewählt sind:
1-(4-Nitrophenyl)sulfonylaziridin-2-carboxamid (C-3216)
1-(4-Aminophenyl)sulfonylaziridin-2-carboxamid (C-3218)
Methyl-1-[4-(trifluormethyl)phenyl]sulfonylaziridin-2-carboxylat (C-3256)
Methyl-1-(4-butylphenyl)sulfonylaziridin-2-carboxylat (C-3257)
Methyl-1-(4-dodecylphenyl)sulfonylaziridin-2-carboxylat (C-3270)
Methyl-1-(4-butoxyphenyl)sulfonylaziridin-2-carboxylat (C-3271)
1-[1-(4-Butylphenyl)sulfonylaziridin-2-yl]ethanon (C-3272)
1-(4-Butylphenyl)sulfonylaziridin-2-carbaldehyd (C-3273)
Methyl-1-(4-pentylphenyl)sulfonylaziridin-2-carboxylat (C-3281)
Methyl-1-(4-hexylphenyl)sulfonylaziridin-2-carboxylat (C-3287)
Methyl-1-(4-heptylphenyl)sulfonylaziridin-2-carboxylat (C-3288)
Methyl-1-(4-tert-butylphenyl)sulfonylaziridin-2-carboxylat (C-3290)
Methyl-1-(4-phenylphenyl)sulfonylaziridin-2-carboxylat (C-3291)
Methyl-1-(2-naphthylsulfonyl)aziridin-2-carboxylat (C-3292)
Methyl-1-(4-isopropylphenyl)sulfonylaziridin-2-carboxylat (C-3295)
Methyl-1-(2-fluor-4-methylphenyl)sulfonylaziridin-2-carboxylat (C-3297)
Methyl-1-(4-propylphenyl)sulfonylaziridin-2-carboxylat (C-3299)
Methyl-1-(m-tolylsulfonyl)aziridin-2-carboxylat (C-3303)
Methyl-1-[3-(trifluormethyl)phenyl]sulfonylaziridin-2-carboxylat (C-3305)
Methyl-1-(4-acetamidophenyl)sulfonylaziridin-2-carboxylat (C-3308)
Methyl-1-(1-naphthylsulfonyl)aziridin-2-carboxylat (C-3311)
Methyl-1-(4-cyclohexylphenyl)sulfonylaziridin-2-carboxylat (C-3316)
Methyl-1-(4-octylphenyl)sulfonylaziridin-2-carboxylat (C-3319)
Methyl-1-(o-tolylsulfonyl)aziridin-2-carboxylat (C-3320)
1-[4-(Trifluormethyl)phenyl]sulfonylaziridin-2-carboxamid (C-3324)
Methyl-1-[[5-(dimethylamino)-1-naphthyl]sulfonyl]aziridin-2-carboxylat (C-3326)
Methyl-1-[(6-methoxy-2-naphthyl)sulfonyl]aziridin-2-carboxylat (C-3327)
Methyl-1-[(6-methyl-2-naphthyl)sulfonyl]aziridin-2-carboxylat (C-3329)
Methyl-1-(4-ethylphenyl)sulfonylaziridin-2-carboxylat (C-3332)
Methyl-1-[4-(dimethylamino)phenyl]sulfonylaziridin-2-carboxylat (C-3343)
Methyl-1-(2,4-difluorphenyl)sulfonylaziridin-2-carboxylat (C-3353)
1-[4-(Dimethylamino)phenyl]sulfonyl-N,N-dimethylaziridin-2-carboxamid (C-3357)
1-(4-Butylphenyl)sulfonyl-N,N-dimethylaziridin-2-carboxamid (C-3362)
1-(4-Isopropylphenyl)sulfonyl-N,N-dimethylaziridin-2-carboxamid (C-3364)
N,N-Dimethyl-1-(1-naphthylsulfonyl)aziridin-2-carboxamid (C-3365)
N,N-Dimethyl-1-[(4-methyl-1-naphthyl)sulfonyl]aziridin-2-carboxamid (C-3366)
N,N-Dimethyl-1-(4-pentylphenyl)sulfonylaziridin-2-carboxamid (C-3368)
1-(4-Hexylphenyl)sulfonyl-N,N-dimethylaziridin-2-carboxamid (C-3369)
N,N-Dimethyl-1-[4-(trifluormethyl)phenyl]sulfonylaziridin-2-carboxamid (C-3374)
1-(4-Hexylphenyl)sulfonyl-N-methylaziridin-2-carboxamid (C-3380)
Methyl-1-[[5-(dimethylamino)-2-naphthyl]sulfonyl]aziridin-2-carboxylat (C-3399)
1-(4-Hexylphenyl)sulfonylaziridin-2-carboxamid (C-3389)
1-(4-Cyclohexylphenyl)sulfonyl-N,N-dimethylaziridin-2-carboxamid (C-3384)
1-(2,4-Difluorphenyl)sulfonylaziridin-2-carboxamid (C-3402)
1-(2,4-Difluorphenyl)sulfonyl-N,N-dimethylaziridin-2-carboxamid (C-3403)
1-[[6-(Dimethylamino)-1-naphthyl]sulfonyl]aziridin-2-carboxamid (C-3459)
[1-(4-Hexylphenyl)sulfonylaziridin-2-yl]pyrrolidin-1-ylmethanon (C-3511)
[1-(4-Hexylphenyl)sulfonylaziridin-2-yl]morpholinomethanon (C-3517)
Methyl-1-(4-fluorphenyl)sulfonylaziridin-2-carboxylat (C-3576)
N,N-Diethyl-1-(4-hexylphenyl)sulfonylaziridin-2-carboxamid (C-3546)
1-(4-tert-Butylphenyl)sulfonylaziridin-2-carboxamid (C-3591)
1-(4-tert-Butylphenyl)sulfonyl-N,N-dimethylaziridin-2-carboxamid (C-3594)
1-(4-tert-Butylphenyl)sulfonyl-N-methylaziridin-2-carboxamid (C-3593).

2. N,N-disubstituierte aromatische Sulfonamide oder ein pharmazeutisch akzeptables Salz davon, ausgewählt aus der folgenden Liste, zur Verwendung bei der Behandlung oder Prävention von Erkrankungen, die mit PDI A1 verbunden sind:
Methyl-1-(p-tolylsulfonyl)aziridin-2-carboxylat (C-3161);
Methyl-1-(4-nitrophenyl)sulfonylaziridin-2-carboxylat (C-3212);
1-(4-Nitrophenyl)sulfonylaziridin-2-carboxamid (C-3216);
1-(4-Aminophenyl)sulfonylaziridin-2-carboxamid (C-3218);
1-(p-Tolylsulfonyl)aziridin-2-carboxamid (C-3220);
Methyl-1-(benzolsulfonyl)aziridin-2-carboxylat (C-3251);
Methyl-1-[4-(trifluormethyl)phenyl]sulfonylaziridin-2-carboxylat (C-3256);
Methyl-1-(4-butylphenyl)sulfonylaziridin-2-carboxylat (C-3257);
1-(p-Tolylsulfonyl)aziridin-2-carbaldehyd (C-3262);
1-[1-(p-Tolylsulfonyl)aziridin-2-yl]ethanon (C-3263);
Methyl-1-(4-dodecylphenyl)sulfonylaziridin-2-carboxylat (C-3270);
Methyl-1-(4-butoxyphenyl)sulfonylaziridin-2-carboxylat (C-3271);
1-[1-(4-Butylphenyl)sulfonylaziridin-2-yl]ethanon (C-3272);
1-(4-Butylphenyl)sulfonylaziridin-2-carbaldehyd (C-3273);
Methyl-1-(4-pentylphenyl)sulfonylaziridin-2-carboxylat (C-3281);
Methyl-1-(4-hexylphenyl)sulfonylaziridin-2-carboxylat (C-3287);
Methyl-1-(4-heptylphenyl)sulfonylaziridin-2-carboxylat (C-3288);
Methyl-1-(4-tert-butylphenyl)sulfonylaziridin-2-carboxylat (C-3290);
Methyl-1-(4-phenylphenyl)sulfonylaziridin-2-carboxylat (C-3291);
Methyl-1-(2-naphthylsulfonyl)aziridin-2-carboxylat (C-3292);
Methyl-1-(4-methoxyphenyl)sulfonylaziridin-2-carboxylat (C-3294);
Methyl-1-(4-isopropylphenyl)sulfonylaziridin-2-carboxylat (C-3295);
Methyl-1-(4-chlorphenyl)sulfonylaziridin-2-carboxylat (C-3296);
Methyl-1-(2-fluor-4-methylphenyl)sulfonylaziridin-2-carboxylat (C-3297);
Methyl-1-(4-propylphenyl)sulfonylaziridin-2-carboxylat (C-3299);
Methyl-1-(m-tolylsulfonyl)aziridin-2-carboxylat (C-3303);
Methyl-1-(4-propylphenyl)sulfonylaziridin-2-carboxylat (C-3304);
Methyl-1-[3-(trifluormethyl)phenyl]sulfonylaziridin-2-carboxylat (C-3305);
Methyl-1-(4-acetamidophenyl)sulfonylaziridin-2-carboxylat (C-3308);
Methyl-1-(1-naphthylsulfonyl)aziridin-2-carboxylat (C-3311);
1-(p-Tolylsulfonyl)aziridin-2-carbonitril (C-3314);
Methyl-1-(4-cyclohexylphenyl)sulfonylaziridin-2-carboxylat (C-3316);
Methyl-1-(4-octylphenyl)sulfonylaziridin-2-carboxylat (C-3319);
Methyl-1-(o-tolylsulfonyl)aziridin-2-carboxylat (C-3320);
1-[4-(Trifluormethyl)phenyl]sulfonylaziridin-2-carboxamid (C-3324);
Methyl-1-[[5-(dimethylamino)-1-naphthyl]sulfonyl]aziridin-2-carboxylat (C-3326);
Methyl-1-[(6-methoxy-2-naphthyl)sulfonyl]aziridin-2-carboxylat (C-3327);
Methyl-1-[(6-methyl-2-naphthyl)sulfonyl]aziridin-2-carboxylat (C-3329);
Methyl-1-(4-ethylphenyl)sulfonylaziridin-2-carboxylat (C-3332);
Methyl-1-[(4-methyl-1-naphthyl)sulfonyl]aziridin-2-carboxylat (C-3336);
N,N-Dimethyl-1-(p-tolylsulfonyl)aziridin-2-carboxamid (C-3342);
Methyl-1-[4-(dimethylamino)phenyl]sulfonylaziridin-2-carboxylat (C-3343);
Methyl-1-(2-furylsulfonyl)aziridin-2-carboxylat (C-3346);
Methyl-1-[(2-oxo-1H-chinolin-6-yl)sulfonyl]aziridin-2-carboxylat (C-3350);
Methyl-1-(2,4-difluorphenyl)sulfonylaziridin-2-carboxylat (C-3353);
Methyl-1-(2-oxochromen-6-yl)sulfonylaziridin-2-carboxylat (C-3355);
1-[4-(Dimethylamino)phenyl]sulfonyl-N,N-dimethylaziridin-2-carboxamid (C-3357);
1-(4-Butylphenyl)sulfonyl-N,N-dimethylaziridin-2-carboxamid (C-3362);
1-(4-Isopropylphenyl)sulfonyl-N,N-dimethylaziridin-2-carboxamid (C-3364);
N,N-Dimethyl-1-(1-naphthylsulfonyl)aziridin-2-carboxamid (C-3365);
N,N-Dimethyl-1-[(4-methyl-1-naphthyl)sulfonyl]aziridin-2-carboxamid (C-3366);
N,N-Dimethyl-1-(4-pentylphenyl)sulfonylaziridin-2-carboxamid (C-3368);
1-(4-Hexylphenyl)sulfonyl-N,N-dimethylaziridin-2-carboxamid (C-3369);
N,N-Dimethyl-1-[4-(trifluormethyl)phenyl]sulfonylaziridin-2-carboxamid (C-3374);
1-(4-Hexylphenyl)sulfonyl-N-methylaziridin-2-carboxamid (C-3380);
1-(4-Cyclohexylphenyl)sulfonyl-N,N-dimethylaziridin-2-carboxamid (C-3384);
1-(4-Hexylphenyl)sulfonylaziridin-2-carboxamid (C-3389);
Methyl-1-[[5-chlor-6-(methylamino)-2-naphthyl]sulfonyl]aziridin-2-carboxylat (C-3393);
1-(2,4-Difluorphenyl)sulfonylaziridin-2-carboxamid (C-3402);
1-(2,4-Difluorphenyl)sulfonyl-N,N-dimethylaziridin-2-carboxamid (C-3403);
1-[[6-(Dimethylamino)-1-naphthyl]sulfonyl]aziridin-2-carboxamid (C-3459);
[1-(4-Hexylphenyl)sulfonylaziridin-2-yl]pyrrolidin-1-ylmethanon (C-3511);
[1-(4-Hexylphenyl)sulfonylaziridin-2-yl]morpholinomethanon (C-3517);
1-(Benzolsulfonyl)aziridin-2-carboxamid (C-3532);
Methyl-(2S)-1-(p-tolylsulfonyl)aziridin-2-carboxylat (C-3535);
Ethyl-1-(p-tolylsulfonyl)aziridin-2-carboxylat (C-3537);
Methyl-(2R)-1-(p-tolylsulfonyl)aziridin-2-carboxylat (C-3539);
N,N-Diethyl-1-(4-hexylphenyl)sulfonylaziridin-2-carboxamid (C-3546);
Methyl-1-(3-pyridylsulfonyl)aziridin-2-carboxylat (C-3559);
1-(3-Pyridylsulfonyl)aziridin-2-carboxamid (C-3562);
Methyl-(2R)-1-[[6-(dimethylamino)-1-naphthyl]sulfonyl]aziridin-2-carboxylat (C-3570);
Methyl-1-(4-fluorphenyl)sulfonylaziridin-2-carboxylat (C-3576);
1-(4-tert-Butylphenyl)sulfonylaziridin-2-carboxamid (C-3591);
1-(4-tert-Butylphenyl)sulfonyl-N-methylaziridin-2-carboxamid (C-3593);
1-(4-tert-Butylphenyl)sulfonyl-N,N-dimethylaziridin-2-carboxamid (C-3594);
Lithium-1-tosylaziridin-2-carboxylat (C-3612).

3. N,N-disubstituierte aromatische Sulfonamide zur Verwendung gemäß Anspruch 2 bei der Behandlung und Prävention einer übermäßigen Thrombozytenaktivierung und Thrombose, insbesondere jeder Erkrankung aus der folgenden Liste:
wobei es sich bei der Erkrankung oder dem Zustand um eine Thrombose oder thrombotische Erkrankungen handelt, wobei die thrombotische Erkrankung insbesondere ein akuter Myokardinfarkt, eine stabile Angina pectoris, eine instabile Angina pectoris, ein akuter Verschluss nach einer Koronarangioplastie und/oder Stentimplantation, transitorische ischämische Attacken, eine zerebrovaskuläre Erkrankung, eine periphere Gefäßerkrankung, eine Plazentainsuffizienz, Vorhofflimmern, eine tiefe Venenthrombose oder eine Lungenembolie sowie andere mit einer übermäßigen Aktivierung der Thrombozyten verbundene Pathologien ist.

4. N,N-disubstituierte aromatische Sulfonamide zur Verwendung gemäß Anspruch 2 bei der Behandlung und Prävention von Krebs, insbesondere jeder Erkrankung aus der folgenden Liste:
gastrointestinaler Krebs, kolorektaler Krebs, Dickdarmkrebs, Leberkrebs, hepatozelluläres Karzinom, Bauchspeicheldrüsenkrebs, Krebs der Gallenwege, Magenkrebs, Krebs des Urogenitaltrakts, Blasenkrebs, Hodenkrebs, Gebärmutterhalskrebs, malignes Mesotheliom, osteogenes Sarkom, Speiseröhrenkrebs, Kehlkopfkrebs, Prostatakrebs, hormonrefraktärer Prostatakrebs, Lungenkrebs, kleinzelliges Lungenkarzinom, nicht-kleinzelliges Lungenkarzinom, Brustkrebs, triple-negativer Brustkrebs, Brustkrebs mit einer Mutation des BRCA1- und/oder BRCA2-Gens, hämatologischer Krebs, Leukämie, akute lymphoblastische Leukämie, akute myeloische Leukämie, chronische lymphatische Leukämie, chronische myeloische Leukämie, Lymphom, Hodgkin-Lymphom, Non-Hodgkin-Lymphom, follikuläres Lymphom, diffus großzelliges B-Zell-Lymphom, Eierstockkrebs, Hirnkrebs, Neuroblastom, Ewing-Sarkom, Nierenkrebs, Plattenepithelkarzinom, Hautkrebs, Melanom, Kopf- und/oder Halskrebs, Plattenepithelkarzinom des Kopfes und Halses sowie Mundkrebs.

## Revendications

1. Sulfonamides aromatiques N,N-disubstitués sous forme de racémates ou d'énantiomères, ou un sel pharmaceutiquement acceptable de ceux-ci, dans lesquels les composés sont choisis dans la liste suivante :
1-(4-Nitrophényl)sulfonylaziridine-2-carboxamide (C-3216)
1-(4-Aminophényl)sulfonylaziridine-2-carboxamide (C-3218)
1-[4-(Trifluorométhyl)phényl]sulfonylaziridine-2-carboxylate de méthyle (C-3256)
1-(4-Butylphényl)sulfonylaziridine-2-carboxylate de méthyle (C-3257)
1-(4-Dodécylphényl)sulfonylaziridine-2-carboxylate de méthyle (C-3270)
1-(4-Butoxyphényl)sulfonylaziridine-2-carboxylate de méthyle (C-3271)
1-[1-(4-Butylphényl)sulfonylaziridin-2-yl]éthanone (C-3272)
1-(4-Butylphényl)sulfonylaziridine-2-carbaldehyde (C-3273)
1-(4-Pentylphényl)sulfonylaziridine-2-carboxylate de méthyle (C-3281)
1-(4-Hexylphényl)sulfonylaziridine-2-carboxylate de méthyle (C-3287)
1-(4-Heptylphényl)sulfonylaziridine-2-carboxylate de méthyle (C-3288)
1-(4-tert-Butylphényl)sulfonylaziridine-2-carboxylate de méthyle (C-3290)
1-(4-Phénylphényl)sulfonylaziridine-2-carboxylate de méthyle (C-3291)
1-(2-Naphtylsulfonyl)aziridine-2-carboxylate de méthyle (C-3292)
1-(4-Isopropylphényl)sulfonylaziridine-2-carboxylate de méthyle (C-3295)
1-(2-Fluoro-4-méthylphényl)sulfonylaziridine-2-carboxylate de méthyle (C-3297)
1-(4-Propylphényl)sulfonylaziridine-2-carboxylate de méthyle (C-3299)
1-(m-Tolylsulfonyl)aziridine-2-carboxylate de méthyle (C-3303)
1-[3-(Trifluorométhyl)phényl]sulfonylaziridine-2-carboxylate de méthyle (C-3305)
1-(4-Acétamidophényl)sulfonylaziridine-2-carboxylate de méthyle (C-3308)
1-(1-Naphtylsulfonyl)aziridine-2-carboxylate de méthyle (C-3311)
1-(4-Cyclohexylphényl)sulfonylaziridine-2-carboxylate de méthyle (C-3316)
1-(4-Octylphényl)sulfonylaziridine-2-carboxylate de méthyle (C-3319)
1-(o-Tolylsulfonyl)aziridine-2-carboxylate de méthyle (C-3320)
1-[4-(Trifluorométhyl)phényl]sulfonylaziridine-2-carboxamide (C-3324)
1-[[5-(Diméthylamino)-1-naphtyl]sulfonyl]aziridine-2-carboxylate de méthyle (C-3326)
1-[(6-Méthoxy-2-naphtyl)sulfonyl]aziridine-2-carboxylate de méthyle (C-3327)
1-[(6-Méthyl-2-naphtyl)sulfonyl]aziridine-2-carboxylate de méthyle (C-3329)
1-(4-Éthylphényl)sulfonylaziridine-2-carboxylate de méthyle (C-3332)
1-[4-(Diméthylamino)phényl]sulfonylaziridine-2-carboxylate de méthyle (C-3343)
1-(2,4-Difluorophényl)sulfonylaziridine-2-carboxylate de méthyle (C-3353)
1-[4-(Diméthylamino)phényl]sulfonyl-N,N-diméthylaziridine-2-carboxamide (C-3357)
1-(4-Butylphényl)sulfonyl-N,N-diméthylaziridine-2-carboxamide (C-3362)
1-(4-Isopropylphényl)sulfonyl-N,N-diméthylaziridine-2-carboxamide (C-3364)
N,N-Diméthyl-1-(1-naphtylsulfonyl)aziridine-2-carboxamide (C-3365)
N,N-Diméthyl-1-[(4-méthyl-1-naphtyl)sulfonyl]aziridine-2-carboxamide (C-3366)
N,N-Diméthyl-1-(4-pentylphényl)sulfonylaziridine-2-carboxamide (C-3368)
1-(4-Hexylphényl)sulfonyl-N,N-diméthylaziridine-2-carboxamide (C-3369)
N,N-Diméthyl-1-[4-(trifluorométhyl)phényl]sulfonylaziridine-2-carboxamide (C-3374)
1-(4-Hexylphényl)sulfonyl-N-méthylaziridine-2-carboxamide (C-3380)
1-[[5-(Diméthylamino)-2-naphtyl]sulfonyl]aziridine-2-carboxylate de méthyle (C-3399)
1-(4-Hexylphényl)sulfonylaziridine-2-carboxamide (C-3389)
1-(4-Cyclohexylphényl)sulfonyl-N,N-diméthylaziridine-2-carboxamide (C-3384)
1-(2,4-Difluorophényl)sulfonylaziridine-2-carboxamide (C-3402)
1-(2,4-Difluorophényl)sulfonyl-N,N-diméthylaziridine-2-carboxamide (C-3403)
1-[[6-(Diméthylamino)-1-naphtyl]sulfonyl]aziridine-2-carboxamide (C-3459)
[1-(4-Hexylphényl)sulfonylaziridin-2-yl]pyrrolidin-1-ylméthanone (C-3511)
[1-(4-Hexylphényl)sulfonylaziridin-2-yl]morpholinométhanone (C-3517)
1-(4-Fluorophényl)sulfonylaziridine-2-carboxylate de méthyle (C-3576)
N,N-Diéthyl-1-(4-hexylphényl)sulfonylaziridine-2-carboxamide (C-3546)
1-(4-tert-Butylphényl)sulfonylaziridine-2-carboxamide (C-3591)
1-(4-tert-Butylphényl)sulfonyl-N,N-diméthylaziridine-2-carboxamide (C-3594)
1-(4-tert-Butylphényl)sulfonyl-N-méthylaziridine-2-carboxamide (C-3593).

2. Sulfonamides aromatiques N,N-disubstitués, ou un sel pharmaceutiquement acceptable de ceux-ci, choisis dans la liste suivante, pour une utilisation dans le traitement ou la prévention de maladies liées à la PDI A1 :
1-(p-Tolylsulfonyl)aziridine-2-carboxylate de méthyle (C-3161) ;
1-(4-Nitrophényl)sulfonylaziridine-2-carboxylate de méthyle (C-3212) ;
1-(4-Nitrophényl)sulfonylaziridine-2-carboxamide (C-3216) ;
1-(4-Aminophényl)sulfonylaziridine-2-carboxamide (C-3218) ;
1-(p-Tolylsulfonyl)aziridine-2-carboxamide (C-3220) ;
1-(Benzènesulfonyl)aziridine-2-carboxylate de méthyle (C-3251) ;
1-[4-(Trifluorométhyl)phényl]sulfonylaziridine-2-carboxylate de méthyle (C-3256) ;
1-(4-Butylphényl)sulfonylaziridine-2-carboxylate de méthyle (C-3257) ;
1-(p-Tolylsulfonyl)aziridine-2-carbaldehyde (C-3262) ;
1-[1-(p-Tolylsulfonyl)aziridin-2-yl]éthanone (C-3263) ;
1-(4-Dodécylphényl)sulfonylaziridine-2-carboxylate de méthyle (C-3270) ;
1-(4-Butoxyphényl)sulfonylaziridine-2-carboxylate de méthyle (C-3271) ;
1-[1-(4-Butylphényl)sulfonylaziridin-2-yl]éthanone (C-3272) ;
1-(4-Butylphényl)sulfonylaziridine-2-carbaldehyde (C-3273) ;
1-(4-Pentylphényl)sulfonylaziridine-2-carboxylate de méthyle (C-3281) ;
1-(4-Hexylphényl)sulfonylaziridine-2-carboxylate de méthyle (C-3287) ;
1-(4-Heptylphényl)sulfonylaziridine-2-carboxylate de méthyle (C-3288) ;
1-(4-tert-Butylphényl)sulfonylaziridine-2-carboxylate de méthyle (C-3290) ;
1-(4-Phénylphényl)sulfonylaziridine-2-carboxylate de méthyle (C-3291) ;
1-(2-Naphtylsulfonyl)aziridine-2-carboxylate de méthyle (C-3292) ;
1-(4-Méthoxyphényl)sulfonylaziridine-2-carboxylate de méthyle (C-3294) ;
1-(4-Isopropylphényl)sulfonylaziridine-2-carboxylate de méthyle (C-3295) ;
1-(4-Chlorophényl)sulfonylaziridine-2-carboxylate de méthyle (C-3296) ;
1-(2-Fluoro-4-méthylphényl)sulfonylaziridine-2-carboxylate de méthyle (C-3297) ;
1-(4-Propylphényl)sulfonylaziridine-2-carboxylate de méthyle (C-3299) ;
1-(m-Tolylsulfonyl)aziridine-2-carboxylate de méthyle (C-3303) ;
1-(4-Propylphényl)sulfonylaziridine-2-carboxylate de méthyle (C-3304) ;
1-[3-(Trifluorométhyl)phényl]sulfonylaziridine-2-carboxylate de méthyle (C-3305) ;
1-(4-Acétamidophényl)sulfonylaziridine-2-carboxylate de méthyle (C-3308) ;
1-(1-Naphtylsulfonyl)aziridine-2-carboxylate de méthyle (C-3311) ;
1-(p-Tolylsulfonyl)aziridine-2-carbonitrile (C-3314) ;
1-(4-Cyclohexylphényl)sulfonylaziridine-2-carboxylate de méthyle (C-3316) ;
1-(4-Octylphényl)sulfonylaziridine-2-carboxylate de méthyle (C-3319) ;
1-(o-Tolylsulfonyl)aziridine-2-carboxylate de méthyle (C-3320) ;
1-[4-(Trifluorométhyl)phényl]sulfonylaziridine-2-carboxamide (C-3324) ;
1-[[5-(Diméthylamino)-1-naphtyl]sulfonyl]aziridine-2-carboxylate de méthyle (C-3326) ;
1-[(6-Méthoxy-2-naphtyl)sulfonyl]aziridine-2-carboxylate de méthyle (C-3327) ;
1-[(6-Méthyl-2-naphtyl)sulfonyl]aziridine-2-carboxylate de méthyle (C-3329) ;
1-(4-Éthylphényl)sulfonylaziridine-2-carboxylate de méthyle (C-3332) ;
1-[(4-Méthyl-1-naphtyl)sulfonyl]aziridine-2-carboxylate de méthyle (C-3336) ;
N,N-Diméthyl-1-(p-tolylsulfonyl)aziridine-2-carboxamide (C-3342) ;
1-[4-(Diméthylamino)phényl]sulfonylaziridine-2-carboxylate de méthyle (C-3343) ;
1-(2-Furylsulfonyl)aziridine-2-carboxylate de méthyle (C-3346) ;
1-[(2-Oxo-1H-quinolin-6-yl)sulfonyl]aziridine-2-carboxylate de méthyle (C-3350) ;
1-(2,4-Difluorophényl)sulfonylaziridine-2-carboxylate de méthyle (C-3353) ;
1-(2-Oxochromén-6-yl)sulfonylaziridine-2-carboxylate de méthyle (C-3355) ;
1-[4-(Diméthylamino)phényl]sulfonyl-N,N-diméthylaziridine-2-carboxamide (C-3357) ;
1-(4-Butylphényl)sulfonyl-N,N-diméthylaziridine-2-carboxamide (C-3362) ;
1-(4-Isopropylphényl)sulfonyl-N,N-diméthylaziridine-2-carboxamide (C-3364) ;
N,N-Diméthyl-1-(1-naphtylsulfonyl)aziridine-2-carboxamide (C-3365) ;
N,N-Diméthyl-1-[(4-méthyl-1-naphtyl)sulfonyl]aziridine-2-carboxamide (C-3366) ;
N,N-Diméthyl-1-(4-pentylphényl)sulfonylaziridine-2-carboxamide (C-3368) ;
1-(4-Hexylphényl)sulfonyl-N,N-diméthylaziridine-2-carboxamide (C-3369) ;
N,N-Diméthyl-1-[4-(trifluorométhyl)phényl]sulfonylaziridine-2-carboxamide (C-3374) ;
1-(4-Hexylphényl)sulfonyl-N-méthylaziridine-2-carboxamide (C-3380) ;
1-(4-Cyclohexylphényl)sulfonyl-N,N-diméthylaziridine-2-carboxamide (C-3384) ;
1-(4-Hexylphényl)sulfonylaziridine-2-carboxamide (C-3389) ;
1-[[5-Chloro-6-(méthylamino)-2-naphtyl]sulfonyl]aziridine-2-carboxylate de méthyle (C-3393) ;
1-(2,4-Difluorophényl)sulfonylaziridine-2-carboxamide (C-3402) ;
1-(2,4-Difluorophényl)sulfonyl-N,N-diméthylaziridine-2-carboxamide (C-3403) ;
1-[[6-(Diméthylamino)-1-naphtyl]sulfonyl]aziridine-2-carboxamide (C-3459) ;
[1-(4-Hexylphényl)sulfonylaziridin-2-yl]pyrrolidin-1-ylméthanone (C-3511) ;
[1-(4-Hexylphényl)sulfonylaziridin-2-yl]morpholinométhanone (C-3517) ;
1-(Benzènesulfonyl)aziridine-2-carboxamide (C-3532) ;
1-(p-Tolylsulfonyl)aziridine-2-carboxylate de méthyle (2S) (C-3535) ;
1-(p-Tolylsulfonyl)aziridine-2-carboxylate d'éthyle (C-3537) ;
1-(p-Tolylsulfonyl)aziridine-2-carboxylate de méthyle (2R) (C-3539) ;
N,N-Diéthyl-1-(4-hexylphényl)sulfonylaziridine-2-carboxamide (C-3546) ;
1-(3-Pyridylsulfonyl)aziridine-2-carboxylate de méthyle (C-3559) ;
1-(3-Pyridylsulfonyl)aziridine-2-carboxamide (C-3562) ;
1-[[6-(Diméthylamino)-1-naphtyl]sulfonyl]aziridine-2-carboxylate de méthyle (2R) (C-3570) ;
1-(4-Fluorophényl)sulfonylaziridine-2-carboxylate de méthyle (C-3576) ;
1-(4-tert-Butylphényl)sulfonylaziridine-2-carboxamide (C-3591) ;
1-(4-tert-Butylphényl)sulfonyl-N-méthylaziridine-2-carboxamide (C-3593) ;
1-(4-tert-Butylphényl)sulfonyl-N,N-diméthylaziridine-2-carboxamide (C-3594) ;
1-Tosylaziridine-2-carboxylate de lithium (C-3612).

3. Sulfonamides aromatiques N,N-disubstitués destinés à être utilisés selon la revendication 2 dans le traitement et la prévention de l'activation excessive des plaquettes et de la thrombose, en particulier de toute maladie de la liste suivante :
la maladie ou l'affection étant une thrombose ou une maladie thrombotique, en particulier la maladie thrombotique étant un infarctus aigu du myocarde, une angine de poitrine stable, une angine de poitrine instable, une occlusion aiguë consécutive à une angioplastie coronaire et/ou à la mise en place d'un stent, des accidents ischémiques transitoires, une maladie cérébrovasculaire, une maladie vasculaire périphérique, une insuffisance placentaire, une fibrillation auriculaire, une thrombose veineuse profonde ou une embolie pulmonaire, ainsi que d'autres pathologies liées à une activation excessive des plaquettes.

4. Sulfonamides aromatiques N,N-disubstitués destinés à être utilisés selon la revendication 2 dans le traitement et la prévention du cancer, en particulier de toute maladie de la liste suivante :
cancer gastro-intestinal, cancer colorectal, cancer du côlon, cancer du foie, carcinome hépatocellulaire, cancer du pancréas, cancer des voies biliaires, cancer de l'estomac, cancer de l'appareil génito-urinaire, cancer de la vessie, cancer du testicule, cancer du col de l'utérus, mésothéliome malin, sarcome ostéogénique, cancer de l'œsophage, cancer du larynx, cancer de la prostate, cancer de la prostate hormono-résistant, cancer du poumon, cancer du poumon à petites cellules, cancer du poumon non à petites cellules, cancer du sein, cancer du sein triple négatif, cancer du sein présentant une mutation du gène BRCA1 et/ou BRCA2, cancer hématologique, leucémie, leucémie aiguë lymphoblastique, leucémie aiguë myéloïde, leucémie lymphoïde chronique, leucémie myéloïde chronique, lymphome, lymphome de Hodgkin, lymphome non hodgkinien, lymphome folliculaire, lymphome diffus à grandes cellules B, cancer de l'ovaire, cancer du cerveau, neuroblastome, sarcome d'Ewing, cancer du rein, cancer épidermoïde, cancer de la peau, mélanome, cancer de la tête et/ou du cou, carcinome épidermoïde de la tête et du cou, et cancer de la bouche.
